(19) Europäisches Patentamt
European Patent Office
Office européen des brevets

(11) EP 3 611 256 A1

(12) EUROPEAN PATENT APPLICATION

(43) Date of publication:
19.02.2020 Bulletin 2020/08

(51) Int Cl.:
*C12N 5/074* (2010.01)

(21) Application number: 19191147.8

(22) Date of filing: 09.08.2019

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 14.08.2018 EP 18188930

(71) Applicants:
- **Universität Heidelberg**
  **69117 Heidelberg (DE)**
- **Universitätsklinikum Jena**
  **07743 Jena (DE)**

(72) Inventors:
- **Cheng, Xinlai**
  **69120 Heidelberg (DE)**
- **Wölfl, Stefan**
  **69120 Heidelberg (DE)**
- **Mrowka, Ralf**
  **07734 Jena (DE)**
- **Adjaye, James**
  **40225 Düsseldorf (DE)**

(74) Representative: **Schulz Junghans Patentanwälte PartGmbB**
**Großbeerenstraße 71**
**10963 Berlin (DE)**

# (54) APPLICATION OF IMIDAZOPYRIDINE DERIVATIVES IN REGENERATIVE MEDICINE

(57) A method of producing a pluripotent stem cell is provided. The method is comprising contacting a non-pluripotent donor cell obtained from a mammalian donor with a compound characterized by general formulas (1) and (3). Furthermore, methods for inducing OCT4 and NANOG, increasing histone 3 lysine methylation and the maintenance of pluripotency are provided.

EP 3 611 256 A1

## Description

The present invention relates to an in vitro/ex vivo method for producing induced pluripotent stem cells from non-pluripotent donor cells.

Pluripotent stem cells, such as embryonic stem cells, have the potential to differentiate into any of the three germ layers: endoderm, mesoderm and ectoderm, and therefore give rise to any cell type derived from these germ layers.

Nuclear reprogramming from terminally differentiated somatic cells into induced pluripotent stem cells (iPSCs) achieved by ectopic expression of a core of transcription factors, like Oct4, Sox2, Klf4, c-Myc, Nanog and Lin28 provides a promising source for autologous organ transplantation, drug discovery and disease modelling, opening a new era in the field of regenerative medicine.

Methods available for the generation of pluripotent stem cells are somatic cell nuclear transfer (SCNT) and reprogramming of human fibroblasts. Both methods have their distinct disadvantages and limitations. The reprogramming of human fibroblasts is a time-consuming (3-4 weeks) and inefficient (<0.01% of cells) process. It also can be hampered by random mutations appearing in the course of reprogramming and during maintenance. Therefore, higher reprogramming efficiency and optimized cultivation conditions for iPSC maintenance can contribute to risk reduction. Improved reprogramming efficiency for generation of individual patients own iPSCs will also allow to avoid risks associated with autologous iPSC cell line derived allografts, like immunoreaction, evidenced by allogeneic transplantation of iPSC-derived neural cells in nonhuman primates (Morizane et al. Stem Cell Reports. 2013 Sep 26;1 (4):283-9).

In contrast to using genetic manipulation for reprogramming somatic cells, the use of small molecules shows distinct advantages in controlling the reprogramming process. Recently a number of small molecules have been disclosed that greatly contribute to the establishment of more efficient and reliable protocols for the generation and maintenance of iPSCs (Watanabe et al, Nat Biotechnol. 2007 Jun;25(6):681-6; Ying et al Nature. 2008 May 22;453(7194):519-23). Small molecule cocktails that exhibit excellent reprogramming efficiency for the generation of mouse iPSCs have been disclosed recently (Hou et al, Science. 2013 Aug 9;341(6146):651-4; Zhao et al, Cell. 2015 Dec 17;163(7):1678-91).

Herein the inventors disclose that 2-arylpyrimidazoles - including zolpidem, an approved drug for insomnia treatment, - act as novel potent pluripotency inducers for the generation and maintenance of human iPSCs.

Based on the above-mentioned state of the art, the objective of the present invention is to provide means and methods for the induction of pluripotency in mammalian donor cells and the maintenance of pluripotency in these cells. This objective is attained by the subject matter of the claims of the present specification.

### Terms and definitions

The term *pluripotent stem cell* in the context of the present specification relates to cells with pluripotent abilities. Pluripotency is a continuum ranging from completely pluripotent (=omnipotent) to partially pluripotent (=multipotent) cells. Omnipotent cells can differentiate into any cell type of the body. Examples for omnipotent stem cells (=completely pluripotent) are embryonic stem cells. In contrast, adult stem cells (=partially pluripotent stem cell) can usually only differentiate into the cell type of the organ they originate from. Multipotent cells have the gene activation potential to differentiate into discrete cell types. A non-limiting example of multipotent cells are neural stem cells. In addition to pluripotency, the pluripotent stem cell has the ability to go through several cycles of cell division while maintaining its undifferentiated state. The term pluripotent stem cell therefore encompasses such cells as embryonic stem cells, adult stem cells, neural stem cells and induced pluripotent stem cells.

The term *$C_1$-$C_3$ alkyl* in the context of the present specification relates to a saturated hydrocarbon having 1, 2 or 3 carbon atoms. Non-limiting examples for a $C_1$-$C_3$ alkyl are methyl, ethyl and *n*- or *iso*-propyl.

The term *$C_1$-$C_3$ alkoxy* moiety in the context of the present specification relates to a *$C_1$-$C_3$ alkyl* as specified above, connected to the core moiety by an oxygen. Examples of a $C_1$-$C_3$ alkoxy are methoxy ($CH_3O$-), ethoxy ($CH_3CH_2O$-) and *n*-propoxy ($CH_3CH_2CH_2O$-) or *iso*-propoxy ($(CH_3)_2CHO$-).

A $C_1$-$C_3$ *carboxylic acid* in the context of the present specification relates to a linear or branched hydrocarbon having 1, 2 or 3 carbon atoms and comprising a carboxyl group (-C(=O)OH). Non-limiting examples for a $C_1$-$C_3$ carboxylic acid are carbonic acid (OH-COOH), methanoic acid (H-COOH), acetic acid ($CH_3$-COOH) and propionic acid ($CH_3CH_2COOH$).

The term *$C_1$-$C_4$ alkyl* in the context of the present specification relates to a saturated linear or branched hydrocarbon having 1, 2, 3 or 4 carbon atoms, wherein in certain embodiments one carbon-carbon bond may be unsaturated and one $CH_2$ moiety may be exchanged for oxygen (ether bridge) or nitrogen (NH, or NR with R being methyl, ethyl, or propyl; amino bridge). Non-limiting examples for a $C_1$-$C_4$ alkyl are methyl, ethyl, propyl, prop-2-enyl, n-butyl, 2-methylpropyl, *tert*-butyl, but-3-enyl, prop-2-inyl and but-3-inyl. In certain embodiments, a $C_1$-$C_4$ alkyl is a methyl, ethyl, propyl or butyl moiety.

A *$C_1$-$C_6$ alkyl* in the context of the present specification relates to a saturated linear or branched hydrocarbon having 1, 2, 3, 4, 5 or 6 carbon atoms, wherein one carbon-carbon bond may be unsaturated and/or one $CH_2$ moiety

may be exchanged for oxygen (ether bridge) or nitrogen (NH, or NR with R being methyl, ethyl, or propyl; amino bridge). Non-limiting examples for a $C_1$-$C_6$ alkyl include the examples given for $C_1$-$C_4$ alkyl above, and additionally 3-methylbut-2-enyl, 2-methylbut-3-enyl, 3-methylbut-3-enyl, n-pentyl, 2-methylbutyl, 3-methylbutyl, 1,1-dimethylpropyl, 1,2-dimethylpropyl, 1,2-dimethylpropyl, pent-4-inyl, 3-methyl-2-pentyl, and 4-methyl-2-pentyl. In certain embodiments, a $C_5$ alkyl is a pentyl or cyclopentyl moiety and a $C_6$ alkyl is a hexyl or cyclohexyl moiety.

The term *$C_4$-$C_7$ cycloalkyl* in the context of the present specification relates to a saturated hydrocarbon ring having 4, 5, 6 or 7 carbon atoms, wherein in certain embodiments, one carbon-carbon bond may be unsaturated and/or one $CH_2$ moiety may be exchanged for oxygen (ether bridge) or nitrogen (NH, or NR with R being methyl, ethyl, or propyl; amino bridge). Non-limiting examples of a $C_4$-$C_7$ cycloalkyl moiety include cyclobutyl (-$C_4H_7$), cyclopentenyl ($C_5H_9$), and cyclohexenyl ($C_6H_{11}$) moieties. In certain embodiments, a cycloalkyl is substituted by one $C_1$ to $C_4$ unsubstituted alkyl moiety. In certain embodiments, a cycloalkyl is substituted by more than one $C_1$ to $C_4$ unsubstituted alkyl moieties.

The term *unsubstituted $C_n$ alkyl* when used herein in the narrowest sense relates to the moiety - $C_nH_{2n}$- if used as a bridge between moieties of the molecule, or -$C_nH_{2n+1}$ if used in the context of a terminal moiety. It may still contain fewer H atoms if a cyclical structure or one or more (nonaromatic) double bonds are present.

The term *$C_n$ alkylene* in the context of the present specification relates to a saturated linear or branched hydrocarbon comprising one or more double bonds. An unsubstituted alkylene consists of C and H only. A substituted alkylene may comprise substituents as defined herein for substituted alkyl.

The term *$C_n$ alkylyne* in the context of the present specification relates to a saturated linear or branched hydrocarbon comprising one or more triple bonds and may also comprise one or more double bonds in addition to the triple bond(s). An unsubstituted alkylyne consists of C and H only. A substituted alkylyne may comprise substituents as defined herein for substituted alkyl.

The term "substituted" refers to the addition of a substituent group to a parent compound.

"Substituent groups" can be protected or unprotected and can be added to one available site or to many available sites in a parent compound. Substituent groups may also be further substituted with other substituent groups and may be attached directly or by a linking group such as an alkyl or hydrocarbyl group to a parent compound. "Substituent groups" useful in the context of alkyl or oxyalkyl substitution as disclosed herein include, without limitation, halogen, oxygen, nitrogen, sulphur, hydroxyl, alkyl, alkenyl, alkynyl, acyl (-C(O)$R^a$), carboxyl (-C(O)O$R^a$), aliphatic groups, alicyclic groups, alkoxy, substituted oxy (-O$R^a$), aryl, aralkyl, heterocyclic radical, heteroaryl, heteroarylalkyl, amino (-N($R^b$)($R^c$)), imino(=N$R^b$), amido (-C(O)N($R^b$)($R^c$) or -N($R^b$)C(O)$R^a$), hydrazine derivates (-C(NH)N$R^a$$R^b$), tetrazole($CN_4H_2$), azido (-$N_3$), nitro (-$NO_2$), cyano (-CN), isocyano (-NC), cyanato (-OCN), isocyanato (-NCO), thiocyanato (-SCN); isothiocyanato (-NCS); carbamido (-OC(O)N($R^b$)($R^c$) or -N($R^b$)C(O)O$R^a$), thiol (-S$R^b$), sulfinyl (-S(O)$R^b$), sulfonyl (-S(O)$_2R^b$), sulfonamidyl (-S(O)$_2$N($R^b$)($R^c$)or -N($R^b$)S(O)$_2R^b$) and fluorinated compounds -$CF_3$, -$OCF_3$, -$SCF_3$, -$SOCF_3$ or -$SO_2CF_3$, wherein each $R^a$, $R^b$ and $R^c$ is, independently from any other $R^a$, $R^b$ and $R^c$, H or a further substituent group with a preferred list including without limitation, $C_1$-$C_3$ alkyl alkyl, $C_2$-$C_3$ alkenyl, $C_2$-$C_3$ alkynyl, alkoxy, $C_1$-$C_3$ acyl, aryl, heteroaryl, alicyclic, heterocyclic and heteroarylalkyl.

The terms *unsubstituted $C_n$ alkyl* and *substituted $C_n$ alkyl* include a linear alkyl comprising or being linked to a cyclical structure, for example a cyclopropane, cyclobutane, cyclopentane or cyclohexane moiety, unsubstituted or substituted depending on the annotation or the context of mention, having linear alkyl substitutions. The total number of carbon and -where appropriate- N, O or other hetero atom in the linear chain or cyclical structure adds up to n.

Where used in the context of chemical formulae, the following abbreviations may be used: *Me* is methyl $CH_3$, *Et* is ethyl -$CH_2CH_3$, *Prop* is propyl -$(CH_2)_2CH_3$ (n-propyl, n-pr) or -$CH(CH_3)_2$ (iso-propyl, i-pr), *but* is butyl -$C_4H_9$, -$(CH_2)_3CH_3$, -$CHCH_3CH_2CH_3$, -$CH_2CH(CH_3)_2$ or - $C(CH_3)_3$.

The term *substituted alkyl* in its broadest sense refers to an alkyl as defined above in the broadest sense, which is covalently linked to an atom that is not carbon or hydrogen, particularly to an atom selected from N, O, F, B, Si, P, S, Cl, Br and I, which itself may be -if applicable- linked to one or several other atoms of this group, or to hydrogen, or to an unsaturated or saturated hydrocarbon (alkyl or aryl in their broadest sense). In a narrower sense, *substituted alkyl* refers to an alkyl as defined above in the broadest sense that is substituted in one or several carbon atoms by groups selected from amine $NH_2$, alkylamine NHR, imide NH, alkylimide NR, amino(carboxyalkyl) NHCOR or NRCOR, hydroxyl OH, oxyalkyl OR, oxy(carboxyalkyl) OCOR, carbonyl O and its ketal or acetal $(OR)_2$, nitril CN, isonitril NC, cyanate CNO, isocyanate NCO, thiocyanate CNS, isothiocyanate NCS, fluoride F, choride Cl, bromide Br, iodide I, phosphonate $PO_3H_2$, $PO_3R_2$, phosphate $OPO_3H_2$ and $OPO_3R_2$, sulfhydryl SH, suflalkyl SR, sulfoxide SOR, sulfonyl $SO_2R$, sulfanylamide $SO_2NHR$, sulfate $SO_3H$ and sulfate ester $SO_3R$, wherein the R substituent as used in the current paragraph, different from other uses assigned to R in the body of the specification, is itself an unsubstituted or substituted $C_1$ to $C_{12}$ alkyl in its broadest sense, and in a narrower sense, R is methyl, ethyl or propyl unless otherwise specified.

The term *amino substituted alkyl or hydroxyl* substituted alkyl relates to an alkyl according to the above definition that is modified by one or several amine or hydroxyl groups $NH_2$, NHR, $NR_2$ or OH, wherein the R substituent as used in the current paragraph, different from other uses assigned to R in the body of the specification, is itself an unsubstituted or substituted $C_1$ to $C_{12}$ alkyl in its broadest sense, and in a narrower sense, R is methyl, ethyl or propyl unless otherwise

specified. An alkyl having more than one carbon may comprise more than one amine or hydroxyl. Unless otherwise specified, the term "substituted alkyl" refers to alkyl in which each C is only substituted by at most one amine or hydroxyl group, in addition to bonds to the alkyl chain, terminal methyl, or hydrogen.

The term *carboxyl substituted alkyl* refers to an alkyl according to the above definition that is modified by one or several carboxyl groups COOH, or derivatives thereof, particularly carboxylamides $CONH_2$, CONHR and $CONR_2$, or carboxylic esters COOR, with R having the meaning as laid out in the preceding paragraph and different from other meanings assigned to R in the body of this specification.

Non-limiting examples of amino-substituted alkyl include $-CH_2NH_2$, $-CH_2NHMe$, $-CH_2NHEt$, $-CH_2CH_2NH_2$, $-CH_2CH_2NHMe$, $-CH_2CH_2NHEt$, $-(CH_2)_3NH_2$, $-(CH_2)_3NHMe$, $-(CH_2)_3NHEt$, $-CH_2CH(NH_2)CH_3$, $-CH_2CH(NHMe)CH_3$, $-CH_2CH(NHEt)CH_3$, $-(CH_2)_3CH_2NH_2$, $-(CH_2)_3CH_2NHMe$, $-(CH_2)_3CH_2NHEt$, $-CH(CH_2NH_2)CH_2CH_3$, $-CH(CH_2NHMe)CH_2CH_3$, $-CH(CH_2NHEt)CH_2CH_3$, $-CH_2CH(CH_2NH_2)CH_3$, $-CH_2CH(CH_2NHMe)CH_3$, $-CH_2CH(CH_2NHEt)CH_3$, $-CH(NH_2)(CH_2)_2NH_2$, $-CH(NHMe)(CH_2)_2NHMe$, $-CH(NHEt)(CH_2)_2NHEt$, $-CH_2CH(NH_2)CH_2NH_2$, $-CH_2CH(NHMe)CH_2NHMe$, $-CH_2CH(NHEt)CH_2NHEt$, $-CH_2CH(NH_2)(CH_2)_2NH_2$, $-CH_2CH(NHMe)(CH_2)_2NHMe$, $-CH_2CH(NHEt)(CH_2)_2NHEt$, $-CH_2CH(CH_2NH_2)_2$, $-CH_2CH(CH_2NHMe)_2$ and $-CH_2CH(CH_2NHEt)_2$ for terminal moieties and $-CH_2CHNH_2-$, $-CH_2CHNHMe-$, $-CH_2CHNHEt-$ for an amino substituted alkyl moiety bridging two other moieties.

Non-limiting examples of hydroxy-substituted alkyl include $-CH_2OH$, $-(CH_2)_2OH$, $-(CH_2)_3OH$, $-CH_2CH(OH)CH_3$, $-(CH_2)_4OH$, $-CH(CH_2OH)CH_2CH_3$, $-CH_2CH(CH_2OH)CH_3$, $-CH(OH)(CH_2)_2OH$, $-CH_2CH(OH)CH_2OH$, $-CH_2CH(OH)(CH_2)_2OH$ and $-CH_2CH(CH_2OH)_2$ for terminal moieties and -CHOH-, $-CH_2CHOH-$, $-CH_2CH(OH)CH_2-$, $-(CH_2)_2CHOHCH_2-$, - $CH(CH_2OH)CH_2CH_2-$, $-CH_2CH(CH_2OH)CH_2-$, $-CH(OH)(CH_2CHOH-$, $-CH_2CH(OH)CH_2OH$, - $CH_2CH(OH)(CH_2)_2OH$ and $-CH_2CHCH_2OHCHOH-$ for a hydroxyl substituted alkyl moiety bridging two other moieties.

The term *halogen-substituted alkyl* refers to an alkyl according to the above definition that is modified by one or several halogen atoms selected (independently) from F, Cl, Br, I.

The term *fluoro substituted alkyl* refers to an alkyl according to the above definition that is modified by one or several fluoride groups F. Non-limiting examples of fluoro-substituted alkyl include $-CH_2F$, $-CHF_2$, $-CF_3$, $-(CH_2)_2F$, $-(CHF)_2H$, $-(CHF)_2F$, $-C_2F_5$, $-(CH_2)_3F$, $-(CHF)_3H$, $-(CHF)_3F$, $-C_3F_7$, $-(CH_2)_4F$, $-(CHF)_4H$, $-(CHF)_4F$ and $-C_4F_9$.

Non-limiting examples of hydroxyl- and fluoro-substituted alkyl include $-CHFCH_2OH$, $-CF_2CH_2OH$, $-(CHF)_2CH_2OH$, $-(CF_2)_2CH_2OH$, $-(CHF)_3CH_2OH$, $-(CF_2)_3CH_2OH$, $-(CH_2)_3OH$, $-CF_2CH(OH)CH_3$, $-CF_2CH(OH)CF_3$, $-CF(CH_2OH)CHFCH_3$, and $-CF(CH_2OH)CHFCF_3$.

The term *aryl* in the context of the present specification relates to a cyclic aromatic $C_5$-$C_{10}$ hydrocarbon that may comprise a heteroatom (e.g. N, O, S). Examples of aryl include, without being restricted to, phenyl and naphthyl, and any heteroaryl. A heteroaryl is an aryl that comprises one or several nitrogen, oxygen and/or sulphur atoms. Examples for heteroaryl include, without being restricted to, pyrrole, thiophene, furan, imidazole, pyrazole, thiazole, oxazole, pyridine, pyrimidine, thiazin, quinoline, benzofuran and indole. An aryl or a heteroaryl in the context of the specification additionally may be substituted by one or more alkyl groups.

An *aryl methylene* in the context of the present specification relates to a $CH_2$ (-methylene) group substituted by an aryl moiety. One non-limiting example of aryl methylene is a benzyl (Bn) group. If used in particular, a heteroaryl methylene in the context of the present specification signifies a $CH_2$ (-methylene) group substituted by a heteroaryl moiety. Examples for heteroaryl include, without being restricted to, pyrrole, thiophene, furan, imidazole, pyrazole, thiazole, oxazole, pyridine, pyrimidine, thiazin, quinoline, benzofuran and indole. An aryl or a heteroaryl in the context of this specification additionally may be substituted by one or more alkyl groups.

A first aspect of the invention relates to a method of producing a pluripotent stem cell, in particular an induced pluripotent stem cell. This method comprises contacting a non-pluripotent donor cell obtained from a mammalian donor with a compound characterized by general formula (1).

Certain embodiments of the present invention do not encompass the manipulation of cells in-vivo, but is practiced in-vitro, and does not relate to the manipulation of primates, particularly humans, or to the manipulation of primate, particularly human,embryos.

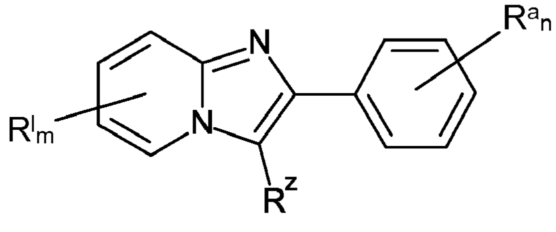

(1),

The substituent $R^l$ is attached to the six-membered ring of the pyrimidazole (imidazo[1 ,2-A]pyridine) moiety.

The point of attachment might be any of the four carbon atoms available for substitution. The index m of $R^I_m$ indicates the number of substituents that are attached to the pyrimidazole moiety. The value of m is 1 or 2.

The substituent $R^I$ is selected from -H, substituted or unsubstituted $C_1$-$C_3$ alkyl, substituted or unsubstituted $C_1$-$C_3$ alkoxy, -F, -Cl, -Br, -I, $-NO_2$, -OH, $-NH_2$, $-SO_3H$, -CN, substituted or unsubstituted $C_1$-$C_3$ carboxylic acid, -NCO, -CNO, -NCS, -SCN, $-CH_2F$, $-CHF_2$, or $-CF_3$.

In certain embodiments, the substituents are selected from halogen, oxygen, nitrogen, sulphur and hydroxyl.

The substituent $R^z$ is selected from general formula (2) or hydrogen.

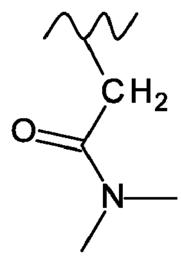

(2)

In certain embodiments, the compound according to aspect 1 is zolpidem (CAS No. 82626-48-0), with m of $R^I_m$ being 1, $R^I$ being $CH_3$, $R^z$ being general formula (2), n of $R^a_n$ being 1 and $R^a$ being $CH_3$.

The substituent $R^a$ is attached to the aryl moiety. The point of attachment might be any of the six carbon atoms of the aryl moiety. The index n of $R^a_n$ indicates the number of substituents that are attached to the aryl moiety. The value of n is 1 or 2.

The substituent $R^a$, independently from each other $R^a$, is selected from -H, substituted or unsubstituted $C_1$-$C_3$ alkyl, substituted or unsubstituted $C_1$-$C_3$ alkoxy, -F, -Cl, -Br, -I, $-NO_2$, -OH, - $NH_2$, $-SO_3H$, -CN, substituted or unsubstituted $C_1$-$C_3$ carboxylic acid, -NCO, -CNO, -NCS, -SCN, - $CH_2F$, $-CHF_2$, or $-CF_3$. $R^a$ may also take any of the values given for $R^I$, as well.

In certain embodiments, two $R^a$ bonded to two adjacent carbon atoms in the aryl moiety form a circular structure via the hydrocarbon $C_4H_4$ (the moiety attached to the imidazole ring is a naphtyl)

The carbon atom of the aryl moiety that is linked to the imidazole moiety is not substituted by $R^a$.

In certain embodiments, the value of m is 1.

In certain embodiments, the value of m is 2.

In certain embodiments, the value of n is 1.

In certain embodiments, the value of n is 2.

In certain embodiments, $R^I$ is methyl.

In certain embodiments, $R^a$ is selected from -H, $-CH_3$, -F, -Cl, -Br, -I, -OH, $-NO_2$, -CN or -OMe.

In certain embodiments, $R^a$ is methyl.

In certain embodiments, the compound used in the method according to the first aspect of the invention is characterized by general formula (3).

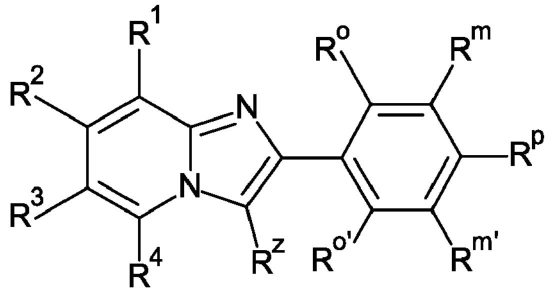

(3)

One of $R^1$, $R^2$, $R^3$ and $R^4$ is selected from: -H, substituted or unsubstituted $C_1$-$C_3$ alkyl, substituted or unsubstituted $C_1$-$C_3$ alkoxy, -F, -Cl, -Br, -I, $-NO_2$, -OH, $-NH_2$, $-SO_3H$, -CN, substituted or unsubstituted $C_1$-$C_3$ carboxylic acid, -NCO, -CNO, -NCS, -SCN, $-CH_2F$, $-CHF_2$, or $-CF_3$. The other ones are -H. In other words, only one of the substituents $R^1$, $R^2$, $R^3$ and $R^4$ is bonded to the pyridine moiety. The meaning of the substituents is according to the definition provided above.

The substituent $R^z$ is selected from general formula (2) or hydrogen.

One of $R^o$, $R^m$, $R^p$, $R^{m'}$ and $R^{o'}$ is selected from: -H, substituted or unsubstituted $C_1$-$C_3$ alkyl, substituted or unsubstituted $C_1$-$C_3$ alkoxy, -F, -Cl, -Br, -I, $-NO_2$, -OH, $-NH_2$, $-SO_3H$, -CN, substituted or unsubstituted $C_1$-$C_3$ carboxylic acid, -NCO, -CNO, -NCS, -SCN, $-CH_2F$, $-CHF_2$, or $-CF_3$. The other ones are -H. In other words, only one of the substituents $R^o$, $R^m$, $R^p$, $R^{m'}$ and $R^{o'}$ is bonded to the aryl moiety. The meaning of the substituents is according to the definition provided above.

In certain embodiments, any two neighbouring positions of $R^o$, $R^m$, $R^p$, $R^{m'}$ or $R^{o'}$ together form a circular structure via the hydrocarbon $C_4H_4$.

In certain embodiments, $R^m$ and $R^p$ together form a circular structure via the hydrocarbon $C_4H_4$.

In certain embodiments, each $R^l$, $R^a$, $R^1$, $R^2$, $R^3$, $R^4$, $R^o$, $R^m$, $R^p$, $R^{m'}$ and $R^{o'}$ is selected independently from each other from -H, $-CH_3$, -F, -Cl, -Br, -I, -OH, $-NO_2$, -CN or -OMe.

In certain embodiments, $R^1$ is selected from $-CH_3$, -H.

In certain embodiments, $R^2$ is selected from $-CH_3$, -H.

In certain embodiments, $R^3$ is selected from $-CH_3$, -H, -F, -Cl, -Br.

In certain embodiments, $R^4$ is selected from $-CH_3$, -H.

In certain embodiments, $R^{o'}$ is selected from $-NO_2$, -F, -H.

In certain embodiments, $R^o$ is selected from $-NO_2$, -F, -H.

In certain embodiments, $R^p$ is selected from $-CH_3$, -F, -Cl, -Br, $-NO_2$, -OMe, -OH, -CN, -H.

In certain embodiments, $R^m$ is selected from -Cl, -H, -F, -OH, $-NO_2$, -OMe.

In certain embodiments, $R^{m'}$ is selected from -Cl, -H, -F, -OH, $-NO_2$, -OMe.

In certain embodiments, $R^z$ is selected from -H or formula (2).

In certain embodiments, the compound is N,N-dimethyl-2-(6-methyl-2-p-tolylimidazo[1,2-a]pyridin-3-yl)acetamid (100).

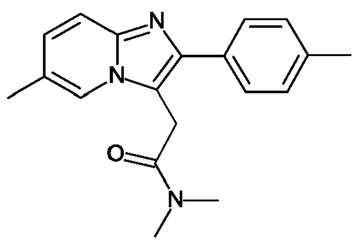

(100)

[Zolpidem; CAS No. 82626-48-0].

In certain embodiments, the compound is 6-methyl-2-(p-tolyl)imidazo[1,2-a]pyridine (101).

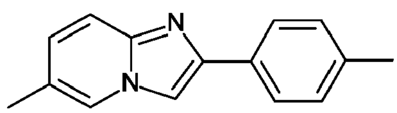

(101)

[O4I3]

In certain embodiments, the compound is 2-(4-fluorophenyl)-6-methyl-imidazo[1,2-a]pyridine (102).

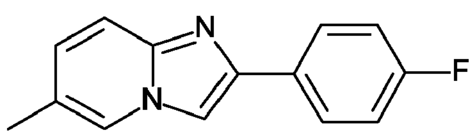

(102)

In certain embodiments, the compound is 2-(4-chlorophenyl)-6-methyl-imidazo[1,2-a]pyridine (103).

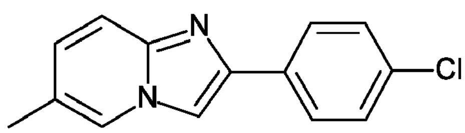

(103)

In certain embodiments, the compound is 2-(4-bromophenyl)-6-methyl-imidazo[1,2-a]pyridine (104).

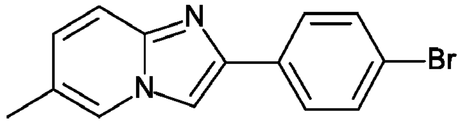

(104)

In certain embodiments, the compound is 6-methyl-2-(4-nitrophenyl)imidazo[1,2-a]pyridine (105).

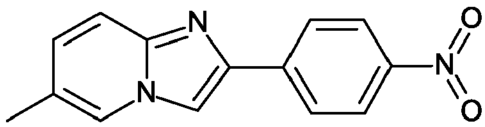

(105)

In certain embodiments, the compound is 2-(4-methoxyphenyl)-6-methyl-imidazo[1,2-a]pyridine (106).

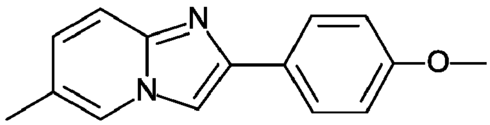

(106)

In certain embodiments, the compound is 4-(6-methylimidazo[1,2-a]pyridin-2-yl)phenol (107).

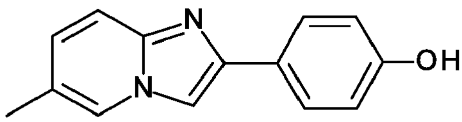

(107)

In certain embodiments, the compound is 4-(6-methylimidazo[1,2-a]pyridin-2-yl)benzonitrile (108).

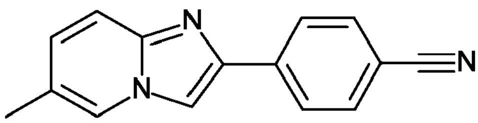

(108)

In certain embodiments, the compound is 6-methyl-2-phenyl-imidazo[1,2-a]pyridine (109).

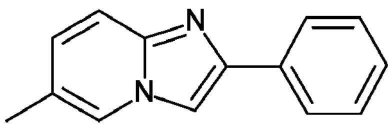

(109)

In certain embodiments, the compound is 6-methyl-2-(3-nitrophenyl)imidazo[1,2-a]pyridine (110).

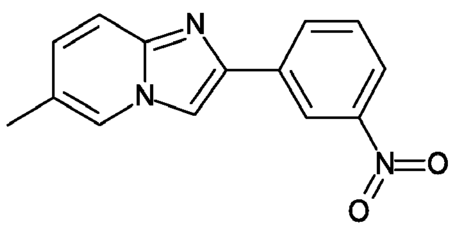

(110)

In certain embodiments, the compound is 6-methyl-2-(2-nitrophenyl)imidazo[1,2-a]pyridine (111).

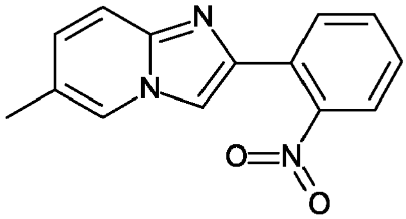

(111)

In certain embodiments, the compound is 2-(3-fluorophenyl)-6-methyl-imidazo[1,2-a]pyridine (112).

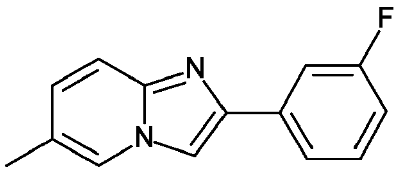

(112)

In certain embodiments, the compound is 2-(2-fluorophenyl)-6-methyl-imidazo[1,2-a]pyridine (113).

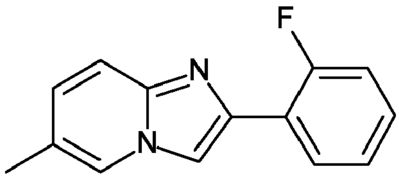

(113)

In certain embodiments, the compound is 2-(3-chlorophenyl)-6-methyl-imidazo[1,2-a]pyridine (114).

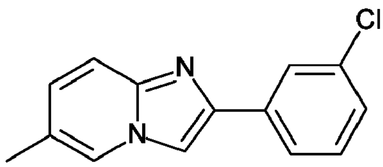

(114)

In certain embodiments, the compound is 3-(6-methylimidazo[1,2-a]pyridin-2-yl)phenol (115).

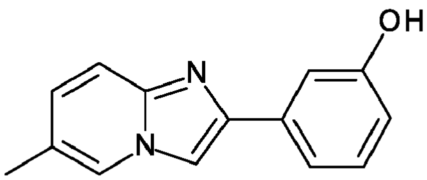

(115)

In certain embodiments, the compound is 2-(3-methoxyphenyl)-6-methyl-imidazo[1,2-a]pyridine (116).

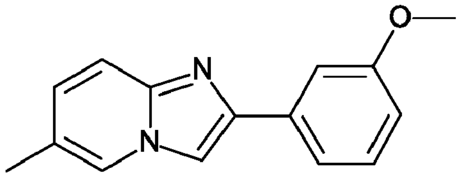

(116)

In certain embodiments, the compound is 7-methyl-2-(p-tolyl)imidazo[1,2-a]pyridine (117).

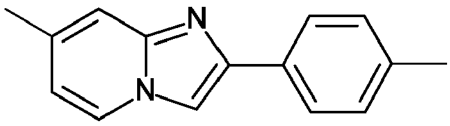

(117)

In certain embodiments, the compound is 8-methyl-2-(p-tolyl)imidazo[1,2-a]pyridine (118).

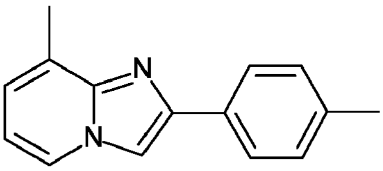

(118)

In certain embodiments, the compound is 5-methyl-2-(p-tolyl)imidazo[1,2-a]pyridine (119).

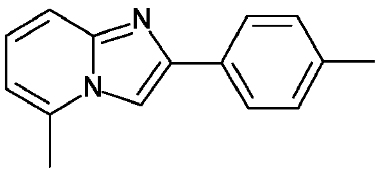

(119)

In certain embodiments, the compound is 6-chloro-2-(p-tolyl)imidazo[1,2-a]pyridine (120).

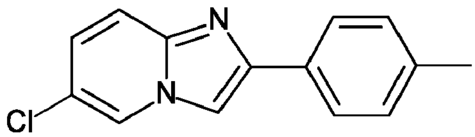

(120)

In certain embodiments, the compound is 6-fluoro-2-(p-tolyl)imidazo[1,2-a]pyridine (121)

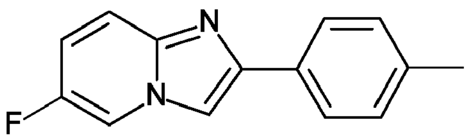

(121)

In certain embodiments, the compound is 6-bromo-2-(p-tolyl)imidazo[1,2-a]pyridine (122).

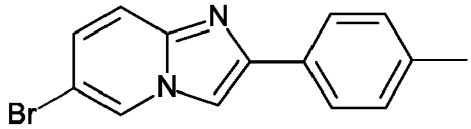

(122)

In certain embodiments, the compound is 6-methyl-2-(2-naphthyl)imidazo[1,2-a]pyridine (123).

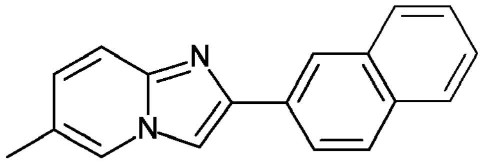

(123)

An alternative of this first aspect of the invention relates to a method of producing a pluripotent stem cell, in particular a method of producing an induced pluripotent stem cell, wherein the method comprises contacting a non-pluripotent donor cell obtained from a mammalian (particularly a human) donor with a compound characterized by general formula (4):

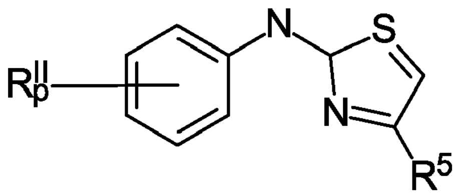

(4),

Therein, each $R^{II}$**,** independently from any other $R^{II}$, is selected from

- -H,
- substituted or unsubstituted $C_1$-$C_3$ alkyl, particularly methyl,
- substituted or unsubstituted $C_1$-$C_3$ alkoxy, particularly O-$CH_3$,
- -F, -Cl, -Br, -I, -$NO_2$, -OH, -$NH_2$, -$SO_3H$, -CN, -NCO, -CNO, -NCS, -SCN, particularly -F, -Cl, -Br, -$NO_2$, -OH, and -CN,
- substituted or unsubstituted $C_1$-$C_3$ carboxylic acid, -$CH_2F$, -$CHF_2$, or -$CF_3$, particularly COOH.

p is 1, 2 or 3, particularly p is 1 or 2, and $R^5$ is selected from

- -H,
- substituted or unsubstituted $C_1$-$C_4$ alkyl, particularly t-butyl,
- substituted or unsubstituted aryl, particularly unsubstituted phenyl or naphtyl;
- COOR, wherein R is an unsubstituted or substituted $C_1$-$C_4$ alkyl, particularly R is an unsubstituted $C_1$-$C_4$ alkyl, more particularly R is ethyl;

The following combinations are not encompassed in the definition of compound (4):

- $R^5$ is COOR with R being an unsubstituted or substituted $C_1$-$C_4$ alkyl, particularly R is an unsubstituted $C_1$-$C_4$ alkyl, more particularly R is ethyl, and
- $R^{II}$ is halogen or hydrogen and p is selected from 1 or 2.

In certain embodiments, the compound according to formula (4) is the compound according to formula (5), (6) or (7):

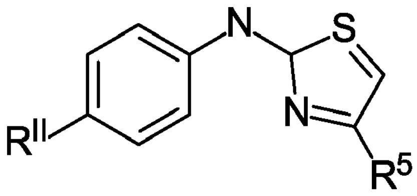

(5)

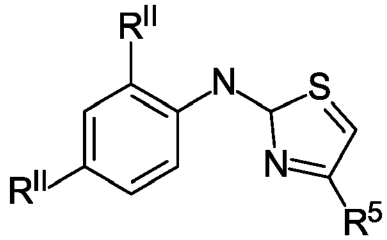

(6)

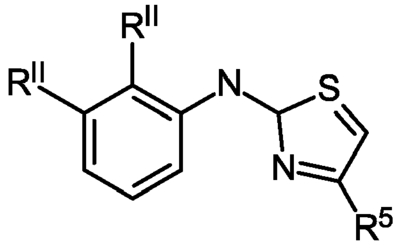

(7)

wherein $R^{II}$ and $R^5$ have the same meanings as indicated above.

In certain embodiments, each $R^{II}$, independently from any other $R^{II}$, is selected from methyl and ethyl. In certain particular embodiments, all $R^{II}$ are methyl.

In certain embodiments, $R^5$ is selected from substituted or unsubstituted $C_3$-$C_4$ alkyl. In certain particular embodiments, $R^5$ is unsubstituted t-butyl.

In certain embodiments, $R^5$ is selected from substituted or unsubstituted aryl. In certain particular embodiments, $R^5$ is unsubstituted phenyl or naphtyl.

In certain embodiments, the compound according to formula (4) is selected from any one of the formulae (419), (420), (421), (422) and (423). A particular embodiment of the method of the invention uses the compound according to formula (423)

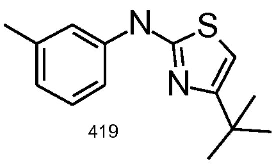

419

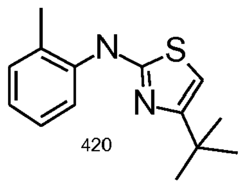

420

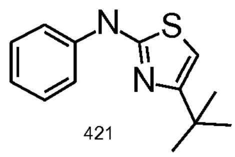

421

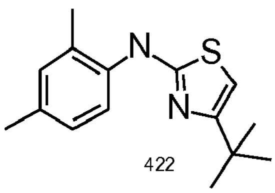

422

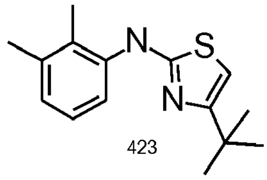

423

In certain embodiments, the mammalian donor is a human, in other words, a cell of human origin is modified.

In certain embodiments, the method according to the first aspect of the invention further comprises introducing into the mammalian non-pluripotent donor cell at least one element selected from:

- a nucleic acid sequence encoding a polypeptide,
- a polypeptide, and
- a non-coding nucleic acid sequence.

The polypeptide is selected from:

- the family of OCT polypeptides,
- the family of KLF polypeptides,

- the family of SOX polypeptides,
- the family of MYC polypeptides, or
- the family of LIN polypeptides.

In certain embodiments, the polypeptide is OCT4.
In certain embodiments, the polypeptide is KLF4.
In certain embodiments, the polypeptide is SOX2.
In certain embodiments, the polypeptide is L-MYC.
In certain embodiments, the polypeptide is Lin28.
The non-coding nucleic acid sequence is selected from: miR-302b, miR-372, miR-302, miR-367, miR-200c and miR-369.
In certain embodiments, a multitude of nucleic acid sequences encoding a polypeptide, polypeptides or non-coding nucleic acid sequences are introduced into the mammalian non-pluripotent donor cell. In other words, one "element" can comprise several nucleic acid sequences or polypeptides within the same category of the "element".
In certain embodiments, the compound is used in a final concentration between 0.0005 μmol/l and 100 μmol/l.
In certain embodiments, the compound is used in a final concentration between 0.001 μmol/l and 15 μmol/l.
In certain embodiments, the compound is used in a final concentration between 0.001 μmol/l and 10 μmol/l.
A second aspect of the invention relates to a method of inducing OCT4 and NANOG in a mammalian cell. The method comprises contacting a mammalian donor cell with a compound according to the first aspect of the invention.
Another (third) aspect of the invention relates to a method of increasing methylation of histone 3 lysine 4 (H3K4) in a mammalian non-pluripotent donor cell to augment a method of producing an induced pluripotent stem cell. This method comprises contacting the donor cell with a compound according to the first aspect of the invention or any of its embodiments enclosed herein.
It is known in the art that fibroblasts may become resistant to reprogramming approaches to induce pluripotency. One condition known to increase the capacity of a donor cell to be reprogrammed is the maintenance of an open chromatin state that allows for transcription factor binding. In this context trimethylation of histone H3 lysine 4 (H3K4Me3) is reported to provide such an open chromatin state (A. Soufi, G. Donahue and K. S. Zaret, Cell, 2012, 151, 994-1004; Koche, et al., Cell Stem Cell, 2011, 8, 96-105; Polo and Hochedlinger, Cell Stem Cell, 2010, 7, 5-6).
According to an alternative aspect of the invention, a method for augmenting a method of producing an induced pluripotent stem cell is provided. The method is comprising contacting the donor cell with a compound according to the first aspect of the invention or any of its embodiments enclosed herein.
In certain embodiments according to the first, second and third aspect of the invention, a compound is used in its final concentration for a duration of 12 hours to 10 days.
In certain embodiments according to the first, second and third aspect of the invention, a compound is used in its final concentration for a duration of 1 day to 8 days.
In certain embodiments according to the first, second and third aspect of the invention, a compound is used in its final concentration for a duration of 2 days to 7 days.
In certain embodiments according to the first, second and third aspect of the invention, a compound is used in its final concentration for a duration of 2 to 8 months.
A fourth aspect of the invention relates to a method of maintaining pluripotency in mammalian pluripotent stem cells. This method comprises contacting the mammalian pluripotent stem cell with a compound according to the first aspect of the invention or any of its embodiments enclosed herein.
In certain embodiments, the mammalian pluripotent stem cell is a multipotent neural stem cell.
In certain embodiments of any aspects of the invention, the method is practiced *in vitro* or *ex vivo*.
In certain embodiments, according to all aspects of the invention the compound is used in a final concentration between 0.0005 μmol/l and 100 μmol/l.
In certain embodiments, according to all aspects of the invention the compound is used in a final concentration between 0.001 μmol/l and 15 μmol/l.
In certain embodiments, according to all aspects of the invention the compound is used in a final concentration between 0.001 μmol/l and 10 μmol/l.
A fifth aspect of the invention relates to an induced pluripotent mammalian stem cell obtained or obtainable by a method according to any one of the other aspects of the invention.
A sixth aspect of the invention relates to a cell culture medium comprising a compound according to the first aspect of the invention.
In certain embodiments, the compound is used in a final concentration between 0.0005 μmol/l and 100 μmol/l.
In certain embodiments, the compound is used in a final concentration between 0.001 μmol/l and 15 μmol/l.
In certain embodiments, the compound is used in a final concentration between 0.001 μmol/l and 10 μmol/l.
A seventh aspect of the invention relates to a composition for promoting formation of induced pluripotent

mammalian stem cells. The composition is comprising a compound according to the first aspect of the invention.

The invention is further illustrated by the following examples and figures, from which further embodiments and advantages can be drawn. These examples are meant to illustrate the invention but not to limit its scope.

*Brief description of the figures*

Fig. 1 shows the validation of activity of O4I3 (101) in luciferase assays. A) Synthesis of O4I3 (101). B) Relative luciferase activity in NCCIT-Oct4 cells treated with increasing concentrations of 0413 for 48 h. The data were collected from at least five independent experiments. C) Relative luciferase activity in NCCIT-Nanog cells. Data are represented as mean ± SD from at least 20 independent experiments ***: p<0.001; **: p<0.01; *: p<0.05. D) Continuous activation of Oct4 in NCCIT-Oct4 reporter cells at indicated days (D1, D2, D3 and D4). ***: p<0.001; **: p<0.01; *: p<0.05.

Fig. 2 shows that 0413 supports maintenance of human induced pluripotent stem cells (hiPSCs). A) 0413 promoted the viability of hiPSCs in single cell expansion. B) Homogeneous expression of Tra-1-80 and Oct4 up to passage (p) 30 in hiPSCs treated with 50 nM 0413 analyzed by FACS C) Relative fold changes to non-treatment in hiPSCs treated with O4I3 (50 nM) for 24 h analyzed by qRT-PCR. D) Gene expression profiling analysis of functional annotation enrichment for categories related to pluripotency, development and homeostasis in O4I3-treated iPSCs for 24 h. Data are represented as mean ± SD from at least 3 independent experiments ***: p<0.001; **: p<0.01; *: p<0.05.

Fig. 3 shows that 0413 facilitates the conversion of fibroblasts into hiPSCs. A) Induction of Oct4 and B) Nanog expression in human fibroblasts (HF2) treated with 0413 (0.5 μM) at day 1 (1D), day 3 (3D), day 5 (5D) and day 7 (7D) detected by qRT-PCR. C) O4I3 increased the formation of AP+ colonies in episome-based reprogramming of four human fibroblasts.

Fig. 4 shows cell identity of newly generated hiPSCs. A) Established hiPSC cell lines showing hESC-like morphologies. B) Expression of differentiation markers of three germ layers detected by qRT-PCR. C) Comparison of E-Cadherin and Tra-1-60 expression by FACS in HF2 and iPSCs. D) pluripotency-associated gene expression by immunoblotting. NCCIT and PSC1/2/3 were used as positive control. E) Pairwise comparison of log2- transformed gene expression using scatter plots. Red dashed lines depict the two-fold change in expression values. F) Heatmap of expression profiles of selected pluripotency-associated genes (bright green label) and fibroblast-associated genes (dark green label) expression. The cells in both heatmaps depict log2 transformed gene expression values, centred on the gene-specific median expression across all samples. Genes and samples are reordered using hierarchical clustering based on complete linkage and Euclidean distance.

Fig. 5 shows that 0413 facilitates the binding of H3K4Me3 to promoter of Oct4 in human fibroblasts. A) Venn diagram for comparison of up- and down-regulated gene expression in 0413 (500 nM) treated human fibroblasts (HFF) with ectopic expression of Oct4, Sox2, Klf4 and Myc (OSKM) as well as hiPSCs. HFF treated with O4I3; HFF transfected with episomal OSKM; hiPSCs. B) Induction of H3K4Me4 by 0413 in HFFs detected by immunoblotting. C) Inhibitors and D) activators of reprogramming, E) Enrichment of H3K4Me3 at the promoter of Oct4 analyzed by Chip-qPCR. The data were normalized to iPSCs.

Fig. 6 shows activity in NCCIT-Oct4 reporter cells treated with derivatives. A) Activity of compounds 100 to 123 relative to treatment with compound 101 (O4I3). B) Activity of compounds 100 to 123 relative to control treatment (DMSO). Data are represented as mean ± SD from at least 4 independent experiments.

Fig. 7 shows the maintenance of neuronal stem cells. Neural stem cells were cultivated in SPN medium (DMEM-F12 Knockout medium containing 2% StemPro® NSC SFM, EGF (20 ng/mL) and FGF (20 ng/mL)) until cell confluence reached 80-90%. Acutase was used to get single cell suspension and washed with PBS. 20,000 cells/cm$^{-1}$ were seeded into matrigel-coated plates using SPN medium with or without O4I3. The MTT assay was applied to determine cell viability.

Fig. 8 shows the luciferase activity in NCCIT-Oct4 cells (luciferase activity is driven by the Oct4-promoter) treated with increasing concentrations of zolpidem (Zo) for 48 hours.

Fig. 9 shows the luciferase activity in NCCIT-Nanog cells (luciferase activity is driven by the Nanog-promoter) treated

with increasing concentrations of zolpidem (Zo) for 48 hours.

Fig. 10 shows the microarray analysis on the phosphorylation level of reprogramming-related proteins in NCCIT or JoPaca-1 cells. Cells were analysed in the presence (24 hours 0413 1μM) or absence (NT) of O4I3.

Fig. 11 shows induction of differentiation markers in hPSCs treated with 0413 at the higher concentration (10 μM).

Fig. 12 shows comparative qRT-PCR analysis of reprogramming related gene expression in pluripotent stem cells (PSC), human fibroblasts (HF2) and human fibroblasts treated with 0413 (0.5 μM) at day 1 (O4I3 1D), day 3 (O4I3 3D), day 5 (O4I3 5D) and day 7 (O4I3 7D).

Fig. 13 shows that 0413 (50 nM) increases the formation of alkaline phosphatase positive (AP+) colonies in episome-based reprogramming of human fibroblasts (HF 1 and HF2). 1 and 2 AP+ colonies were obtained by using episome-based reprogramming in HF5 in the presence of Zolpidem (10 μM) from two independent experiments.

Fig. 14 shows the abundance of H3K4Me3 in O4I3-treated fibroblasts in the presence/absence of reprogramming factors Oct4, Sox2, Klf4 and Myc (OSKM). The fluorescent intensities of single cells selected by using ImageJ software are compared.

Fig. 15 shows functional enrichment analysis of differentially expressed genes in O4I3-treated HFF, including only the top ten Gene Ontology terms enriched in the sets of up-regulated and down-regulated genes, separately. The most enriched terms in the sets of common genes that are also differentially expressed in hPSC (as compared to HFF) was included.

Fig. 16 shows the activation of H3K4Me3. Levels of H3K4Me3 have been determined after indicated treatment and normalized to the level of iPSCs.

Fig. 17 0413 Is an Epigenetic Modulator. (A) Venn diagram of up- and downregulated genes in 0413 (50 nM)-treated human fibroblasts (HF) with ectopic expression of OCT4, SOX2, KLF4, and MYC (OSKM) as well as hiPSCs. HF-O4I3, HF cells treated with 0413 (50 nM for 24 h); HF-OSKM, HF cells transfected with episomal OSKM; iPSCs, the newly generated iPSCs. Differential expression was calculated with respect to HF cells. (B) Comparison of expression profiles of genes involved in epigenetic modification, chromatin remodeling, and related biological processes. (C) Occupation of H3K4Me3 or H3K27Me3 at the promoter of OCT4 in HF cells transfected with OSKM alone or in the presence of 50 nM of 0413 (O3-OSKM) for 3 days. (D) 0413 elevates the global levels of H3K4Me3 detected by immunocytochemistry. Cells were treated with 250 nM 0413 for 3 days. Scale bar, 40 mm. (E) Immunoblotting results show that O4I3 promotes global H3K4Me3 expression, whereas it represses H3K9Me3 protein levels in a concentration- and time-dependent manner. Cells were treated either with increasing concentrations of 0413 for 24 h or with 250 nM of 0413 for the indicated time points.

Fig. 18 0413 Is a Selective KDM5A Inhibitor. (A) Comparison of 0413 inhibitory effect on KDM5, LSD1, and KDM4 in vitro using the whole-cell nuclear extraction. (B) The inhibitory effect of 0413 on the members of KDM5 family of demethylases isolated from cells. (C) A selective KDM5A inhibitor JIB-04 induces OCT4 expression inNCCIT-OCT4 cells. Four histone demethylase inhibitors (HDMs), namely, CPI-455, JIB-04, GSK-J4, and daminozide, were incubated with NCCIT-OCT4 reporter cells for 48 h. (D) JIB-04 (5 mM) induces OCT4 expression in fibroblasts at the indicated time points (D, days). (E) Comparison of KDM5A and KDM5B expression levels in fibroblast (HF1), resistant fibroblast (HF4), HF4 transfected with OSKM, iPSCs, and NCCIT. (F) Knockdown of KDM5A (si5A) activates OCT4 in NCCIT-OCT4 reporter cells. Cells were transfected with various KDM5 siRNA concentrations for 48 h. (G) Transient knockdown (48 h) of KDM5A induces OCT4 and SOX2 expression in fibroblasts. (H) Overexpression of KDM5A (OE5A) compromises the effect of 0413 (100 nM, 48 h) in NCCIT-OCT4 and HEK-OCT4 cells. (I) The number of TRA-1-60-positive colonies was reduced in reprogramming-resistant fibroblasts with overexpression of KDM5A. In (C), (D), and (F) statistical significance was compared withmock (0.1% DMSO) treatment, in (G) with non-targeting siRNA and in (E) with HF4, whereas in (H) and (I) it was compared with OSKM-treated fibroblasts using two-way ANOVA followed by a post-hoc Tukey test. Data are represented as mean G SD. ***p<0.001, **p<0.01, *p<0.05.

Fig. 19 KDM5A Is a Reprogramming Barrier (A and B) (A) Suppression of KDM5A activity by siRNA or JIB-04 (5 mM, 48 h) promotes the enrichment of H3K4Me3 at the promoter of OCT4 in resistant fibroblasts, (B) but not that of H3K27Me3. Ct values were obtained from qRT-PCR. (C) Suppression of KDM5A activity by JIB-04 or KDM5A siRNA (siKA) induces POU5F1, SOX2, and CDH1mRNA levels during reprogramming, as determined by qRT-PCR.

(D) Correlation between reprogramming efficiency and KDM5A expression in HF1-HF11. The same amount of each cDNA was used as template for qRT-PCR where the Ct values indicate the expression of KDM5A. (E) Knockdown effects of increasing concentrations of anti-KDM5A siRNA (siKDM5A, 48 h) on H3K4Me3 expression levels in HF. (F) Reprogramming efficiency using various patient primary fibroblasts, as determined by the number of TRA-1-60-positive colonies. Cells were either transiently transfected with the indicated siRNA oligos in the first 5 days or treated with JIB-04 (5 mM, 48 h). siK5ABCD, anti-KDM5A/B/C/D siRNA; siK5AD, anti-KDM5A/D siRNA; siK5CD, anti-KDM5C/D siRNA; siK5A, anti-KDM5A siRNA; siK5B, anti-KDM5B siRNA; siK5C, anti-KDM5C siRNA; and siK5D, anti-KDM5D siRNA. In (A), (B), and (F) statistical significance was compared withOSKM-treated fibroblasts, whereas in (C) it was compared with DMSO (0.1%) treatment using two-way ANOVA and a post-hoc Tukey test. Data are represented as mean G SD. ***p<0.001, **p<0.01, *p<0.05.

Fig. 20 Metabolic stability is the key factor for maintaining the activity of O4I2 derivatives in PSCs. **A)** Synthesis of O4I2 derivatives. **B)** Structures of carboxylic derivatives. **C)** Relative luciferase activity in HEK-OCT4, NCCIT-OCT4 and NCCIT-NANOG reporter cell lines upon treatment with O4I2 ester derivatives. 0.2% DMSO was used as mock treatment. **D)** Metabolic analysis of O4I2 incubated with NCCIT for 24 hours. O4I2 and its metabolite O4I2-COOH were detected by HPLC (UV 254 nm, blue chromatogram) coupled to MS (Total ion chromatogram [TIC] indicated in black). Extracted ion chromatograms (EIC) of ions at *m/z* 255 (red, O4I2-COOH) and *m/z* 283 (green, 0412) are gated by their respective red and green boxes. **E)** Structures of non-carboxylic O4I2 derivatives. **F)** Activity of compounds **(15-23)** measured in the three reporter cell lines. Data are represented as mean values ± one SD from at least 10 independent experiments. *: p<0.05; **: p<0.01 and ***: p<0.001.

Fig. 21 Reprogramming of human fibroblasts without exogenous overexpression of OCT4. **A)** Illustration of hiPSCs generation without OCT4. HF1-LTR7-EGFP were pre-treated with 0413 (250 nM) for 2 days and then with O4I4 (20 μM) in combination with episomal expression of SKM (CSKM). **B)** GFP-positive colonies could be detected in HF1-LTR7-EGFP treated with O4I4 (20 μM) or in combination. **C)** The number of TRA-1-60 positive colonies in human fibroblasts (HF1, HF2 and HF3) treated with OSKM, SKM or CSKM. **D)** Comparison of relative Ct-vaules of OCT4 expression (DeltaCt = $Ct_{gene}$-$Ct_{actin}$) analyzed by qRT-PCR. Actin was used as control. **E)** Expression of OCT4 and SOX2 in CSKM-iPSCs detected by immunostaining. **F)** Expression of OCT4 and other pluripotency markers in CSKM. PSC1-3 and NCCIT were used as positive control, while HF1 as negative control. **G)** Hypomethylation of OCT4 promoter in CSKM-iPSCs detected by bisulfite sequencing. H) Comparison of global gene expression pattern of CSKM-iPSCs to PSC and HF1.

Fig. 22 Reprogramming of human fibroblasts without exogenous overexpression of OCT4. **A)** GFP-positive colonies could be detected in HF1-LTR7-EGFP treated with O4I4 (20 μM) or in combination. **B)** Illustration of hiPSCs generation without OCT4. HF1-LTR7-EGFP were pre-treated with 0413 (250 nM) for 2 days and then with O4I4 (5 μM) in combination with episomal expression of SKML (CSKML). The medium was refreshed every day. O4I4 was added in the first 7 days. E8 medium was used at day 10. **C)** The number of TRA-1-60 positive colonies in human fibroblasts (HF1, HF2 and HF3) treated with OSKML, SKML or CSKML. **D)** Comparison of relative Ct-vaules of OCT4 expression (DeltaCt = $Ct_{gene}$-$Ct_{actin}$) analyzed by qRT-PCR. Actin was used as control. **E)** Expression of OCT4 and SOX2 in CSKML-iPSCs detected by immunostaining. **F)** Expression of OCT4 and other pluripotency markers in CSKML. PSC1-3 and NCCIT were used as positive control, while HF1 as negative control. **G)** Hypomethylation of OCT4 promoter in CSKM-iPSCs detected by bisulfite sequencing. **H)** Comparison of global gene expression pattern of CSKML-iPSCs to PSC and HF1.

Fig. 23 ATAC-seq analysis for the comparison of chromatin accessibility in OSKML-mediated and CSKML-mediated reprogramming. **A)** Principal component comparison of HF1, HF1 treated with OSKML, HF1 treated with CSKML and iPSCs for 7 days, 14 days and 21 days. At 21 days TRA-1-60+ cells were collected for analysis. **B)** Comparison of global chromatin accessibility. **C)** Comparison of chromatin accessibility at the promoter region of OCT4.

Fig. 24 An essential role of HMGA1 in CSKML-mediated reprogramming. **A)** Heat map of top upregulated genes in O4I4-treated fibroblasts in comparison to those expressions in PSC. **B)** Comparison of chromatin accessibility at the promoter of HMGA1 in HF1 treated either with OSKML or CSKML for 7 days. **C)** Chemical Induction of HMGA1 is prior to OCT4 expression. HF1 was treated with 0413 (0.5 μM) and O4I4 (10 μM) over the indicated period. The medium was changed very day. **D)** HMAG1 and OCT4 are co-expressed in CSKML-treated fibroblasts. **E)** HMGA1 siRNA blocks CSKML-induced OCT4 expression. HF1 was treated as indicated. Cells were collected at day 7 for immunoblot analysis. **F)** HMGA1 knockdown inhibits OCT4 expression in both CSKML- and OSKML-mediated reprogramming. HMGA1 siRNA (siH) was co-transfected with SKML. Cells were collected at day 5 for immunoblot analysis and qPCR. **G)** Occupation of HMGA1 at the promoter of OCT4 detected by CHIP-qPCR. HF1 was transfected

with SKML. HMGA1 was co-transfected at day 0 and day 3. **H)** Comparison of the number of TRA-1-60 positive colony according to the day when HMGA1 was overexpressed. HMGA1 was transfected at indicated days and every 2-3 days the transfection was re-performed. **I)** Reprogramming efficiency of HSKML-induced reprogramming in three human fibroblasts calculated by the number of TRA-1-60 positive colony. HMGA1 was co-trasfected with SKML for 7 days. **: p<0.01 and ***: p<0.001.

Fig. 25 CSKML-mediated HMGA1 activation is dependent on BMP6-ID. **A)** Comparison of genes expression which up-regulated or down-regulated in CSKML-treated fibroblasts to these in SKML-treated obtained from RNA-Seq, as well as in O4I4-treated to mock treatment. The heat map shows genes up-regulated or down-regulated in both case. HF1 was treated with O4I4 (10 μM) or 0.1 % DMSO as mock, or transfected with SKML or in the combination (CSKML) for 48 h. The total RNA was collected for RNA-seq. **B)** CSKML activates BMP-SMAD-ID signalling pathway. BMP receptor inhibitor DM, BMP antagonist Noggin or SMAD4 siRNA blocks this effect. Dorsophormin (DM) or Noggin (200 ng/mL), or SMAD4 siRNA was added together with the treatment of CSKML for 48 h. C) Overexpression of ID1/2 enhances expression of HMGA1 and OCT4. HF1 was transfected with ID1/2 or in the combination with O4I3/4 or CSKML for 48 h. D) BMP6 enhances the expression of OCT4 induced by CSKML, which was blocked by ID1/2 siRNAs. BMP6 with/without ID1/2 siRNAs was added during the treatment with CSKML in the first 72 h. E) Adding BMP6 or overexpression of ID1/2 enhances the number of TRA-1-60 positive colony. **F)** Illustration of CSKML-mediated OCT4 expression.

## Examples

Drug repurposing provides a successful way to develop new therapies with known, medically approved drugs within remarkably short periods. The inventors intentionally sought for chemicals sharing backbones with approved drugs with the expectation of having advantages in pharmacodynamics and kinetics.

In an effort to find such chemical structures able to induce pluripotency, a high-throughput screening (HTS) was performed. The screen was based on cellular luciferase reporter assays under the control of the promoter activity of a pluripotency-associated transcription factor (Oct4) in HEK293 cells. About 4000 imidazopyridine derivatives with significantly differential luciferase activity were identified in this screen.

All methods and reagents used in these examples are disclosed below.

### *Synthesis of lead compound:*

To study the activating and inhibitory effects of the identified compounds in detail, one of the lead structures, 6-methyl-2-(p-tolyl)imidazo[1,2-a]pyridine (O4I3;(101)), was selected and synthesized. This was achieved by heating ω-bromoacetophenone with an excess of 2-aminopyridine for 3 h (Fig. 1A), achieving an excellent yield (>95%), because the product could be readily isolated from the reaction mixture according to distinct solubility under acidic conditions.

### *Effect of lead compound (0413) and Zolpidem on Oct4*

The ability of the lead compound to induce Oct4 at translational level was tested in a luciferase reporter cell line in human embryonic carcinoma NCCIT cells driven by Oct4 response elements (NCCIT-Oct4). The compound lead to a nearly 10-fold induction of Oct4 at 10 nM compared to untreated cells (Fig. 1B). Consequently, the lead compound was named "Oct4 inducing compound 3" (O4I3).

It is known in the art that Nanog is a direct target of Oct4 and can be activated by other Oct4 inducing compounds. Accordingly, a similar pattern of activation was observed in O4I3-treated NCCIT-Nanog reporter cells, carrying a Nanog response element (Fig. 1C). At higher concentrations of 0413 (Fig. 1B and 1C) less activity was observed. Moreover, the activity of zolpidem (N,N-Dimethyl-2-(6-methyl-2-p-tolylimidazo[1,2-a]pyridin-3-yl)acetamid) (100) containing a similar backbone to O4I3 was tested in both reporter cell lines. An approximate 2-fold induction at 10 μM was detected (Fig. 8 and Fig. 9). Notably, 0413 (1 nM) could achieve a comparable induction at a 10,000-fold lower concentration. Collectively, these results documented that 0413 and zolpidem activated Oct4 at both transcriptional and translational level.

### *Time course of Oct4 activation*

To address the question whether short or longer-term activation is achieved, the induction of Oct4 was monitored over several days in these experiments. A continuous and constant activation over 4 days was observed in NCCIT-Oct4 cells treated with 0413 (Fig. 1C). Without wishing to be bound by theory, the inventors believe that the lower activity at

higher concentrations might be caused by activation of certain signaling pathways antagonizing pluripotency. Indeed, excessive Oct4 expression can lead to differentiation of embryonic stem cells (ESCs) to extra-embryonic endoderm and mesoderm.

In good agreement, the inventors found the induction of those markers, T and Gata4, in hiPSCs treated with 10 μM 0413 (Fig. 11), suggesting that the differentiation was induced by 0413 at high concentrations.

*Effect on iPSC self renewal*

Since 0413 induced sustainable expression of Oct4 and Nanog, the inventors questioned if it supports iPSC self-renewal in single cell expansion. Therefore, the viability of iPSCs, after replating Accutase®-dissociated single iPSCs, was tested in the presence or absence of O4I3. Rocki (Y-27632) was used for the first 24 h to increase iPSCs survival. A nearly 300% higher viability in cells treated with O4I3 (1nM) was observed. Using 50 nM of 0413 even 400% increase was observed (Fig. 2A). FACS analysis confirmed homologous expression of iPSCs markers, Tra-1-81 and Oct4, for at least 30 passages (Fig. 2B).

*O4I3 maintains pluripotency of human iPSCs*

The cellular responses to O4I3 was analyzed in two stem cell lines, NCCIT and JoPaca-1 (Fig. 10). Using a phosphorylation microarray (Holenya et al, Proteomics. 2011 May;11(10):2129-33) revealed that 0413 selectively activated Erk1, an essential element for supporting mouse ESC self-renewal, but not Erk2, whose knockdown protects mouse ESC differentiation.

Moreover, inactive phosphorylation of GSK3Rß was observed. Without wishing to be bound by theory, the inventors believe that this is implicating activation of PI3K/Akt and Wnt, two signaling pathways that are fundamental for maintenance of human PSCs.

Inhibition of p38 and Src was also observed. Both are known in the art as constituents of chemical cocktails for conversion of human primed iPSCs to a naïve state.

In addition, zolpidem also sustained the maintenance of iPSCs, although at a much higher concentration and with lower efficacy than 0413 (Fig. 2A).

Therefore, the inventors investigated if 0413 can support human PSC self-renewal. The viability of previously well-characterized hiPSCs (thereafter called hPSCs) was investigated after replating Accutase®-dissociated single hPSCs in the presence/absence of O4I3, where Rocki (Y-27632) was used for the first 24 h to increase hPSCs survival. A nearly 300% higher viability was found in cells treated with 0413 even at 1 nM, and 400% at 50 nM (Fig. 2A). FACS analysis confirmed homologous expression of PSCs markers, Tra-1-81 and Oct4, for at least 30 passages under feeder free condition (Figure 2B). In addition, zolpidem also sustained the maintenance of iPSCs, although at a much higher concentration and with lower efficacy (Figure 2A).

Gene expression analysis by qRT-PCR showed that 0413 stabilized or slightly increased the expression of pluripotency-associated genes in iPSCs, including Oct4, Sox2, Nanog and Nodal, while expression of genes regulating differentiation was reduced, like Gata4, Gata6, Pax6 and Sox7 (Fig. 2C).

Functional annotation analysis of DNA microarray profiles of gene expression in O4I3-treated iPSCs showed that 0413 inhibited cell differentiation, while maintained cellular homeostasis (Fig. 2D). Thus, the synergism of promoting iPSC proliferation and self-renewal, and in parallel inhibiting differentiation could explain the observed positive effect of 0413 on iPSCs maintenances.

*Effects* of *0413 on self-renewal of neural stem cells*

The self-renewal of multipotent neural stem cells was also tested with the compound of the current invention. Neural stem cells were cultivated in SPN medium with or without 0413 at different final concentrations (Fig. 7). The MTT assay was applied to determine cell viability. Starting from a concentration of 1nM O4I3 the viability of neural stem cells was increased in comparison to mock treated neural stem cells.

*Effects* of *0413 on human fibroblasts*

Analyzing the effects of 0413 on human fibroblasts, the activation of reprogramming associated genes, including an about 10-fold induction of Oct4, Nanog and Lin28, and an about 100-fold increase of Sox2 and E-Cadherin mRNA levels was detected. In particular, expression of several essential genes for reprogramming is equal to those in pluripotent stem cells, such as Esrrb, FGF2 and Sall4 (Fig. 12).

0413 was applied in commercially available episome-based reprogramming cocktail containing episomal vectors for overexpression of Oct4, Sox2, Klf4, Lin28 and L-Myc, as well as suppression of p53. In comparison to non-treated fibroblast cultures, 20 times more alkaline phosphatase (AP) positive colonies were detected in the presence of 0413 at a concentration of 50 nM in two tested human primary fibroblasts (HF1 and HF2) (Fig. 13).

The iPSCs exhibited pluripotent characteristics and showed typical iPSC morphology (Fig. 4A). More than 95% of them co-expressed Tra-1-60 and E-Cadherin (Fig. 4C) as well as Tra-1-81 and Oct4 as analyzed by FACS (Fig. 16). Immunoblotting (Fig. 4D) and immunocytochemistry (Fig. 15) further revealed co-expression of pluripotent markers Nanog and E-Cadherin, and Oct4 and Sox2. Immunoblotting also confirmed high levels of putative iPSC markers, comparable to established pluripotent stem cells and NCCIT (Fig. 4D).

The inventors compared DNA microarray data and found that the resulting iPSCs show a distinct pattern of global gene expression to human fibroblasts, which are similar to hPSCs (Fig. 4F).

Pluripotency-associated genes (Pou5F1 encoding Oct4, CDH1 encoding E-Cadherin, Epcam and DNMT3B) were highly expressed in newly generated hiPSC_1 and hiPSC_2, whereas fibroblast-related genes (CDH2, Twist1, Twist2 and Snai2) were suppressed, comparable to characterized hPSCs (Fig. 4F). Consistently, scatter plots also show high similarity of gene expression pattern between new hiPSCs and hPSCs, but not fibroblasts (Fig. 4E) Moreover, the established iPSCs were differentiated using standard protocols and expression of markers of mesoderm (T, Slug and Snail1), Ectoderm (Pax6, Nestin and Tuj1) and Endoderm (AFP, SoxA2 and Sox17) was detected by qRT-PCR (Fig. 4B), confirming the pluripotency of our hiPSCs, which can give rise to all three germ layers.

*O4I3 enhances the binding affinity of H3K4Me3 at the promoter of Oct4*

To obtain a deeper insight into the mechanism of action, global gene expression in HFF, HFF treated with O4I3 (HFF_O4I3), HFF transfected with episomal OSKM (HFF_OSKM) and hiPSCs was compared. It was found that 0413 induced expression of genes positively regulating cell cycle progression and DNA replication (e.g. CDK1, MYC and CDC25) and promoted de-differentiation by upregulation of epithelial genes (CDH1/E-Cadherin, Epcam, Crb3 and Ocln) and downregulation of mesenchymal genes (Twist2), two essential cellular responses at the early stage of reprogramming. Of note, the overlap of up- and down- regulated genes of HFF_O4I3 and HFF_OSKM or iPSCs were more than 40% (Fig. 5A). More than 30% among them are involved in epigenetic modification and chromatin remodeling, including Oct4 (Pou5f1), Lin28, MYCN, KIFs, DNMT3B, HDAC2, TET1, EZH2 and JARID2, which are of importance in somatic reprogramming and maintenance of pluripotency.

Gene expression profiling using O4I3-treated HFF with a subset of dysregulated genes (160 microarray probes) coding for 140 proteins with protein-protein interactions involved in epigenetic modification and chromatin remodelling. The network was built with STRING using only highest confidence level interactions between the 140 proteins (and all source of information available in STRING). Expected number of interactions (edges) for random network of the same size is 242, while the observed network has 879. Average node degree 12.6 and average local clustering coefficient is 0.665.

Gene Ontology-based functional enrichment analysis of regulated genes in HFF_O4I3 compared to HFF was performed and found that a number of specialized terms associated with epigenetic and chromatin regulation were the top regulated terms (Fig. 15), including sister chromatid segregation (GO:0000819), sister chromatid cohesion (GO:0007062), ribonucleoprotein complex biogenesis (GO:0022613), organelle fission (GO:0048285), DNA replication (GO:0006260).

Recently, Onder et al. (Nature, 2012, 483, 598-602) screened the influence of a panel of epigenetic regulators and revealed that inhibition of DOT1L promoted iPSCs generation. The inventors analyzed the expression of those genes upon O4I3 treatment in HFFs and found that 7 genes, including DOT1L, were downregulated, which were reported by Onder et al. to enhance the reprogramming, while MBD3 was significantly upregulated, whose suppression might counteract the generation of iPSCs.

The importance of active tri-methylation of histone H3 lysine 4 (H3K4Me3) in promoting transcription factors binding to an 'open' chromatin state and thereby initiation of reprogramming is known in the art. In good agreement, the inventors found the abundance of H3K4Me3 in 0413-treated reprogramming-resistant fibroblasts by immunostaining (Fig. 14) and immunoblotting (Fig. 5B). To directly study if 0413 facilitated the binding of H3K4Me3 to the promoter of Oct4, a CHIP-qPCR experiment was performed. An increased enrichment of H3K4Me4 at the promoter of Oct4 was detected in reprogramming-resistant patient fibroblasts (Fig. 5E), which might explain the conversion of resistant fibroblasts into hiPSCs using the combination of 0413 together with transient expression of OSKM.

*Effect on fibroblasts resistant to reprogramming*

It is known in the art that senescence or other unknown factors can confer donor fibroblasts resistance to reprogramming. Therefore, 0413 was tested for its ability to enable reprogramming of refractory fibroblasts. Two human fibroblasts were selected. HFF-LTR7-EGFP at passage >40 carrying EGFP driven by a LTR7 promoter, which was activated during reprogramming, and HF5, isolated from a patient sample, both of which were resistant to an episome-based reprogramming approach (Fig. 13). Treatment with 0413 (500Nm) resulted in the appearance of iPSC-like colonies appeared (Fig. 3C). Additional, Zolpidem also facilitated the reprogramming of resistant fibroblasts at 1 μM but with lower efficiency (Fig. 13).

*O4I3 Promotes the Methylation of H3K4*

hiPSC derivation is an epigenetic reprogramming process. Genome-wide analysis of histone modification and chromatin remodeling revealed the number of alternations occurring at the early stage of reprogramming, including the hypermethylation of H3K4 and the demethylation of H3K27 and H3K9. These loosen the compacted heterochromatin and promote transcription factors binding to the "open" chromatin to initiate the reprogramming.

The inventors investigated the transfection efficiency in HF1 and HF4 using the same episomal vector carrying cytomegalovirus (CMV)-driven GFP. The inventors could not observe a significant difference between two cell lines, as determined by FACS analysis. This result suggested that the resistance was unlikely associated with low transfection efficiency. To study the epigenetic effects of O4I3 and its relevance to reprogramming, the inventors focused on two histone modifications at the promoter of OCT4, namely, H3K4Me3, known to be related to gene activation, and H3K27Me3, which indicates gene repression. Chromatin immunoprecipitation-qPCR results in two reprogrammable fibroblasts (HF1 and HF2) and in two reprogramming-resistant fibroblasts (HF3 and HF4) showed that OSKM was sufficient to induce abundant occupation of H3K4Me3 at the promoter of OCT4 in HF1 and HF2 in a comparable manner to those in iPSCs, while producing 1,000- to 10,000-fold less in reprogramming-resistant cells (Figures 17C). The level of H3K27Me3 at the OCT4 promoter was minimally affected in our experiments (Figure 17C). Analysis on the global level of H3K4Me3 by immunocytochemistry showed the increase of H3K4Me3 upon 0413 treatment (Figure 17D). Immunoblotting confirmed a dose- and time-dependent increase of global H3K4Me3 expression in fibroblast, whereas H3K27Me3 remained mostly unaffected (Figure 17E). In an in vitro methylation assay, 0413 protected methylated H3K4 with an IC50 value of 20 nM (Figure 17F). Trimethylation of H3K9 has been reported to block reprogramming by recruiting heterochromatin protein 1 to form heterochromatin at the core of pluripotency loci, which interferes with the hypermethylation of H3K4. Accordingly, the inventors found the reduction of global H3K9Me3 posterior to H3K4Me3 activation (Figures 17E).

*0413 Is* a *Potent KDM5 Inhibitor*

HMT and HDM are two major classes of enzymes, contributing to the regulation of histone methylation. Lysine-specific demethylase 1 (LSD1) and histone lysine demethylase 5 (KDM5, also known as JARID1) majorly catalyze demethylation of H3K4. A few KDM5 chemical inhibitors have been reported to inhibit demethylation of H3K4, leading to an increase of global methylated H3K4 in various cell types. The inventors tested the inhibitory effect of 0413 on LSD1 and KDM5. KDM4 (also known as JMJD2), the HDM of H3K9 and H3K36, was also included. The inventors found that 0413 inhibited KDM5 with IC50 values of 0.79 nM, whereas it inhibited KDM4 with a 500-fold less potency (IC50: 249 nM). In the case of LSD1, the inventors hardly detected the inhibitory effect of the molecule even at a concentration of 100 mM (Figure 18A).

In mammalian cells, the KDM5 family consists of four members, namely, KDM5A (known as JARID1A), KDM5B (known as JARID1B or PLU1), KDM5C (JARID1C), and KDM5D (JARID1D or SMCY). Selectivity was found for KDM5A with an IC50 value of 0.19 nM, whereas 20-, 40-, and 1,000-fold less potent IC50 values were obtained in the case of KDM5D, KDM5C, and KDM5B, respectively (Figure 18B). As expected, zolpidem protected H3K4 methylation and inhibited the expression of both KDM5 and KDM4 atmicromolar concentrations. Next, the inventors tested four KDM chemical inhibitors. The inventors found 3- and 5-fold induction of OCT4 expression in NCCIT-OCT4 reporter cell line in the presence of KDM5 inhibitors, CPI-455 and JIB-04, respectively, but not in the presence of H3K27 demethylase inhibitor, GSK-J4 or the KDM2/7 inhibitor, daminozide, a plant growth regulator (Figure 18C). The inventors detected the induction of OCT4, SOX2, and E-Cadherin expression in JIB-04-treated HFs (Figure 18D).

JIB-04 is a potent KDM5A inhibitor (IC50: 230 nM) and was found to produce a higher induction of OCT4 compared with the pan KDM5 inhibitor, CPI-445 (Figure 18C). The inventors questioned if the members of the KDM5 family contribute differently to the induction of pluripotency. KDM5B has been shown to be crucial in the early embryonic development and plays an important role in ESC maintenance and neural differentiation. However, little is known about the function of other members of the KDM5 family of genes in development. The inventors thus re-analyzed the KDM5A-D expression during human preimplantation and compared their expression patterns with those of the putative pluripotency markers, OCT4 and NANOG. KDM5B showed a similar expression pattern to OCT4 and NANOG, whereas KDM5A behaved oppositely. In the case of KDM5D, the difference was not significant and KDM5C remained unaffected between ESC and oocyte. The inventors further compared the expression of KDM5A-5D in fibroblasts, reprogrammed fibroblasts by OSKM, iPSCs, and NCCIT (Figure 18E). KDM5A was preferentially expressed in reprogramming-resistant fibroblasts, whereas KDM5B was more pluripotency related (Figure 18E), which was in agreement with previous gene expression

profiling data, as well as immunoblotting results. As JIB-04 also inhibits KDM4 and KDM6 demethylases, the inventors repressed the expression of KDM5A-D using RNA interference (small interfering RNA [siRNA]) and found significantly higher OCT4 reporter activity in NCCIT-OCT4 cells (Figure 18F), as well as higher mRNA levels of OCT4 and SOX2 in HFs in the absence of KDM5A and KDM5D expression, but not in KDM5B and KDM5C knockdown cells (Figure 18G). Notably, the effects of KDM5D repression were found to be less significant when compared with those of KDM5A knockdown, most probably due to the various transfection efficiencies. Moreover, knockdown of KDM5A also increased the global levels of H3K4Me3 with or without OSKM. Overexpression of KDM5A compromised these effects caused by KDM5A inhibition (either 0413 or JIB-04) in NCCIT-OCT4 reporter cells in a concentration-dependent manner, as well as in HEK-OCT4 reporter cells (Figure 18H). Conversely, cells were resistant to KDM5A chemical inhibitors in the absence of KDM5A expression. Of note, at concentrations of 10 and 100 nM, 0413 treatment alone showed higher OCT4 levels when compared with that of 0413 combined with siRNA-mediated KDM5A inhibition, which may be due to the insufficient knockdown efficiency. As expected, overexpression of KDM5A also affected the formation of TRA-1-60-positive colonies in OSKM-mediated reprogramming of HFs (Figure 18I). Taken together, the inventors' results confirm the importance of KDM5A in the induction of pluripotency markers and the process of reprogramming of HFs.

*Inhibition of KDM5A Promotes Reprogramming of Resistant Fibroblasts*

In reprogramming-resistant HF4, the level of H3K4Me3 at the promoter of OCT4 was increased by OSKM, but was still nearly 1,000-fold lower than that in PSCs (Figure 19A). In combination with OSKM overexpression, genetic repression of KDM5A expression using either KDM5A siRNA or chemical inhibition of KDM5A activity by JIB-04, significantly elevated the levels of H3K4Me3 (Figure 19A). However, a clear influence on H3K4 methylation was not observed in the presence of KDM5B-, KDM5C-, or KDM5D-siRNA (Figure 19A). Correspondingly, OSKM failed to force the expression of reprogramming-associated genes (POU5F1, SOX2, and CDH1) in the absence of KDM5A siRNA or JIB-04 in reprogramming-resistant fibroblasts (Figure 19C), implicating that OSKM was not sufficient to activate OCT4 to initiate reprogramming in those patient primary fibroblasts. Indeed, the reprogramming efficiency is negatively correlated with the expression of KDM5A in 11 HFs (Figure 19D). Moreover, KDM5A siRNA increased the global level of H3K4Me3 in a dose-dependent manner (Figure 19E). Of note, the alternation in the occupation of H3K27Me3 at the promoter of OCT4 was more refractory to chemical inhibition or genetic repression of KDM5s in the inventors' experiments (Figure 19B). Transient suppression of KDM5A expression with either KDM5A siRNA or chemical inhibition mimicked the effect of 0413 to either increase the episomal-based reprogramming efficiency in normal fibroblasts or to facilitate the formation of ESC-like TRA-1-60-positive colonies in reprogramming-resistant fibroblasts (Figure 19F). These ESC-like colonies could be isolated and expanded to establish stable hiPSC cell lines with the expression of pluripotency markers, like OCT4, SOX2, E-Cadherin, TRA-1-60, and TRA-1-81, and full differentiation potential.

*Modifications of lead compound*

A structure-activity relationship of the lead compound was performed to identify modifications that might further enhance the activity or exhibit other beneficial properties. The modified compounds were synthesized according to the methods disclosed herein.

*Activation of Oct4*

The methyl group on the aryl moiety was replaced with various substituents and their activity was evaluated in NCCIT-Oct4 cells (Fig. 6). Replacement with halogens (compounds (102), (103) and (104)) generally increased the activity (relative to compound (101); O4I3), while either electron withdrawing group, -$NO_2$ (compound (105)) and -CN (compound (108)), or electron donating groups, -OMe (compound (106)) and -OH (compound (107)), as substituents significantly reduced the induction at the tested concentration (100 nM) as compared to the activity of O4I3. The Oct4 inducing activity was not affected by the replacement of the methyl group with hydrogen (compound (109)).

The position of the substituents $NO_2$ and F was varied and resulted in a declined activity in the meta (m-) or ortho (o-) position as compared to the para position of compounds (105) and (102).

The position of the substituent was varied and a declined activity of the resulting derivatives was observed regardless of position (m- or o-) or substituent ($NO_2$ or F).

Further modifications on the aryl moiety as well as on the pyridine moiety were tested and their activity measured in NCCIT-Oct4 cells as shown in Table 1.

*Table 1: Synthesized compounds tested in NCCIT-Oct4 cells*

| | | |
|---|---|---|
| 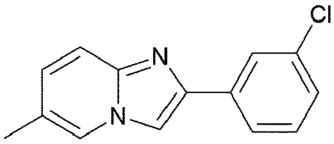<br>(114)<br>Formula: $C_{14}H_{11}ClN_2$<br>Mass: 242,0611<br>Luciferase-activity: **1.01** | 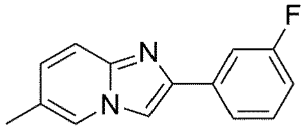<br>(122)<br>Formula: $C_{14}H_{11}FN_2$<br>Mass: 226,0906<br>Luciferase-activity: **0.36** | 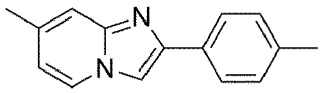<br>(117)<br>Formula: C15H14N2<br>Mass: 222,1157<br>Luciferase-activity: **0.54** |
| 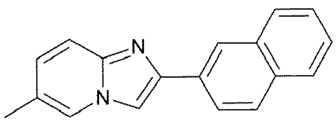<br>(123)<br>Formula: $C_{18}H_{14}N_2$<br>Mass: 258,1157<br>Luciferase-activity: **1.04** | 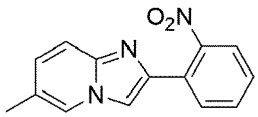<br>(111)<br>Formula: $C_{14}H_{11}N_3O_2$<br>Mass: 253,0851<br>Luciferase-activity: **0.48** | 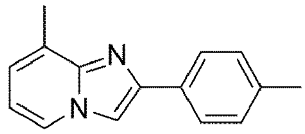<br>(118)<br>Formula: $C_{15}H_{14}N_2$<br>Mass: 222,1157<br>Luciferase-activity: **1.12** |
| 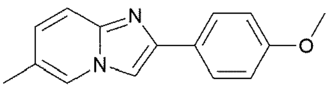<br>(106)<br>Formula: $C_{15}H_{14}N_2O$<br>Mass: 238,1106<br>Luciferase-activity: **0.51** | 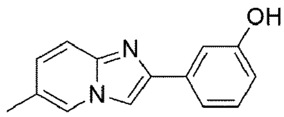<br>(115)<br>Formula: $C_{14}H_{12}N_2O$<br>Mass: 224,0950<br>Luciferase-activity: **0.58** | 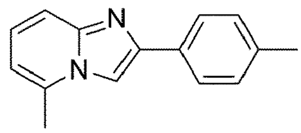<br>(119)<br>Formula: $C_{15}H_{14}N_2$<br>Mass: 222,1157<br>Luciferase-activity: **0.43** |
| 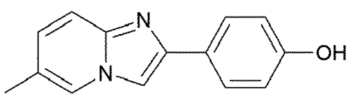<br>(107)<br>Formula: $C_{14}H_{12}N_2O$<br>Mass: 224,0950<br>Luciferase-activity: **0.41** | 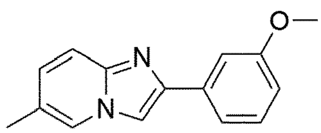<br>(116)<br>Formula: $C_{15}H_{14}N_2O$<br>Mass: 238,1106<br>Luciferase-activity: **0.65** | 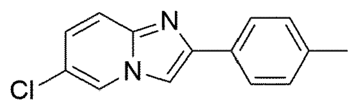<br>(120)<br>Formula: $C_{14}H_{11}ClN_2$<br>Mass: 242,0611<br>Luciferase-activity: **0.65** |
| 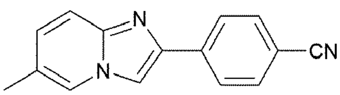<br>(108)<br>Formula: $C_{15}H_{11}N_3$<br>Mass: 233,0953<br>Luciferase-activity: **0.47** | 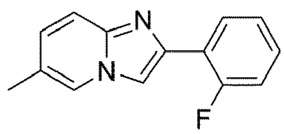<br>(113)<br>Formula: $C_{14}H_{11}FN_2$<br>Mass: 226,0906<br>Luciferase-activity: **0.41** | 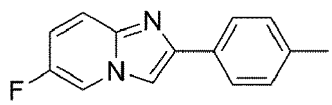<br>(121)<br>Formula: $C_{14}H_{11}FN_2$<br>Mass: 226,0906<br>Luciferase-activity: **0.79** |

(continued)

| | | |
|---|---|---|
| 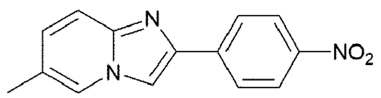 (105) Formula: $C_{14}H_{11}N_3O_2$ Mass: 253,0851 Luciferase-activity: **0.64** | 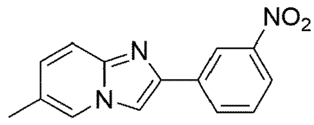 (110) Formula: $C_{14}H_{11}N_3O_2$ Mass: 253,0851 Luciferase-activity: **0.66** | 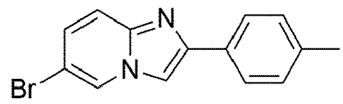 (122) Formula: $C_{14}H_{11}BrN_2$ Mass: 286,0106 Luciferase-activity: **0.65** |

Luciferase activity was measured in NCCIT-Oct4 cells and is expressed in relation to the activity of 0413 (101).

*Chemical optimization* of *0412 results metabolically stable O4I4*

The inventors adopted Hantzsch thiazole synthesis (Figure 20A) and produced a series of new O4I2-ester derivatives (Fig. 20B). Their activities were measured in three luciferase report cell lines, namely HEK-OCT4, driven by the activation of OCT4-promoter, and NCCIT-OCT4 and NCCIT-NANOG, whose intensities are controlled by endogenous OCT4 and NANOG respectively. Nearly all compounds showed the induction in HEK-OCT4 cells, while lost their activities in NCCIT cells (Fig. 20C). Because acid hydrolysis of 0412 resulting 2-(4-chlorophenylamino)thiazole-4-carboxylic acid (called O4I2-COOH) was inactive in the HEK-OCT4 cells, the inventors hypothesized that metabolic instability interfered the function of chemicals. Carboxylesterase 1 (CES1) is an enzyme majorly contributing to ester-to-acid conversion. The inventors compared the expression of CES1-5A using published gene expression data and found that CES1 indeed is highly expressed in NCCIT and PSCs. HPCL and LC/MS analysis on O4I2-treated cellular extraction implicated the occurrence of ester-to-acid conversion (Fig. 20D).

The inventors replaced the ethyl ester with other groups, including *t*-Butyl, -Ph and Naph (Fig. 20E) and detected comparable activities of all resulting compounds **(415-423)** in tested three luciferase reporter cell lines (Fig. 20F). Of those, **423** was among the most active compounds (Fig. 20F). The inventors called it OCT4 inducing compound 4 (O4I4) and assessed its activity further.

*0414 activates OCT4-associated signalings in various cells and extends lifespans and C.elegans models*

Consistently, in unmodified NCCIT cells 0414 forced the expression of OCT4 (3-fold increase as compared to 0.1% DMSO treatment) and NANOG (30-fold increase) as well as other pluripotency-related genes, like E-CADHERIN, SOX2, REX1 and STAT3, O4I4-mediated enrichment of OCT4 and NANOG, as well as SOX2 and E-CADHERIN, was also detected by fluorescence-activated cell sorting. Immunoblotting showed a concentration-dependent increase of OCT4 level in 0414-treated NCCIT cells. It was reported that removal of bFGF led to the loss of pluripotency-marker expression in hPSCs, including OCT4, SOX2, E-CADHERIN and NANOG, which however was rescued in the presence of O4I4 (Fig. 21A), indicating a positive function of O4I4 in maintaining the pluripotency-associated transcriptional circuitry in hPSC.

In human primary fibroblasts, O4I4 promoted the expression of pluripotency-associated genes, including OCT4, SOX2, NANOG, LIN28, E-CADHERIN, REX1 and SALL4. A dose-dependent increase of OCT4 protein in human primary fibroblasts upon treatment was confirmed by immunoblotting (Fig. 21B). FACS analysis further confirmed the O4I4-mediated induction of OCT4 and E-CADHERIN. Based on Gene Ontology and Pathway (KEGG and REACTOME) annotations, functional enrichment analysis of differentially expressed genes in fibroblasts treated with 0414 (20 μM) for 24 h revealed high enrichment of biological processes associated with repression of differentiation and promotion of cell proliferation and adhesion.

Recently, it was reported that transient expression of Yamanaka factors extended the lifespan in mice, indicating anti-aging effect of partial reprogramming. *C. elegans* provides a leading *in vivo* system in aging study. The inventors observed an increased survival ratio under oxidative stress (Fig. 21C) and life extension in *C.elegans* treated with 0.5 μM O4I4 (Fig. 21D). *daf-16* encodes an ortholog of human forkhead box O transcription factor (FOXO), whose activation in nuclei is related to extend the lifespan in *C.elegans.* O4I4 treatment led to the nucleic translocation of *darf-16* (Fig. 21E), suggesting that O4I4-mediated anti-aging effect might be due to the positive interaction of OCT4 and replated pluripotency-gene with FOXO.

*0414 can replace OCT4 for the generation of human iPSCs*

To examine if O4I4 can replace OCT4 to reprogram human fibroblasts into the pluripotent state, the inventors first introduced the pluripotent reporter pT2-LTR7-GFP into human primary fibroblasts, to generate a cell line called HF1-LTR7-GFP fibroblasts. Interestingly, some colonies with weak GFP signal appeared in fibroblasts treated merely with O4I4 (Fig. 22A). Those might be the partially reprogrammed cells (Fig. 22A), as the inventors failed to expand these colonies, implicating the lack of other factors for the generation of fully reprogrammed iPSCs. Therefore, the inventors then combined O4I4 with transient episomal expression of SOX2, KLF4, L-MYC and LIN28 (SKML) reprogram HF1-LTR7-GFP fibroblasts. ESC-like colonies appeared occasionally.

The inventors made the final combination containing O4I3, O4I4 plus SOX2, KLF4, L-MYC and LIN28, named CSKML (Fig 22B).

In CSKML-treated fibroblasts the inventors observed a few hESC-like colonies with high GFP signal (Fig. 22A). The inventors successfully isolated and expanded the colonies reprogrammed from various primary human fibroblasts (HF1-HF3). The number of TRA-1-60 positive colonies was comparable to episomal$^{OSKML}$-mediated reprogramming (Fig. 22C). Some of colonies still showed typical hESC-like morphologies after expansion for long term and could be maintained in both feeder and feeder-free conditions. These cells expressed pluripotency markers, like OCT4 and SOX2, whose levels were comparable to PSCs1-4 (Fig. 22D). At the protein level, abundant expression of OCT4, SOX2, E-CADHERIN and NANOG was detected (Fig. 22E, 22F). FACS analysis confirmed that more than 95% cells were positive to OCT4, TRA-1-81, E-CADHERIN and TRA-1-60. Bisulfite sequencing demonstrated the hypomethylation at the promoter region of OCT4 expressed in CSKML-induced iPSCs (Fig. 22G) indicating reactivation of endogenous OCT4. Global gene expression profile of CSKML-iPSCs showed higher similarity to that of pluripotent stem cell lines than that of fibroblasts (Fig. 21H). To evaluate the differentiation potential of the CSKML-iPSCs, the inventors performed *in vitro* differentiation assays using well-established monolayer or embryonic bodies and detected the expression of genes from all three germ layers by qRT-PCR and immunocytochemistry.

Several chemical cocktails (e.g. A83-01, PD0325901, PS48, parnate, CHIR99021 and sodium butyrate) with ectopic expression of OCT4 have been reported to generate human pluripotent cells. In combination of 0413 and O4I4 (O4I3/4) with those chemicals, however, the inventors could not establish fully reprogrammed iPSCs from human fibroblasts, suggesting that the further optimization of the chemical cocktail is required.

*O4I3/4 facilitates the chromatin remodeling at the OCT4 locus during reprogramming*

To probe the effects of O4I3/4 on chromatin remodeling during reprogramming, the inventors performed ATAC-seq on fibroblasts and TRA-1-60$^{+}$ cells treated with either CSKML or OSKML at different time points during reprogramming. The Principal component analysis (PCA) of the genome-wide chromatin accessibility data showed that O4I3/4 dramatically promoted chromatin remodeling toward the pluripotent state similar to OSKML-mediated reprogramming (Fig. 23A and 23B). Consistent with the transcription level, the inventors found that the chromatin at the OCT4 locus was opened more robustly in CSKML-treated fibroblasts in comparison to those in OSKML-treated fibroblasts (Fig. 23C), indicating that endogenous OCT4 was reactivated in CSKML-treated fibroblasts and the activation mechanism could be distinct from OSKML-mediated.

*HMGA1 is involved in the generation of CSKML*

To explore how O4I3/4 reactivates endogenous OCT4 transcription to reprogram human fibroblasts into iPSCs, the inventors performed RNA-seq on human fibroblasts treated with O4I3, O4I4, O4I3/4, CSKML and OSKML, respectively and ranked the gene up-regulated in human fibroblasts treated with O4I3/4 and compared to those in PSCs. As shown in Fig 24A, HMGA1 (high-mobility group A1) was the top hit and has been reported to support pluripotency. Re-analysis of gene expression during the human preimplantation showed that the up-regulation of HMGA1 took place as early as at 4-cell stage prior to OCT4 activation (from 8-10-cell stage), while HMGB1, a homologue of HMGA1, was kept as constant. Moreover, ATAC-seq analysis on the promoter of HMGA1 revealed higher chromatin accessibility upon CSKML treatment than OSKML (Fig. 24B), indicating that HMGA1 might involve in OCT4 activation during pluripotency establishment *in vivo.*

Definitely, in fibroblasts, the combination of O4I3/4 activated HMGA1 expression prior to OCT4 expression (Fig. 24C and 24D), while a comparison level of HMGA1 in chemical-treated fibroblasts to that in PSCs was found after 4 days incubation (Fig. 24C). SKML activated neither HMGA1 nor OCT4 expression. Moreover, CSKML failed to promote OCT4 expression in HMGA1-deficient fibroblasts (Fig. 24E and 24F), supporting the hypothesis that HMGA1 expression is essential for endogenous OCT4 activation during reprogramming.

To figure out if HMGA1 directly activates OCT4 transcription, the inventors analyzed the potential binding sites of transcription factors at the promoters of human OCT4 using MatInspector. HMG family (indicated as SORY for

SOX/SRY-sex/testis determining and related HMG box factors) is the top one in the rank list with totally matched numbers of 50. Consistent with the previous report, the occupation of HMGA1 at the promoter of OCT4 was further confirmed by ChIP-qPCR using specific HMGA1 antibody (Fig. 24G).

Finally, the inventors attempted to replace O4I4 with HMGA1 for reprogramming. The inventors overexpressed HMGA1 in human primary fibroblasts and found that transient overexpression of HMGA1 from day 2- day 9 was more efficient to reprogram human primary fibroblasts (Fig. 24H). The efficiency was slightly lower than those induced by either by CSKML or OSKML (Fig. 24H and 24I). The inventors called iPSCs generated by overexpression of HMGA1 and SKML as HSKML (Fig. 24I). The new HSKML-iPSCs showed high expression of pluripotency markers, like OCT4, SOX2 and E-CADHERIN in various biological assesses.

Taken together, the inventors' results suggest that O4I3/4 enhanced expression of HMGA1 that contribute to endogenous OCT4 reactivation during human fibroblast reprogramming into iPSCs. Importantly, the inventors identified a novel reprograming factor HMGA1 that can replace OCT4 during human somatic cell reprograming.

*HMGA1 expression is dependent on BMPs signaling pathway*

To explore if there are other signaling pathways involving in HMGA1 transcription regulation, the inventors compared the differently expressed genes in CSKML-treated and SKML-treated fibroblasts as well as O4I4-treated and DMSO-treated (mock) obtained from RNA-seq. *PDGFRL*, *BMP6* and *DACT1* were induced only in CSKML and are evident to be involved in the regulation of stemness-associated signaling pathways (Fig. 25A). Because BMP6 was reported to influence on fertility and BMPs promoted chemical- and transcription factor-mediated reprogramming, the inventors examined the role of BMP in CSKML-mediated reprogramming. BMPs activation requires phosphorylation of SMAD1/5/8, which in turn forms a complex with SMAD4 to induce expression of downstream gene, like *IDs.* Indeed, O4I4 promoted *ID1* and *ID2* expression, while *ID3* was unaffected. In CSKML-treated fibroblasts phosphorylation of SMAD1/5/8 and *ID1/2* expression was detected (Fig. 25B and Fig. 25C). Overexpression of IDs activated HMGA1 and slightly increased the level of OCT4, which was further elevated in the presence of chemicals or CSKML (Fig. 25C). Repressing BMP signaling in co-incubation with BMP receptor inhibitor DM (Dorsomormin), BMP antagonist protein Noggin or siRNA targeting SMAD4 inhibited the phosphorylation of SMAD1/5/8 and expression of ID1/2 (Fig. 25B), while CSKML-induced expression of HMGA1 and OCT4 also disappeared (Fig. 25B). Adding BMP6 enhanced OCT4 expression, while knockdown of ID1/2 abolished CSKML-induced either HMGA1 or OCT4 activation (Fig. 25D). An improved reprogramming efficiency was observed in the presence of BMP6 or ectopic expression of ID1/2 (Fig. 25E). Those iPSCs were characterized as abovementioned and expressed pluripotency markers including OCT4, SOX2, E-CADHERIN and TRA-1-60, Except for BMP6 BMP2 also phosphorylated SMAD1/5/and activated HMGA1 and OCT4 expression in CSKML-treated reprogramming. Interestingly, BMP9 failed to induce HMGA1 and OCT4.

*Material and methods*

*Reagents*

Solvents and reagents obtained from commercial suppliers were at least of reagent grade and were distilled or dried according to prevailing methods prior to use, if necessary. For monitoring the reactions, Alugram SIL G/UV254 sheets for TLC (Macherey & Nagel) were used. Flash column chromatography was accomplished using silica gel 60 (Macherey & Nagel, 0.040-0.063 mm). The purity of compounds was determined at least more than 96% by HPLC analysis.

*General procedure for the synthesis*

A mixture of 5-methylpyridin-2-amine and 2-bromo-1-phenylethan-1-ones (2:1) in EtOH was stirred at 60°C for 3 h. TLC was employed to control the reaction. After removal of solvent, the solid was suspended in 1N HCl and stirred for 2-3 h, filtered and washed with water to obtain the products in good yield (>90%).

*Analytical Methods*

1H and 13C NMR spectra were recorded on a Varian 300 MHz NMR system (1H: 300 MHz, 13C: 75 MHz). Chemical shifts are reported in ppm from tetramethylsilane with solvent as the internal standard (1H DMSO-d6: $\delta$ 2.50; 13C DMSO-d6: $\delta$ 39.5). The following abbreviations were used to explain the multiplicities in NMR spectra: s =singlet, d = doublet, t = triplet, q = quartet, m = multiplet. High-resolution mass spectra (HRMS) were recorded on a Bruker ApexQe hybrid 9.4 T FT-ICR (ESI).

*6-Methyl-2-(p-tolyl)imidazo[1,2-a]pyridine (101), O4I3*

0413 1H NMR (300 MHz, DMSO-d6) δ 8.71 - 8.62 (m, 2H), 7.93 - 7.69 (m, 4H), 7.32 (d, J = 8.0 Hz, 2H), 2.41 - 2.24 (m, 6H). 13C NMR (75 MHz, DMSO-d6) δ 140.6, 139.1, 136.1, 135.6, 130.3, 127.5, 126.8, 126.5, 123.9, 111.6, 110.7, 21.4, 17.9. HRMS (ESI, C15 H14 N2) [M + H]+: calculated m/z, 223,1230; found m/z, 223,1230. IR (cm-1): 3342, 2159, 2021, 1661, 1540, 1534, 1527, 1505, 1456, 1366, 1360, 1311, 1269, 1195, 949, 848, 839, 805, 783, 767, 761, 740, 722.

*2-(4-Fluorophenyl)-6-methyl-imidazo[1,2-a]pyridine (102)*

1H NMR (300 MHz, DMSO-d6) δ 8.83 - 8.54 (m, 2H), 8.30 - 7.88 (m, 2H), 7.90 - 7.65 (m, 2H), 7.55- 7.17 (m, 2H), 2.38 (d, J = 1.1 Hz, 3H). 13C NMR (75 MHz, DMSO-d6) δ 163,4 (d, J = 248.5 Hz), 139.4, 136.2, 134.9, 129.1 (d, J = 8.7 Hz), 127.5, 126.9, 123,6 (d, J = 3.3 Hz),116.9 (d, J = 22.1 Hz), 111.7, 111.1, 17.8. HRMS (ESI, C14 H11 F N2) [M + H]+: calculated m/z, 227,0979; found m/z, 227.0979. IR (cm-1): 3325, 3114, 2524, 2362, 2165, 2017, 1659, 1501, 1446, 1271, 1238, 1165, 849, 823, 805, 788, 742.

*2-(4-Chlorophenyl)-6-methyl-imidazo[1,2-a]pyridine (103)*

1H NMR (300 MHz, DMSO-d6) δ 8.76 (d, J = 0.6 Hz, 1H), 8.68 (q, J = 1.2 Hz, 1H), 8.07 - 7.99 (m, 2H), 7.89 - 7.73 (m, 2H), 7.65 - 7.56 (m, 2H), 2.39 (d, J = 1.1 Hz, 3H). 13C NMR (75 MHz, DMSO-d6) δ 139.5, 136.3, 135.2, 134.8, 129.8, 128.3, 127.5, 126.9, 126.0, 111.8, 111.6, 17.9. HRMS (ESI, C14 H11 Cl N2) [M + H]+:calculated m/z, 243.0684; found m/z, 243.0684. IR (cm-1): 3303, 2528, 2362, 2162, 2020, 1660, 1527, 1488, 1474, 1455, 1416, 1273, 1094, 1010, 941, 933, 843, 803, 769, 742, 729.

*2-(4-Bromophenyl)-6-methyl-imidazo[1,2-a]pyridine (104)*

1H NMR (500 MHz, DMSO-d6) δ 8.77 (d, J = 0.7 Hz, 1H), 8.68 (q, J = 1.3 Hz, 1H), 8.00 - 7.93 (m, 2H), 7.85 (d, J = 9.1 Hz, 1H), 7.80 - 7.73 (m, 3H), 2.40 (d, J = 1.2 Hz, 3H). 13C NMR (75 MHz, DMSO-d6) δ 139.6, 136.3, 135.0, 132.8, 128.6, 127.5, 126.9, 126.5, 124.0, 112.0, 111.6, 17.9. HRMS (ESI, C14 H11 Br N2) [M + H]+: calculated m/z, 287.0178; found m/z, 287.0178. IR (cm-1): 2534, 2364, 2161, 2023, 1978, 1655, 1525, 1488, 1455, 1443, 1274, 1067, 1007, 839, 804, 786, 774, 742, 712.

*6-Methyl-2-(4-nitrophenyl)imidazo[1,2-a]pyridine (105)*

1H NMR (300 MHz, DMSO-d6) δ 8.79 (s, 1H), 8.58 (d, J = 1.6 Hz, 1H), 8.42 - 8.28 (m, 2H), 8.34 - 8.18 (m, 2H), 7.76 (d, J = 9.2 Hz, 1H), 7.59 (d, J = 9.3 Hz, 1H), 2.36 (d, J = 1.1 Hz, 3H). 13C NMR (75 MHz, DMSO-d6) δ 147.8, 141.6, 136.7, 136.0, 134.3, 127.3, 126.3, 126.0, 124.8, 113.7, 113.0,17.9. HRMS (ESI, C14 H11 N3O2) [M + H]+: calculated m/z, 254.0924; found m/z, 254.0924. IR (cm-1): 2521, 2364, 2160, 2022, 1977, 1601, 1509, 1490, 1449, 1343, 1264, 1245, 1221, 1108, 854, 834, 806, 768, 752, 741, 715.

*2-(4-methoxyphenyl)-6-methylimidazo [1,2-a] pyridine*

1H NMR: δ 8.67 (q, J = 1.2 Hz, 1H), 8.63 (d, J = 0.7 Hz, 1H), 8.03 - 7.97 (m, 2H), 7.86 (d, J = 9.1 Hz, 1H), 7.77 (dd, J = 9.2, 1.6 Hz, 1H), 7.15 - 7.09 (m, 2H), 3.83 (s, 2H), 2.50 (p, J = 1.8 Hz, 3H), 2.41 (d, J = 1.1 Hz, 2H). 13C NMR: δ 161.2, 139.1, 135.8, 135.8, 128.3, 127.4, 126.7, 119.2, 115.2, 111.6, 109.9, 55.9, 17.9. HRMS (ESI) calculated m/z, 239.1179; found C15H15N2O m/z, 239.1179 [M + H]+. IR(cm-1):2539,2436,2366,2157,2028, 1975, 1660, 1617, 1594, 1577, 1561, 1507, 1441, 1367, 1294, 1256, 1186, 1160, 1023, 952, 840, 808, 795, 743.

*4-(6-methylimidazo [1,2-a] pyridin-2-yl) phenol*

1H NMR: δ 10.22 (s, 1H), 8.66 (s, 1H), 8.54 (s, 1H), 7.85 (dd, J = 9.0, 2.3 Hz, 3H), 7.76 (dd, J = 9.2, 1.5 Hz, 1H), 7.03 - 6.90 (m, 2H), 2.41 (d, J = 1.2 Hz, 3H). 13C NMR δ 159.7, 139.5, 137.2, 134.9, 128.2, 126.8, 126.5, 118.3, 116.5, 112.0, 109.2, 17.9. HRMS (ESI) calculated m/z, 225.1022; found C14 H15N2O m/z, 225.1022 [M + H]+. IR (cm-1): 2489, 2158, 2027, 1975, 1617, 1591, 1506, 1459, 1397, 1375, 1292, 1275, 1246, 1219, 1179, 1112, 951, 837, 812, 766, 741.

*4-(6-methylimidazo [1,2-a] pyridin-2-yl) benzonitrile*

1H NMR: δ 8.90 (s, 1H), 8.70 (q, J = 1.3 Hz, 1H), 8.29 - 8.18 (m, 2H), 8.09 - 7.98 (m, 2H), 7.88 (d, J = 9.2 Hz, 1H), 7.79 (dd, J = 9.3, 1.5 Hz, 1H), 2.41 (d, J = 1.1 Hz, 3H). 13C NMR: δ 140.1, 136.4, 134.5, 133.6, 131.9, 127.4, 127.2, 126.9, 118.8, 113.0, 112.5, 112.2, 17.9. HRMS (ESI) calculated m/z, 234.1026; found C15H12N3 m/z, 234.1026 [M + H]+. IR (cm-1): 2781, 2702,2536, 2498, 2446, 2368, 2156, 2025, 1974, 1656, 1613, 1557, 1528, 1498, 1446, 1302, 1267, 1163, 1135, 1039, 1021, 996, 950, 8757, 848, 835, 808, 791, 746, 717.

*6-methyl-2-(3-nitrophenyl) imidazo [1,2-a] pyridine*

1H NMR: δ 8.79 (t, J = 2.0 Hz, 1H), 8.68 (s, 1H), 8.47 (q, J = 1.4 Hz, 1H), 8.42 (dt, J = 7.8, 1.3 Hz, 1H), 8.21 (dd, J = 8.2, 1.0 Hz, 1H), 7.78 (t, J = 8.0 Hz, 1H), 7.66 (d, J = 9.2 Hz, 1H), 7.38 (dd, J = 9.0, 1.7 Hz, 1H), 2.34 (s, 3H). 13C NMR: δ 150.31, 142.50, 137.70, 136.28, 133.14, 131.96, 131.58, 128.54, 127.49, 125.29, 121.80, 113.70, 113.01, 18.10.. HRMS (ESI) calculated m/z, 254.0924; found C14H12N3O2 m/z, 254.0924 [M + H]+. IR (cm-1): 2947, 2930, 2541, 2456, 2363, 2158, 2028, 1974, 1656, 1619, 1554, 1517, 1486, 1456, 1348, 1266, 1215, 1163, 1114, 1071, 970, 904, 879, 837, 808, 768, 724.

*6-methyl-2-(2-nitrophenyl) imidazo [1,2-a] pyridine*

1H NMR: δ 8.40 - 8.34 (m, 1H), 8.18 (d, J = 0.7 Hz, 1H), 7.91 (dd, J = 7.8, 1.4 Hz, 1H), 7.83 (dd, J = 8.0, 1.2 Hz, 1H), 7.71 (td, J = 7.6, 1.3 Hz, 1H), 7.56 (td, J = 7.7, 1.4 Hz, 1H), 7.48 (d, J = 9.1 Hz, 1H), 7.14 (dd, J = 9.2, 1.7 Hz, 1H), 2.28 (d, J = 1.1 Hz, 3H). 13C NMR: δ 149.2, 144.1, 140.0, 132.4, 130.9, 129.2, 129.0, 127.4, 124.9, 124.0, 122.3, 116.7, 111.1, 18.0. HRMS (ESI) calculated m/z, 254.0924; found C14H12N3O2 m/z, 254.0924 [M + H]+. IR (cm-1): 2540, 2487, 2443, 2364, 2341, 2159, 2027, 1974, 1609, 1527, 1459, 1420, 1372, 1345, 1285, 1260, 1208, 1164, 1144, 1105, 1033, 1018, 990, 949, 854, 836, 800, 776, 761, 727.

*2-(3-fluorophenyl)-6-methylimidazo [1,2-a] pyridine*

1H NMR: δ 8.83 (s, 1H), 8.71 (q, J = 1.2 Hz, 1H), 7.97 (dt, J = 10.2, 2.2 Hz, 1H), 7.96 - 7.83 (m, 2H), 7.81 (dd, J = 9.2, 1.5 Hz, 1H), 7.62 (td, J = 8.1, 6.0 Hz, 1H), 7.43 - 7.28 (m, 1H), 2.41 (d, J = 1.1 Hz, 3H). 13C NMR: δ 162.9 (d, J = 244.2 Hz), 139.6 , 136.4 , 134.7 (d, J = 2.8 Hz), 132.0 (d, J = 8.5 Hz), 129.4 (d, J = 8.8 Hz), 127.5 , 126.9 , 122.8 (d, J = 2.9 Hz), 117.4 (d, J = 21.1 Hz), 113.5 (d, J = 24.1 Hz), 112.0 , 111.9 , 17.8 . HRMS (ESI) calculated m/z, 227.0979; found C14H11FN2 m/z, 227.0979 [M + H]+. IR (cm-1): 3058, 3023, 3012, 2929, 2536, 2448, 2366, 2157, 2102, 2024, 1975, 1664, 1604, 1585, 1529, 1491, 1452, 1362, 1311, 1271, 1196, 1159, 1088, 799, 879, 816, 795, 746.

*2-(2-fluorophenyl)-6-methylimidazo[1,2-a] pyridine*

1H NMR: δ 8.75 (q, J = 1.3 Hz, 1H), 8.64 (d, J = 3.0 Hz, 1H), 8.26 (td, J = 7.8, 1.7 Hz, 1H), 7.91 (d, J = 9.2 Hz, 1H), 7.81 (dd, J = 9.2, 1.6 Hz, 1H), 7.63 - 7.53 (m, 1H), 7.53 - 7.41 (m, 2H), 2.41 (s, 3H). 13C NMR: δ 159.6 (d, J = 250.8 Hz), 139.4 , 136.3 , 132.5 (d, J = 9.0 Hz), 130.0 , 128.9 , 127.2 , 126.8, 125.9 , 117.0 (d, J = 21.4 Hz), 115.5 (d, J = 11.9 Hz), 113.8 (d, J = 14.0 Hz), 112.0 , 18.0 (d, J = 3.7 Hz). HRMS (ESI) calculated m/z, 227.0979; found C15H14FN2 m/z, 227.0979 [M + H]+. IR(cm-1): 2930, 2542, 2449, 2371,2160, 2025, 1973, 1656, 1621, 1523, 1498, 1451, 1357, 1313, 1268, 1227, 1164, 1118, 949, 861, 805, 764, 743.

*Ethyl 2-((4-hydroxyphenyl)amino)thiazole-4-carboxylate*

$^{1}$H-NMR: 1.28 (t, $^{3}$J=6.9 Hz, 3H), 4.24 (q, $^{3}$J=6.9, 2H), 6.74 (dd, $^{3}$J=6.9 Hz, $^{4}$J=2.1 Hz, 2H), 7.36 (dd, $^{3}$J=6.9 Hz, $^{4}$J=2.1 Hz, 2H), 7.63 (s, 1H), 9.12 (s, 1H), 10.00 (s, 1H). $^{13}$C-NMR: 14.2, 60.3, 115.5, 117.2, 119.8, 132.7, 142.4, 152.8, 160.9, 164.6. IR (cm$^{-1}$): 3308, 3127, 1693, 1586, 1545, 1508, 1464, 1437, 1393, 1297, 1272, 1240, 1212, 1159, 1093, 1021, 959, 876, 840, 819, 793, 771, 722, 674, HRMS (ESI) Calculated m/z: 265.0641; found m/z: 265.0641 [M+H]$^{+}$. Anal. Calcd for $C_{12}H_{12}N_2O_3S$: C,54.53; H,4.58; N,10.60. Found: C, 54.91; H, 4.67; N, 10.23.

*Ethyl 2-((4-methoxyphenyl)amino)thiazole-4-carboxylate*

$^{1}$H-NMR: 1.28 (t, $^{3}$J=7.2 Hz, 3H), 3.72 (S, 3H), 4.25 (q, $^{3}$J=7.2 Hz, 2H), 6.92 (dd, $^{3}$J=6.9 Hz, $^{4}$J=2.1 Hz, 2H), 7.52 (dd, $^{3}$J=6.9 Hz, $^{4}$J=2.4 Hz, 2H), 7.67(s, 1H), 10.23 (s, 1H). $^{13}$C-NMRspectrum: 14.2, 55.3, 60.4, 114.3, 117.7, 119.3, 134.1, 142.0, 154.6, 160.8, 164.1. IR (cm$^{-1}$): 3019, 2833, 2541, 2159, 2031, 1976, 1714, 1635, 1588, 1535, 1510, 1323, 1267, 1253, 1212, 1182, 1125, 1109, 1035, 1017, 822, 801, 767, 661, HRMS (ESI) Calculated m/z: 279.0798; found

m/z: 279.0798 [M+H]+. Anal. Calcd for $C_{13}H_{14}N_2O_3S$: C, 56.10; H, 5.07; N, 10.07. Found: C, 56.18; H, 5.47; N, 9.82.

*Ethyl 2-(p-tolylamino)thiazole-4-carboxylate*

$^1$H-NMR: 1.27 (t, $^3$J=6.9, 3H), 2.23 (s, 3H), 4.23 (q,$^3$J=6.9 Hz, 2H), 7.11 (d, $^3$J=8.1 Hz, 2H), 7.47 (d, $^3$J=8.7 Hz, 2H), 7.69 (s, 1H), 10.25 (s, 1H).$^{13}$C-NMR: 14.7, 20.8, 60.8, 117.7, 118.4, 129.9, 131.1, 138.8, 142.8, 161.3, 163.9. IR (cm$^{-1}$): 3262, 2509, 2158, 2023, 1977, 1732, 1542, 1508, 1438, 1190, 1188, 1026, 795, 704, 675. HRMS (ESI) Calculated m/z: 263.0849; found m/z: 263.0849 [M+H]$^+$. Anal. Calcd for $C_{13}H_{14}N_2O_2S$: C,59.52; H,5.38; N,10.68. Found: C,59.32; H, 5,66; N, 10,51.

*Ethyl 2-((4-nitrophenyl)amino)thiazole-4-carboxylate*

$^1$H-NMR: 1.31 (t, $^3$J=7.2 Hz, 3H), 4.29 (q, $^3$J=6.9 Hz, 2H), 7.83-7.86 (m, 2H), 7.95 (s, 1H), 8.23-8.26 (m, 2H), 11.16 (s, 1H). $^{13}$C-NMR: 14.2, 60.6, 116.3, 120.7, 125.5, 140.5, 142.4, 146.4, 160.5, 161.8. IR (cm$^{-1}$): 3316, 3114, 2440, 2159, 2031, 1976, 1688, 1594, 1561, 1527, 1510, 1487, 1419, 1323, 1303, 1254, 1234, 1181, 1112, 1093, 1027, 977, 882, 859, 847, 822, 772, 749, 689, HRMS (ESI) Calculated m/z: 294.0543; found m/z: 294.0543 [M+H]$^+$. Anal. Calcd for $C_{12}H_{11}N_3O_4S$: C,49.14; H,3.78; N,14.33. Found: C,49.18; H,3.47; N,14.42,

*4-((4-(Ethoxycarbonyl)thiazol-2-yl)amino)benzoicacid*

$^1$H-NMR: 1.28 (t, $^3$J= 8.1 Hz), 4.24 (q, $^3$J=6.9 Hz, 2H), 7.75 (d, $^3$J=9.0 Hz, 2H), 7.87(s, 1H), 7.93 (d, $^3$J=9.0 Hz, 2H), 10.82 (s, 1H), 11.17 (s, 1H). $^{13}$C-NMR: 14.2, 60.2, 116.1, 119.7, 122.3, 130.6, 142.4, 144.7, 160.6, 162.2, 165.3. IR (cm$^{-1}$): 3640, 3261, 2924, 2520, 2163, 2023, 1722, 1674, 1595, 1548, 1439, 1372, 1322, 1277, 1179, 1121, 1099, 1020, 848, 764, 715. HRMS (ESI) Calculated m/z: 315,0410; found m/z: 315,0410 [M+Na]$^+$. Anal. Calcd for $C_{13}H_{12}N_2O_4S$ C,53.42; H,4.14; N,9.58. Found: C, 53.63; H, 4.24; N, 9.38.

*Ethyl 2-((4-cyanophenyl)amino)thiazole-4-carboxylate*

$^1$H-NMR: 1.30 (t,$^3$J=6.9 Hz, 3H), 4.28 (d, $^3$J=6.9 Hz, 2H), 7.76-7.83 (m, 4H), 7.90(s, 1H), 10.92(s, 1H), $^{13}$C-NMR: 14.2, 60.6, 102.7, 116.8, 120.0, 121.9, 133.5 142.4, 144.4, 160.5, 162.0. IR(cm$^{-1}$): 3631, 3304, 3190, 3120, 2514, 2212, 2162, 2023, 1975, 1694, 1601, 1524, 1448, 1411, 1316, 1216, 1174, 1121, 1088, 1019, 828, 785, 720. HRMS (ESI) Calculated m/z: 296.0464; found m/z: 296.0464 [M+Na]$^+$. Anal. Calcd for $C_{13}H_{11}N_3O_2S$ C,57.13; H,4.06; N,15.37. Found: C,57.18; H,4.07; N,15.43.

*Ethyl 2-((2,4-dimethoxyphenyl)amino)thiazole-4-carboxylate*

$^1$H-NMR: 1.27 (t, $^3$J=6.9 Hz, 3H), 3.76 (s, 3H), 3.81 (s, 3H), 4.24 (q, $^3$J=7.2 Hz, 2H), 6.51-6.55 (m, 1H), 6.64-6.65 (m, 1H), 7.62(s, 1H), 7.83-7.86 (m, 1H), 9.56 (s, 1H). $^3$C-NMR: 14.2, 55.4, 55.7, 60.4, 99.3, 104.4, 118.0, 122.5, 122.6, 141.1, 151.5, 156.8, 160.6, 166.2. IR (cm$^{-1}$): 3053, 2974, 2522, 2159, 2031, 1977, 1729, 1618, 1586, 1518, 1460, 1334, 1257, 1213, 1197, 1179, 1156, 1129, 1097, 1032, 1011, 836, 818, 768, 666. HRMS (ESI) Calculated m/z: 309.0904; found m/z: 309.0904 [M+H]$^+$. Anal. Calcd for $C_{14}H_{16}N_2O_4S$ C,54.53; H,5.23; N,9.09. Found: C,54.42; H, 5.47; N, 9.01.

*Ethyl 2-((2,5-dimethoxyphenyl)amino)thiazole-4-carboxylate*

$^1$H-NMR: 1.29 (t, $^3$J=6.9 Hz, 3H), 3.72 (s, 3H), 3.80 (s, 3H), 4.25 (q, $^3$J=7.2 Hz, 2H), 6.49-6.53 (m, 1H), 6.91-6.94 (m, 1H), 7.75 (s, 1H), 8.25-8.26 (m, 1H), 9.79 (s, 1H). $^{13}$C-NMR: 14.2, 55.1, 56.3, 60.3, 105.1, 105.7, 111.4, 130.6, 141.8, 142.0, 142.1, 153.3, 160.8. 163.2. IR (cm$^{-1}$): 3321, 3119, 2520, 2159, 2031, 1977, 1687, 1602, 1541, 1518, 1481, 1462, 1449, 1368, 1331, 1280, 1251, 1216, 1206, 1173, 1125, 1088, 1052, 1020, 977, 957, 895, 876, 860, 786, 761, 751, 711, 654. HRMS (ESI) Calculated m/z: 309.0904; found m/z: 309.0904 [M+H]$^+$. Anal. Calcd for $C_{14}H_{16}N_2O_4S$: C,54.53; H,5.23; N,9.09. Found: C,54.43; H, 5.48; N, 9.11.

*Ethyl 2-((3,5-bis(trifluoromethyl)phenyl)amino)thiazole-4-carboxylate*

$^1$H-NMR: 1.31(t, $^3$J=7.2 Hz, 3H), 4.27 (d, $^3$J=7.2 Hz, 2H), 7.61 (s, 1H), 7.93 (m, 2H), 8.38 (s, 1H), 11.10 (s, 1H), $^{13}$C-NMR: 14.4, 61.0, 113.6-114.5, 116.9 (d, J = 4.3 Hz), 120.5, 123.7 (d, J = 272.8 Hz), 127.0, 134.2 (q, J = 32.6 Hz), 142.7 (d, J = 1.7 Hz), 161.0, 162.4. IR (cm$^{-1}$): 3642, 3315, 2510, 2162, 2023, 1975, 1682, 1578, 1536, 1377, 1269, 1114, 1011, 936, 874, 691, HRMS (ESI) Calculated m/z: 407.0259; found m/z: 407.0259 [M+Na]$^+$. Anal. Calcd for $C_{14}H_{10}F_6N_2O_2S{\cdot}0.25\ H_2O$ C, 43.25; H, 2.72; N, 7.21. Found: C, 43.46; H,2.80; N, 6.95.

*Ethyl 2-((5-chloro-2-methoxyphenyl)amino)thiazole-4-carboxylate*

$^1$H-NMR: 1.30 (t, $^3$J=7.2 Hz, 3H), 3.87 (s, 3H), 4.26 (q, $^3$J=7.2 Hz, 2H), 6.99-7.01 (m, 2H), 7.80(s, 1H), 8.67 (d, $^3$J=2.1 Hz, 1H), 9.97(s, 1H). $^{13}$C-NMR: 14.2, 56.0, 60.4, 111.9, 117.0, 119.9, 120.9, 124.3, 131.0, 141.8, 146.4, 160.7, 162.8. IR (cm$^{-1}$): 3315, 3122, 2500, 2159, 2031, 1976, 1686, 1596, 1536, 1509, 1491, 1416, 1331, 1250, 1212, 1173, 1125, 1021, 876, 798, 786, 761, 752. HRMS (ESI) Calculated m/z: 313.0408; found m/z: 313.0408 [M+H]$^+$. Anal. Calcd for $C_{13}H_{13}ClN_2O_3S{\cdot}0.5H_2O$: C,48.53; H,4.39; N,8.71. Found: C, 48.93; H, 4.35; N,8.37.

*Ethyl 2-((2,4-difluorophenyl)amino)thiazole-4-carboxylate*

$^1$H-NMR: 1.28 (t, $^3$J=6.9 Hz, 3H), 4.25 (q, $^3$J=7.2 Hz, 2H), 7.11-7.14 (m, 1H), 7.23-7.36 (m, 1H), 7.78 (s, 1H), 8.36-8.40 (m, 1H), 10.13 (s, 1H). $^{13}$C-NMR: $^{13}$C-NMR: 14.6, 60.9, 106.3 (d, J = 2.1 Hz), 107.9 (dd, J =24.6, 7.5 Hz), 116.2 (dd, J =21.5, 10.2 Hz), 120.8, 130.2 (m), 142.3, 147.9 (dd, J = 239.1, 2.4 Hz), 158.5 (dd, J = 237.3, 2.0 Hz), ,161.7, 162.8. IR (cm$^{-1}$): 3246, 2928, 2518, 2159, 2031, 1976, 1716, 1702, 1538, 1511, 1456, 1432, 1369, 1333, 1318, 1257, 1226, 1197, 1172, 1142, 1116, 1092, 1020, 971, 957, 848, 839, 810, 786, 768, 742, 730, 719, 670, HRMS (ESI) Calculated m/z: 285.0504; found m/z: 285.0504 [M+H]$^+$. Anal. Calcd for $C_{12}H_{10}F_2$ $N_2O_2S{\cdot}0.67H_2O$:C, 49.65; H, 3.70; N, 9.65. Found: C, 49.65; H, 3.46; N, 9.22.

*Ethyl 2-((2,5-difluorophenyl)amino)thiazole-4-carboxylate*

$^1$H-NMR: 1.30 (t, $^3$J=6.9 Hz, 3H), 4.27 (q, $^3$J=7.2 Hz, 2H), 6.78-6.84 (m, 1H), 7.25-7.33 (m, 1H), 7.86(s, 1H), 8.46-8.53 (m, 1H), 10.45 (s, 1H). $^{13}$C-NMR: $^{13}$C-NMR: 14.6, 60.8, 104.5 (dd, J = 27.1, 23.4 Hz), 111.6 (dd, J = 21.7, 3.6 Hz), 120.0, 121.8 (dd, J = 9.1, 2.8 Hz), 125.8 (dd, J = 11.1, 3.4 Hz), 142.4, 152.4 (dd, J = 247.2, 12.2 Hz), 157.3 (dd, J = 241.8, 11.3 Hz), 161.1, 164.1. IR (cm$^{-1}$): 3308, 3124, 2517, 2159, 2031, 1976, 1702, 1644, 1551, 1524, 1491, 1483, 1444, 1373, 1331, 1241, 1214, 1194, 1182, 1116, 1093, 1027, 967, 876, 865, 798, 780, 743, 723, HRMS (ESI) Calculated m/z: 307.0323; found m/z: 307.0323 [M+Na]$^+$. Anal. Calcd for $C_{12}H_{10}F_2N_2O_2S{\cdot}0.67H_2O$: C, 49.65; H, 3.70; N, 9.65. Found: C, 49.44; H, 3.91; N, 9.35.

*Ethyl 2-((2-bromo-4-methylphenyl)amino)thiazole-4-carboxylate*

$^1$H-NMR: 1.27 (t, $^3$J=6.9 Hz, 3H), 2.29 (s, 3H), 4.23 (q, $^3$J=7.2 Hz, 2H), 7.19-7.20 (m, 1H), 7.22-7.23 (m, 1H), 7.71 (s, 1H), 7.81-7.84 (m, 1H), 9.63 (s, 1H). $^{13}$C-NMR: 14.2, 20.0, 60.3, 115.9, 1!8.9, 124.1, 129.1, 133.2, 135.7, 136.1, 142.1, 160.8, 165.5. IR (cm$^{-1}$): 3630, 3121, 2982, 2925, 2517, 2162, 2024, 1975, 1718, 1549, 1485, 1384, 1203, 1090, 1035, 800, 728, 666. HRMS (ESI) Calculated m/z: 340.9954; found m/z: 340.9954 [M+H]$^+$. Anal. Calcd for $C_{13}H_{13}BrN_2O_2S{\cdot}0.5\ H_2O$: C, 44.58; H, 4.03; N, 8.00. Found: C, 44.59; H, 3.63; N, 7.61.

*Ethyl 2-((4-bromo-2-methylphenyl)amino)thiazole-4-carboxylate*

$^1$H-NMR: 1.27 (t, $^3$J=8.4 Hz, 3H), 2.26 (s, 3H), 4.24 (q, $^3$J=7.2 Hz, 2H), 7.36-7.43 (m,2H), 7.73(s, 1H), 7.90-7.93 (m, 1H), 9.55 (s, 1H). $^{13}$C-NMR: 14.2, 17.7, 60.4, 115.0, 118.8, 122.5, 129.2, 131.4, 132.9, 138.4, 142.1, 160.8, 164.8. IR (cm$^{-1}$): 3128, 2984, 2929, 2517, 2162, 2023, 1975, 1725, 1548, 1479, 1387, 1207, 1089, 1024, 876, 792,711. HRMS (ESI) Calculated m/z: 340.9954; found m/z: 340.9954 [M+H]$^+$. Anal. Calcd for $C_{13}H_{13}BrN_2O_2S{\cdot}0.5\ H_2O$: C, 44.58; H, 4.03; N, 8.00. Found: C, 44.80; H, 3.81; N, 7.66.

*4-(Tert-butyl)-N-(4-chlorophenyl)thiazol-2-amine*

$^1$H-NMR: 1.27 (s, 9H), 6.44 (s, 1H), 7.33 (dd, $^3$J=2.1 Hz, $^4$J=6.9 Hz, 2H), 7.65 (dd, $^3$J=2.1 Hz, $^4$J=6.9 Hz, 2H), 10.20 (s, 1H). $^{13}$C-NMR: 14.7, 20.8, 117.7, 118.4, 129.9, 138.8, 142.8, 161.3, 163.9. IR (cm$^{-1}$): 3147, 2923, 2853, 2511, 2030, 1976, 1561, 1529, 1488, 1308, 1218, 1201, 1100, 1091, 1014, 954, 854, 827, 801, 713, 692. HRMS (ESI) Calculated m/z: 267.0717; found m/z: 267.0717 [M+H]$^+$. Anal. Calcd for $C_{13}H_{15}ClN_2S$: C,58.53; H,5.67; N,10.50. Found: C,58.20; H,5.77; N,10.45.

*N-(4-chlorophenyl)-4-phenylthiazol-2-amine*

$^1$H-NMR: 7.31-7.46 (m, 6H), 7.77 (d, $^3$J=8.7 Hz, 2H), 7.92 (d, $^3$J=7.2 Hz, 2H), 10.41 (s, 1H). $^{13}$C-NMR: 103.3, 118.2, 124.4, 125.7, 127.6, 128.6, 128.8, 134.4, 140.0, 150.1, 162.7. IR (cm$^{-1}$): 3378, 3112, 2509, 2159, 2031, 1976, 1585, 1556, 1481, 1442, 1416, 1386, 1323, 1301, 1278, 1215, 1199, 1172, 1090, 1071, 1055, 1025, 1011, 915, 843, 831, 919, 770, 687, 671. HRMS (ESI) Calculated m/z: 287.0404; found m/z: 287.0404 [M+H]$^+$.Anal. Calcd for

$C_{15}H_{11}ClN_2S$: C,62.84; H,3.87; N,9.77. Found: C,62.91; H,3,72; N, 9.85.

*N-(4-chlorophenyl)-4-(naphthalen-2-yl)thiazol-2-amine*

$^1$H-NMR: 7.41-7.45 (m, 2H), 7.48-7.56 (m, 3H), 7.81-7.85 (m, 2H), 7.90-8.08 (m, 4H), 8.46 (s, 1H), 10.49 (s, 1H). $^{13}$C-NMR: 104.2, 118.4, 124.0, 124.3, 124.5, 126.0, 126.4, 127.6, 128.1, 128.2, 128.9, 131.8, 132.5, 133.2, 140.1, 150.0, 162.8. IR (cm$^{-1}$): 3624, 2965, 2521, 2β31, 1976, 1615, 1583, 1571, 1530, 1491, 1097, 815, 806, 770, 743. HRMS (ESI) Calculated m/z: 337.0561; found m/z: 337.0561 $[M+H]^+$. Anal. Calcd for $C_{19}H_{13}ClN_2S$ : C,67.75; H,3.89; N, 8.32. Found: C,67,51; H,3,99; N, 8.24.

*4-(tert-Butyl)-N-(p-tolyl)thiazol-2-amine*

$^1$H-NMR: 1.26 (s, 9H), 2.23 (s, 3H), 6.35 (s, 1H), 7.09 (d, $^3$J=6.9 Hz, 2H), 7.47 (d, $^3$J=6.4 Hz, 2H), 9.91 (s, 1H). $^{13}$C-NMR: 20.3, 29.6, 34.3, 98.7, 116.8, 129.3, 129.7, 139.1, 161.5, 162.8. HRMS (ESI) Calculated m/z: 247.1263; found m/z: 247.1263 $[M+H]^+$. Anal. Calcd for $C_{14}H_{18}N_2S$: C, 68.25; H, 7.36; N, 11.37. Found: C, 68,01; H 7,35; N, 11,30.

*4-(tert-Butyl)-N-(m-tolyl)thiazol-2-amine*

$^1$H-NMR: 1.26 (s, 9H), 2.27 (s, 3H), 6.38 (s, 1H), 6.71-6.75 (m, 1H), 7.16(t, $^3$J=7.7 Hz, 1H), 7.39-7.43 (m, 2H), 9.95 (s, 1H). HRMS (ESI) Calculated m/z: 247.1263; found m/z: 247.1263 [M+H]+. Anal. Calcd for $C_{14}H_{18}N_2S$: C, 68.25; H, 7.36; N, 11.37. Found: C, 68,07; H 7,21; N, 11,35.

*4-(tert-Butyl)-N-(o-tolyl)thiazol-2-amine*

$^1$H-NMR: 1.23 (s, 9H), 2.20 (s, 3H), 6.25 (s, 1H), 6.96 (dd, $^3$J=8.1 Hz, $^4$J=2.1 Hz, 1H), 7.00 (s, 1H), 7.23 (d, $^3$J=8.1 Hz, 1H), 7.63 (s, 1H), 9.03 (s, 1H). $^{13}$C-NMR: 18.1, 29.6, 34.3, 99.1, 120.5, 123.0, 126.4, 128.5, 130.5, 139.7, 161.3, 164.8. HRMS (ESI) Calculated m/z: 247.1263; found m/z: 247.1263 $[M+H]^+$. Anal. Calcd for $C_{14}H_{18}N_2S$: C, 68.25; H, 7.36; N, 11.37. Found: C, 68,13; H 7,31; N, 11,25.

*4-(tert-Butyl)-N-phenylthiazol-2-amine*

$^1$H-NMR: 1.27 (s, 9H), 6.40 (s, 1H), 6.91 (dt, $^3$J=7.4 Hz, $^4$J=1.1 Hz, 1H), 7.26-7.32 (m, 2H), 7.59-7.63 (m, 2H), 10.04 (s, 1H). $^{13}$C-NMR: 29.6, 34.4, 99.1, 116.5, 120.8, 128.7, 141.5, 161.5, 162.5. HRMS (ESI) Calculated m/z: 233.1107; found m/z: 233.1107 $[M+H]^+$. Anal. Calcd for $C_{13}H_{16}N_2S$: C, 67.20; H, 6.94; N, 12.06. Found: C, 67,10; H 6,92; N, 12,25.

*4-(tert-Butyl)-N-(2,4-dimethylphenyl)thiazol-2-amine*

$^1$H-NMR: 1.23 (s, 9H), 2.20 (s, 3H), 2.23 (s, 3H), 6.25 (s, 1H), 6.96 (dd, $^3$J=8.1 Hz, $^4$J=2.1 Hz, 1H), 7.00 (s, 1H), 7.63 (d, $^3$J=8.1 Hz, 1H), 9.03 (s, 1H). $^{13}$C-NMR: 17.9, 20.4, 29.6, 34.3, 98.5, 121.6, 126.9, 129.5, 131.2, 132.5, 137.3, 161.5, 165.7. HRMS (ESI) Calculated m/z: 261.1420; found m/z: 261.1420 $[M+H]^+$. Anal. Calcd for $C_{15}H_{20}N_2S$: C, 69,19; H, 7.74; N, 10.76. Found: C, 68.80; H, 7,53; N 10,48.

*4-(tert-Butyl)-N-(2,3)dimethylphenyl)thiazol-2-amine (423, O4I4)*

$^1$H-NMR: 1.23 (s, 9H), 2.13 (s, 3H), 2.25 (s, 3H), 6.26 (s, 1H), 6.93 (d, $^3$J=7.4 Hz, 1H), 7.05 (t, $^3$J=7.8 Hz, 1H), 7.52 (d, $^3$J=8.0 Hz, 1H), 9.13 (s, 1H). $^{13}$C NMR: 13.8, 20.3, 29.6, 34.5, 120.1, 125.6, 125.7, 128.8, 131.2, 137.2, 139.7, 161.5, 166.1. HRMS (ESI) Calculated m/z: 261.1420; found m/z: 261.1420 $[M+H]^+$. Anal. Calcd for $C_{15}H_{20}N_2S$: C, 69,19; H, 7.74; N, 10.76. Found: C, 68.81; H, 7,63; N 10,49.

*Cell culture*

The generation of HEK-Oct4, NCCIT-Oct4 and NCCIT-Nanog reporter cell lines, as well as HFF-LTR7-EGFP, is known in the art (Cheng et al, J Med Chem 2015, 58 (12), 4976-83; Cheng et al, J Med Chem 2015, 58 (15), 5742-50; Wang et al, Nature 2014, 516 (7531), 405-9).

Human primary fibroblasts were isolated as described elsewhere (approved by the Ethikkommission I Heidelberg S-186/2016) and cultivated in DMEM (Gibco, Germany) containing 10% FBS (Gibco) and 1% Penicillin/Streptomycin (Gibco). NCCIT was in RPMI1640 (Gibco) containing 10% FBS and 1% P/S. Cells were maintained under 5% $CO_2$ at 37 °C in a humidified atmosphere. iPSCs were in Essential®8 medium (life technologies, Germany). Colony expansion

was used for iPSCs maintenance, in which iPSC-like colonies were mechanically selected and isolated in a plate coated with Geltrex® (life technologies); the medium was refreshed very day.

*Luciferase reporter assay*

Luciferase reporter assay was performed using Beatle Juice Kit (PJK, Germany) according to the manufacturer's instruction. NCCIT-Oct and NCCIT-Nanog reporter cells were plated into a 24-well plate (100,000 cells/well) for 24 h. Cells were treated with compounds as indicated and harvested with luciferase lysis buffer (25 mM Tris phosphate buffer pH=7.8, 4 mM EGTA, 1% Triton X-100, 10% glycerol and 2mM fresh DTT, filtered through 0.45 μm sterile filter) at 37°C for 15 min. The protein concentration was determined by Bradford assay (Sigma, Germany). 100 μL reaction mixture containing luciferin and ATP was added to 20 μL cell lysis in a white plate (Gibco, Germany), incubated for 5 min and measured by a luminometer plate-reader. The activity was determined as percent luminous intensity of treated cells over control cells from at least five-independent experiments.

*qRT-PCR*

Quantitative reverse-transcription real-time-PCR was performed using a Light Cycler 96 (Roche, Germany) following the manufacturer's protocol. Briefly, total RNA was isolated from cells using TRIzol (Qiagen, Germany) or NucleoSpin®RNA Plus (Macherey-Nagel, Germany). The same amount of RNA was used to reverse-transcriptional synthesize cDNA by using random primers ProtoScript® First Strand cDNA Synthesis Kit (NEB, Germany). qPCR was performed using the SYBR Green PCR master mix (qPCRBIO SyGreen Mix Lo-Rox, Nippon Genetics, Germany) and the primer pairs indicated below (MWG, Eurofins, Germany). Actin was used as an endogenous control. Data were normalized to the value of untreated cells showing the mean ± SD of quadruplicates and are representative of at least three independent experiments.

*Table 2: PCR primers*

| Gene | Forward | Reverse |
|---|---|---|
| Oct4 | SEQ ID 001: gaaggatgtggtccgagtgt | SEQ ID 002: gtgaagtgagggctcccata |
| Sox2 | SEQ ID 003: aaccccaagatgcacaactc | SEQ ID 004: gcttagcctcgtcgatgaac |
| Nanog | SEQ ID 005: aatacctcagcctccagcagatg | SEQ ID 006: tgcgtcacaccattgctattcttc |
| Lin28A | SEQ ID 007: gtggatgtctttgtgcaccag | SEQ ID 008: gacacggatggattccagac |
| Klf4 | SEQ ID 009: ggacctggactttattctctcc | SEQ ID 010: gataggtgaagctgcaggtg |
| Esrrb | SEQ ID 011: ccttccaatcagctgccttc | SEQ ID 012: gatctgcctgcctctctcat |
| Nodal | SEQ ID 013: gtcgacatcacttgccagac | SEQ ID 014: tggtcggatgaaactcctcc |
| FGF2 | SEQ ID 015: aggagtgtgtgctaaccgtt | SEQ ID 016: cagttcgtttcagtgccaca |
| E-Cadherin | SEQ ID 017: gagagactgggttattcctc | SEQ ID 018: gatgctgtagaaaaccttgcc |
| Rex1 | SEQ ID 019: tggacacgtctgtgctcttc | SEQ ID 020: gtcttggcgtcttctcgaac |
| Sall4 | SEQ ID 021: atttgtgggaccctcgacat | SEQ ID 022: ggtaaaagctcgcccacaaa |
| Actin | SEQ ID 023: ctgactacctcatgaagatcctc | SEQ ID 024: cattgccaatggtgatgacctg |

*Immunoblotting*

iPSCs were lysed using urea-lysis buffer (1 mM EDTA, 0.5% Triton X-100, 5 mM NaF, 6 M Urea, 1 mM Na3VO4, 10 μg/mL Pepstatin, 100 μM PMSF and 3 μg/mL Aprotinin in PBS). Enhanced chemiluminescence (ECL) immunoblot analysis was performed. 40 μg of total protein was resolved on 12% SDS-PAGE gels and immunoblotted with antibodies. β-Actin antibody was used as loading control. Primary antibodies were incubated at 1:1000 dilution in TBS (pH 7.5) with 0.1% Tween-20 and 5% BSA/milk with gentle agitation overnight at 4°C. Secondary antibodies (Dianova, Germany) were incubated in TBS (pH 7.5) with 5% Milk and 0.1% Tween-20 at a 1:10,000 dilution for 1 h at room temperature.

*Table 3: Antibodies used*

| Antibody | Company | Cat# | Antibody | Company | Cat# |
|---|---|---|---|---|---|
| Oct4A (30A3) | Cell signaling | 2840S | β-Catenin (D10A8) XP | Cell signaling | 8480P |
| Sox2 | Cell signaling | 4900 | β-Actin (C4) | santa cruz | sc-47778 |
| Lin28 | epitomics | 3334-1 | Nanog (1 E6C4) | Cell signaling | 4893S |
| E-Cadherin | Cell Signaling | 3195 | Tra-1-60 (S) | Cell signaling | 4746P |
| Tra-1-81 | Cell signaling | 4745P | | | |

*Immunocytochemistry*

iPSCs were seeded in a 96-well plate coated with Geltrex. Cells were fixed with 4% PFA at RT for 15 min, and blocked with blocking buffer (5% goat serum and 0.3% Triton X-100 in PBS) for 1 h. Blocking solution was aspirated and incubated with antibodies in antibody dilution buffer (1% BSA and 0.2% Triton X-100 in PBS) at 4°C overnight. The secondary antibodies (Goat anti-rabbit Alexa Flor 488 and Goat anti-mouse Alexa Flor 594, Dianova) were added and incubated for 1 h. Hoechst 33342 (1 μg/mL in PBS) was used to visualize nuclei.

*FACS*

iPSCs were seeded in a 6-well plate coated with Geltrex. Cells were harvested, fixed and permeabilized (if appropriately) as known in the art (Cheng et al, Mol Oncol. 2016 Jun; 10(6):806-24). Fixed cells were incubated with the primary antibody for 24 h at 4°C and with the secondary antibody for 1 h at room temperature.

*Sample preparation for LC/MS analysis*

NCCIT cells were treated with O4I2 (50 μM) in RPMI 1640 medium containing 2% BSA/FBS. Medium and cells were collected. Cell pellet was solved in 0.1% Triton-x. Protein precipitated by adding MeOH (pH=2) in lysate supernatant on ice for 2 h. Metabolites were extracted from the combined supernatant with ethyl acetate. After removal of organic solvent, residue was dissolved in acetonitrile, frozen immediately in liquid N2 and stored in -80 °C until further analysis.

Additional methods including experimental procedures, chemical synthesis and characterization data can be found in the supplementary information.

*HPLC analysis*

Purity of synthetic products was verified using HPLC analysis on a Jasco HPLC system (PU 2085-plus, Jasco GmbH, Umstadt, Germany) equipped with a porous silica column (ReproSil-Pur ODS-3, dimensions 50x2 mm, Dr. Maisch GmbH, Ammerbuch-Entringen Germany). The synthetic products (10 mM in acetonitrile) were diluted to a final concentration of 25 μM in water. Solvent A consisted of water with 0.1% trifluoroacetic acid and solvent B consisted of acetonitrile with 0.1% trifluoroacetic acid. A linear gradient was applied going from 0% solvent B to 100% solvent B in 7 minutes, followed by another minute of 100% solvent B, after which the system was immediately brought back to 100% solvent A and flushed for 2 minutes. Absorbance was monitored at wavelength of 254 nm. It appeared that O4I2-COOH eluted after approximately 3.65 minutes and O4I2 after approximately 4.50 minutes (see chromatograms in SI 3).

*LC-MS*

Samples obtained after purification were diluted to contain 20% MeOH. 100 μL of the sample was transferred to an HPLC vial and placed in the autosampler of an Agilent 1200 series HPLC machine. Sample (20μL) was injected into the system and metabolites were separated on a C18 column (Kinetex 2.6μm, C18, 100 Å, dimensions100×2.1 mm, Phenomenex, Aschaffenburg, Germany). Solvent A was water with 0.1% trifluoroacetic acid and solvent B was 80% acetonitrile in water with 0.1 % trifluoroacetic acid. Metabolites were separated using a linear gradient from 20% solvent A to 80% solvent B in 40 minutes. Effluent of the column was sprayed directly into the source of the ESI-Q-TOF (electrospray ionization quadrupole time-of-flight) mass spectrometer (micrOTOF, Bruker Daltonics, Bremen, Germany). Scanning mass range was set to m/z 100-1000.

*Episome-mediated reprogramming*

The episomal vectors carrying OCT44 (#41813), SOX2/KLF4 (#27078) and L-MYC/LIN28 (#27080) were purchased from Addgene (USA). To reach the maximal efficiency, episomal expression of EBNA1 (#37624) was co-transfected with OSKML. Reprogramming was performed in 24-well plate as previously reported (Okita et al. Nat Protoc 5, 418-428 (2010); Okita et al. Nat Methods 8, 409-412 (2011); Okita et al. Science 322, 949-953 (2008)). Briefly, 10 000-20 000 cells were seeded in a 24-well plate for 24 h and then treated with O4I3 (0.25 μM) for 48 h. Cells were transfected with OSKML or SKML in presence of O4I4 (5 μM for HF1 and HF2, 20 μM for HF3) for 7 days (HF1 and HF2) or for 5 days (HF3) in DMEM/F12 GlutaMAX knockout medium containing 1% N2, 2% B27 and 100 ng/mL bFGF. Lipofectamine 3000 was used as transfection reagent (Life Technologies). Essential®8 medium was used after 10 days. After 21-28 days, TRA-1-60 staining was conducted as described before (Cheng et al. J Med Chem 58, 4976-4983 (2015); Cheng et al. J Med Chem 58, 5742-5750 (2015)). TRA-1-60 positive iPSCs-like colonies were mechanically and manually isolated and transferred into a 6-well plate coated with Geltrex® or Matrigel in essential®8 medium. Medium was refreshed daily.

*Differentiation of mono-layer CSKML-iPSCs into three germ layers*

Mono layer iPSCs (density 60-80%) in Geltrex-coated 24-well plate were used for differentiation. Differentiation medium (500 mL) contains 5 mL BSA, 20 μL 1-thioglycerol, 10 μg/mL insulin, 15 μg/mL transferrin, 0.1% polyvinyl alcohol in DMEM/F12 Glutamax knockout medium.

Endoderm: Day 1) E8 medium was switched to differentiation medium (DE) additionally containing 100 ng/mL Activin, 100 ng/mL bFGF, 10 ng/mL BMP4, 10 μM Ly294002 and 2.5 μM Chir99021. Day 2) Medium was changed with DE containing 100 ng/mL Activin, 100 ng/mL bFGF, 10 ng/mL BMP4 and 10 μM Ly294002. Day 3) RPMI 1640 medium containing 100 ng/mL Activin and 100 ng/mL bFGF was used.

Mesoderm: E8 medium was switched to differentiation medium (DE) additionally containing 100 ng/mL Activin, 20 ng/mL bFGF, 10 ng/mL BMP4, 10 μM Ly294002 and 5 μM Chir99021. The medium was changed daily for 2-3 days.

Neuroectoderm: E8 medium was switched to differentiation medium (DE) additionally containing 10 μM SB431542, 1 μM LDN193189 and 10 ng/mL bFGF. The medium was changed daily for 14 days.

*In vitro differentiation of CSKML-iPSCs via embryoid body*

To spontaneously differentiate the novel human iPSCS to embryoid bodies (EBs), the differentiation was performed according to a previously published protocol (Wang et al. Nature 516, 405-409 (2014)). Briefly, the novel human iPSCs were cultured on matrigel-coated 6-well plates. Cells from one well were dissociated with collagenase IV (1 mg $ml^{-1}$), and then split into small cell clumps. The small cell clumps were transferred into one 10-cm low-attachment dish, and cultured in EB medium (knockout DMEM, 20% knockout serum replacement, 1 mM L-glutamine, 1% nonessential amino acids, 0.1 mM 2-mercaptoethanol and primocin). The medium was changed every 2 days. The EBs were cultured for 8-10 days followed by collection for RNA isolation and qPCR.

| Gene | Forward | Reverse |
|---|---|---|
| DNMT3B | SEQ ID NO 25: AGAGGGACATCTCACGGTTC | SEQ ID NO 26: GGTTGCCCCAGAAGTATCG |
| DPPA2 | SEQ ID NO 27: GGTGCCAGTTAAAGATGACGC | SEQ ID NO 28: GAGGCAAAATGGTCGGCAAG |
| DPPA4 | SEQ ID NO 29: GACCTCCACAGAGAAGTCGAG | SEQ ID NO 30: TGCCTTTTTCTTAGGGCAGAG |
| FGF4 | SEQ ID NO 31: GCAAGGGCAAGCTCTATGG | SEQ ID NO 32: AGGACTCGTAGGCGTTGTAGTT |
| GDF3 | SEQ ID NO 33: CGCTTTCTCCCAGACCAA | SEQ ID NO 34: GGCAGACAGGTTAAAGTAGAGGAG |
| MYC | SEQ ID NO 35: CACCAGCAGCGACTCTGA | SEQ ID NO 36: GATCCAGACTCTGACCTTTTGC |
| NANOG | SEQ ID NO 37: AGATGCCTCACACGGAGACT | SEQ ID NO 38: GGACTGGTGGAAGAATCAGG |
| POU5F1 | SEQ ID NO 39: CGACCATCTGCCGCTTTG | SEQ ID NO 40: GCCGCAGCTTACACATGTTCT |
| SALL4 | SEQ ID NO 41: AGCACATCAACTCGGAGGAG | SEQ ID NO 42: CATTCCCTGGGTGGTTCACTG |
| SOX2 | SEQ ID NO 43: | SEQ ID NO 44: |

| | | |
|---|---|---|
| | ACAGCAAATGACAGCTGCAAA | TCGGCATCGCGGTTTTT |
| PAX6 | SEQ ID NO 45: GGTTGGTATCCGGGGACTTC | SEQ ID NO 46: TCCGTTGGAACTGATGGAGT |
| SOX1 | SEQ ID NO 47: ACCAGGCCATGGATGAAG | SEQ ID NO 48: CTTAATTGCTGGGGAATTGG |
| ZIC1 | SEQ ID NO 49: CTGGCTGTGGCAAGGTCTTC | SEQ ID NO 50: CAGCCCTCAAACTCGCACTT |
| GATA4 | SEQ ID NO 51: GGAAGCCCAAGAACCTGAAT | SEQ ID NO 52: GTTGCTGGAGTTGCTGGAA |
| GATA6 | SEQ ID NO 53: AATACTTCCCCCACAACACAA | SEQ ID NO 54: CTCTCCCGCACCAGTCAT |
| SOX17 | SEQ ID NO 55: ACGCCGAGTTGAGCAAGA | SEQ ID NO 56: TCTGCCTCCTCCACGAAG |
| EOMES | SEQ ID NO 57: ACCGCCACCAAACTGAGAT | SEQ ID NO 58: TTGTAGTGGGCAGTGGGATT |
| HAND1 | SEQ ID NO 59: AACTCAAGAAGGCGGATGG | SEQ ID NO 60: GGAGGAAAACCTTCGTGCT |
| T | SEQ ID NO 61: ACCCAGTTCATAGCGGTGAC | SEQ ID NO 62: CCATTGGGAGTACCCAGGTT |
| GAPDH | SEQ ID NO 63: agccacatcgctcagacac | SEQ ID NO 64: gcccaatacgaccaaatcc |

*Bisulfite sequencing*

The bisulfite reaction was performed using the EpiTect Bisulfite Kit (Qiagen) according to the manufacturer's instructions. 5ul of bisulfite-treated genomic DNA was used to amplify the *OCT4* a promoter region in human iPSCs and the parental fibroblast. PCR products were cloned into pGEM-T vectors (Promega) and sequenced using the M13 forward primer. Primers used to amplify the OCT4 promoter region were described in a previous publication (Grabundzija et al. Nucleic acids research 41, 1829-1847 (2013)).

| | | |
|---|---|---|
| OCT4_meth | SEQ ID NO 65: GGATGTTATTAAGAT GAAGATAGTTGG | SEQ ID NO 66: CCTAAACTCCCCTT CAAAATCTATT |

*ATAC-Seq*

Cells (50 000) were lysed in a lysis buffer (10 mM Tris HCL pH 7.4, 10 mM NaCl, 3 mM $MgCl_2$, 0.1% IGEPAL-CA630). TransNGS®Tn5 DNA library pre Kit for Illumina was used to prepare DNA library for sequencing.

*Reprogramming*

Epi5® Episomal iPSC Reprogramming Kit (Life technologies) was used to reprogram human fibroblasts according to the manufacturer's protocol. Briefly, 5,000-50,000 cells were seeded in a 24-well plate for 24 h and then transfected with episomal vectors in N2 B27 medium. After 14 days, cells were re-suspended with 0.5 mM EDTA and re-plated in a 6-well plate in essential®8 medium. Colonies were isolated after 3-4 weeks with the help of alkaline phosphatase (AP) live stain (Life technologies). Medium was refreshed daily.

*iPSC single cell expansion*

iPSC single cell expansion was performed according to a well-established protocol known in the art (Watanabe et al, Nat Biotechnol, 2007, 25(6), 681-6). Briefly, iPSCs were dissociated with Accutase® as single cell suspension and re-seeded in a Geltrex-coated 96-well plate in the presence of Rock inhibitor for the first 24 h in combination with/without O4I3. Cell viability was performed in MTT assay as disclosed elsewhere (Cheng et al, Mol Oncol. 2016 Jun; 10(6):806-24).

*Gene expression profiling and data analysis*

Gene expression profiling of human PSCs, iPSCs and fibroblast samples was conducted on the Illumina Human Sentrix-12v4 BeadChip array by the Genomics and Proteomics Core Facility of DKFZ. Two sets of samples were profiled: (i) three hPSC samples, corresponding to treatments with two O4I3 doses (1 nM and 10 nM) and not-treated hPSCs; (ii) three fibroblast samples, corresponding to two treatments (one with O4I3 and one with OSKM) and not-treated fibroblast.

Gene expression analysis was performed in R 3.3.2 computing environment and packages from the open-source software development project Bioconductor 3.4. Clustering and functional enrichment analysis was performed with the standalone tool STEM 1.3.11, while heatmap visualization of gene expression was performed with the package pheatmap 1.0.8. The genome-wide expression profiles of iPSCs were determined using the Human-HT12-V4 Expression BeadChip. Three samples were profiled, corresponding to two O4I3 treatments (one with 1 $\mu$M and one with 10 $\mu$M) and not-treated iPSCs. Quality assessment and pre-processing of raw data was done with the package beadarray 2.24.2. Pre-processing involved default image processing (with median-based local background), default Illumina removal for outlying observations, mean summarization of bead-level observations into probe-level data, quantile normalization of probe-level data and log2 transformation. The resulting probes were matched to genes using annotations from the package illuminaHumanv4.db 1.26.0. After filtering probes that poorly matched the annotated genes (quality status of no-match and bad) as well as low-expression probes (detection score > 0.05), 14109 probes were available for clustering analysis with STEM, a tool for clustering short time series data that can differentiate between real and random temporal expression patterns. For this purpose, one probe per gene (the one with highest variance, if multiple probes matched to single gene) was selected and the data converted into fold changes with respect to the not-treated iPSCs. The data was analyzed as short time series of length three (corresponding to dose level of 0, 1, and 10 $\mu$M), by treating dose level as time factor. STEM was applied in default mode, except for the following settings: at least one measurement with absolute fold change of 0.65; significance of model profiles corrected by false discovery rate method; cellular component terms, terms with evidence code IEA and NAS or terms that had less than 10 genes in-common with the identified model profiles (or clusters of similar model profiles) were excluded from the Gene Ontology (GO) enrichment analysis; multiple testing correction of GO term significance values were determined by the randomization test, with 5000 randomly sampled gene sets per term.

Fibroblast/iPSC experiment data analysis; approximately 18 000 probes, after filtering, were available for differentially expression analysis. The latter was based on fold changes, i.e., the probes with at least two-fold expression relative to the untreated fibroblast were selected as differentially expressed; since single biological replicates per treatment were used, statistical tests were not applicable. Global gene expression comparison between pairs of samples were visualized by scatter plots, plotted with the package ggplot2 2.2.1, while the heatmaps of gene expressions were visualized with the package ComplexHeatmap 1.12.0. In addition, columns and rows in the heatmaps were reordered with hierarchical clustering based on average linkage and Euclidean distance or Pearson correlation. Functional enrichment analysis on differentially expressed probes was performed with gProfileR version r1732_e89_eg36 via the R interface in the package gProfileR version 0.6.1. More specifically, the query lists were treated as unordered human gene lists, and the set of probes retained after filtering was supplied as a background. Multiple testing correction was done by the FDR method according to Benjamini and Hochberg with 0.05 FDR cut-off, including biological processes and molecular functions from the Gene Ontology database with size of 5 up to 500 genes.

*Analysis of H3K4Me3 activation*

Fibroblasts were treated with O4I3 0.5 μM for 2 days in the presence/absence of reprogramming transcription factors. The expression of H3K4Me3 was visualized by using H3K4Me3 antibody as described before for immunocytochemistry. The signal intensity was analyzed using ImageJ software as single cell level randomly selected from at least three independent experiments.

*Statistics*

Statistical and graphical analyses of the data (except DNA microarray) were performed using Microsoft Excel 2007 software. The statistical significance of compared measurements was performed by using the Student's one-tailed or two-tailed t-test, considering P=0.05 as statistically significant.

*TRA-1-60+ colonies counting*

To assess the number of TRA-1-60+ colonies, 10,000 fibroblast cells/well in 96-well plates were transfected with episomal OSKM (Epi5 kit, Thermofisher Scientific) in the presence/absence of small molecules, as indicated in the text. Immunostaining was performed using TRA-1-60 antibody in living cells after 32 days. Transfection efficiency was indicated by quantifying the expression of EGFP (Addgene plasmid # 27082).

*iPSCs differentiation*

Cellartis iPS cell to hepatocyte differentiation system (Takara, Germany) was used for differentiation of iPSCs to endoderm. StemDiff neuron differentiation kit (08500, Stem Cell Technologies, Germany) was used for differentiation of iPSCs to neuroectoderm. To differentiate iPSCs to mesoderm, cells were seeded in Geltrex-coated plates as single cells and cultivated till the density reached to at least 80%. N2+B27 medium was used to replace E8 medium. Activin (100 ng/mL), bFGF (20 ng/mL), BMP4 (10 ng/mL), Ly2 (10 μ M) and Chir (5 μ M) were freshly added. The medium was changed every two days for 14 days.

*Measurement of methylated H3K4 and the in vitro activity of LSD1, KDM4, and KDM5 demethylases*

Epiquik histone demethylase (H3K4 specific) activity/inhibition assay kit (Epigentek, Biocat, Germany) was used to measure the protective effect of O4I3 and zolpidem on the methylation of H3K4. Their inhibitory effects on LSD1, KDM5s, and KDM4 were determined using Epigenase jarid demethylase activity/inhibition assay kit, Epigenase JMJD2 demethylase activity/inhibition assay kit and EpiQuik histone demethylase LSD1 activity/inhibition assay kit (Epigentek, Biocat, Germany). The total nuclear extraction was used according to manufacturer's instructions. For measuring KDM5A, KDM5B, KDM5C, and KDM5D activity, magnetic beads were used to immunoprecipitate the respective enzymes from $10^8$ cells according to the manufacturer's instructions (Cell Signaling Technologies).

*Chromatin immunoprecipitation assay*

SimpleChIP Plus Enzymatic Chromatin IP Kit (Cell Signaling Technologies) was used to isolate DNA precipitated with H3K4Me3 or H3K27Me3 antibody. According to the manufacturer's protocol, $10^6$ cells/sample were collected. Non-target Rabbit IgG and total Histone H3 antibodies (provided in the kit) were used as negative and positive controls, respectively. KDM5A-D enzymes were purified using immunoprecipitation with magnetic beads according to the manufacture' s protocol. Recruited DNA was subjected to qRT-PCR using ChIP primers sets, and human RPL30 exon 3

primer pairs were used as positive control.

*C. elegans strains and cultural condition*

Three strains N2 (wild type), TJ356 [daf-16p::daf-16a/b::GFP + rol-6(su1006)] and CF1038 [daf-16(mu86)] were offered by Caenorhabditis Genetics Center (CGC, University of Minnesota, USA). All strains were maintained on nematode growth media (NGM) agar, which content *Escherichia coli* OP50 as a nutrient source and incubated at 20°C.

*Worm Synchronization*

Eggs were released by lysing gravid adults with bleaching for 10 min. Resuspended eggs in sterile M9 buffer and incubated at 20°C for 16-18 h. After all the worms were synchronized at the same L1 larval stage, transferred them into the liquid S-media which contents E. coli OP50 (OD600=0.8-1.2). Then, synchronized worms were treated with DMSO, 50 μg/ml epigallocatechin gallate (EGCG) or O4I4 (5 μM, 10 μM, and 20 μM) for further experiments.

*Intracellular ROS*

2'-7'-dichlorofluorescein diacetate ($H_2$DCFDA) is a broadly known substance to quantify the ROS (reactive oxygen species) in *C. elegans.* After $H_2$DCFDA (non-fluorescent) penetrates the cell membrane, it will be turned into a highly fluorescent dye 2'-7'-dichlorofluorescein (DCF) via the biochemical reaction of enzymatically deacetylated and oxidization by ROS. Due to the fluorescent intensity, the ROS level in *C. elegans* can be estimated.

The strains N2 (wild type) and CF1038 (daf-16 mutant) were synchronized and treated as described before. 50 μM $H_2$DCFDA was added into all samples after incubated 48 h. Samples were covered by foil paper to avoid the light and incubated another 1 h. Afterward, 10 μl worms were transferred on a glass slide and paralyzed with 10μl of 10 mM sodium azide. BIOREVO BZ9000 fluorescence microscope was used to detect the production of DCF, and Image J 1.52a could analyze the ROS level in *C. elegans* via estimating the densitometrically.

*Survival assay*

After 48 h treatment, around 60 wild-type *C. elegans* (N2) worms in each sample were transferred into a new liquid S-media which contains 80 μM of superoxide-generating compound juglone (5-hydroxy-1,4-naphthalenedione) for 24 h. If a worm has response via a gentle touch with platinum wire, it is considered as a live worm. Conversely, a dead worm was considered by no response. The function of the survival rate is shown below.

$$\frac{live\ worms}{total\ worms} x\ 100\% = Survival\ Rate(\%)$$

*daf-16 localization assay*

In this experiment, transgenic worms TJ356 (Daf-16::GFP) are employed to study our target substance affects the localization of daf-16. After 24 h treatment, worms were transferred to a glass slide and paralyzed with 10 mM sodium azide. BZ9000 fluorescence microscope was used to observe the localization of daf-16. In each sample, around 50 worms were scored for three localization patterns: cytoplasmic, intermediate and nuclear.

*Lifespan assay*

O4I4 (0.5 μM) were continuously added in medium until all the worms die. During the experiment period, every two days or three days, worms were counted either live or die by responding to a gentle touch with a platinum wire. Dead worms were discarded, and live worms were transferred to a new NGM plate to ensure they do not go through the dauer stage.

## Claims

**1.** A method of producing a pluripotent stem cell, in particular an induced pluripotent stem cell, said method comprising contacting a non-pluripotent donor cell obtained from a mammalian (particularly a human) donor with a compound **characterized by** general formula (1),

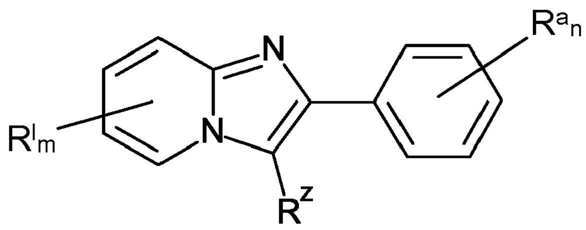

(1),

wherein

- m is 1 or 2,
- each $R^l$, independently from any other $R^l$, is selected from -H, substituted or unsubstituted $C_1$-$C_3$ alkyl, substituted or unsubstituted $C_1$-$C_3$ alkoxy, -F, -Cl, -Br, -I, $-NO_2$, -OH, $-NH_2$,$-SO_3H$, -CN, substituted or unsubstituted $C_1$-$C_3$ carboxylic acid, -NCO, -CNO, -NCS, -SCN, $-CH_2F$, $-CHF_2$, or $-CF_3$,
- $R^z$ is selected from:

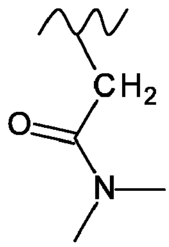

(2), or -H,

- n is 1 or 2,

  • each $R^a$ independently from any other $R^a$ is selected from -H, substituted or unsubstituted $C_1$-$C_3$ alkyl, substituted or unsubstituted $C_1$-$C_3$ alkoxy, -F, -Cl,-Br, -I, $-NO_2$, -OH, $-NH_2$, $-SO_3H$, -CN, substituted or unsubstituted $C_1$-$C_3$ carboxylic acid, -NCO, -CNO, -NCS, -SCN, $-CH_2F$, $-CHF_2$, or $-CF_3$,
  or
  • two $R^a$ on adjacent positions are linked by $C_4H_4$.

**2.** The method according to claim 1, wherein the compound is **characterized by** general formula (3),

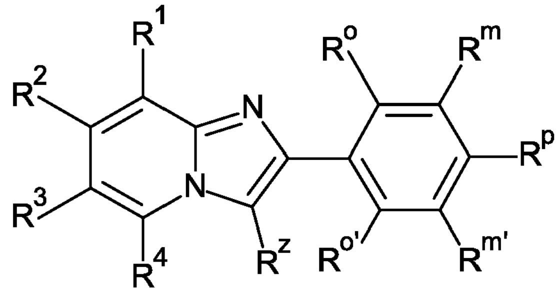

(3),

wherein

- one of $R^1$, $R^2$, $R^3$ and $R^4$ is selected from:
-H, substituted or unsubstituted $C_1$-$C_3$ alkyl, substituted or unsubstituted $C_1$-$C_3$ alkoxy, -F, -Cl, -Br, -I, $-NO_2$, -OH, $-NH_2$, $-SO_3H$, -CN, substituted or unsubstituted $C_1$-$C_3$ carboxylic acid, -NCO, -CNO, -NCS, -SCN, $-CH_2F$, $-CHF_2$, or $-CF_3$,
and the other ones are -H.
- $R^z$ is selected from general formula (2) or -H,
- one of $R^o$, $R^m$, $R^p$, $R^{m'}$ and $R^{o'}$ is selected from:
- H, substituted or unsubstituted $C_1$-$C_3$ alkyl, substituted or unsubstituted $C_1$-$C_3$ alkoxy,-F, -Cl, -Br, -I, $-NO_2$,

-OH, $-NH_2$, $-SO_3H$, -CN, substituted or unsubstituted $C_1$-$C_3$ carboxylic acid, -NCO, -CNO, -NCS, -SCN, $-CH_2F$, $-CHF_2$, or $-CF_3$,
and the other ones are
- H, or
- two neighbouring positions selected from $R^o$, $R^m$ and $R^p$ together are $C_4H_4$.

**3.** The method according to any one of claims 1 or 2, wherein

- each $R^l$, $R^a$, $R^1$, $R^2$, $R^3$, $R^4$, $R^o$, $R^m$, $R^p$, $R^{m'}$ and $R^{o'}$ is independently selected from -H, $-CH_3$, -F, -Cl, -Br, -I, -OH, $-NO_2$, -CN or -OMe.

**4.** The method according to any one of the above claims 1 to 3, wherein

- $R^1$ is selected from $-CH_3$, and -H;
- $R^2$ is selected from $-CH_3$, and -H;
- $R^3$ is selected from $-CH_3$, -H, -F, -Cl, and -Br;
- $R^4$ is selected from $-CH_3$, and -H;
- $R^o$ and $R^{o'}$ are selected from $-NO_2$, -F, and -H;
- $R^p$ is selected from $-CH_3$, -F, -Cl, -Br, $-NO_2$, -OMe, -OH, -CN, and -H;
- $R^m$ and $R^{m'}$ are selected from -Cl, -H, -F, -OH, $-NO_2$, and -OMe, and/or
- $R^z$ is selected from -H and formula (2).

**5.** The method according to any one of the above claims, wherein the compound according to formula (1) is

a) N,N-dimethyl-2-(6-methyl-2-p-tolylimidazo[1,2-a]pyridine-3-yl)acetamid (100),
b) 6-methyl-2-(p-tolyl)imidazo[1,2-a]pyridine (101),
c) 2-(4-fluorophenyl)-6-methyl-imidazo[1,2-a]pyridine (102),
d) 2-(4-chlorophenyl)-6-methyl-imidazo[1,2-a]pyridine (103),
e) 2-(4-bromophenyl)-6-methyl-imidazo[1,2-a]pyridine (104),
f) 6-methyl-2-(4-nitrophenyl)imidazo[1,2-a]pyridine (105),
g) 2-(4-methoxyphenyl)-6-methyl-imidazo[1,2-a]pyridine (106),
h) 4-(6-methylimidazo[1,2-a]pyridin-2-yl)phenol (107),
i) 4-(6-methylimidazo[1,2-a]pyridin-2-yl)benzonitrile (108),
j) 6-methyl-2-phenyl-imidazo[1,2-a]pyridine (109),
k) 6-methyl-2-(3-nitrophenyl)imidazo[1,2-a]pyridine (110),
l) 6-methyl-2-(2-nitrophenyl)imidazo[1,2-a]pyridine (111),
m) 2-(3-fluorophenyl)-6-methyl-imidazo[1,2-a]pyridine (112),
n) 2-(2-fluorophenyl)-6-methyl-imidazo[1,2-a]pyridine (113),
o) 2-(3-chlorophenyl)-6-methyl-imidazo[1,2-a]pyridine (114),
p) 3-(6-methylimidazo[1,2-a]pyridin-2-yl)phenol (115),
q) 2-(3-methoxyphenyl)-6-methyl-imidazo[1,2-a]pyridine (116),
r) 7-methyl-2-(p-tolyl)imidazo[1,2-a]pyridine (117),
s) 8-methyl-2-(p-tolyl)imidazo[1,2-a]pyridine (118),
t) 5-methyl-2-(p-tolyl)imidazo[1,2-a]pyridine (119),
u) 6-chloro-2-(p-tolyl)imidazo[1,2-a]pyridine (120),
v) 6-fluoro-2-(p-tolyl)imidazo[1,2-a]pyridine (121),
w) 6-bromo-2-(p-tolyl)imidazo[1,2-a]pyridine (122),
x) 6-methyl-2-(2-naphthyl)imidazo[1,2-a]pyridine (123).

**6.** A method of producing a pluripotent stem cell, in particular a method of producing an induced pluripotent stem cell, said method comprising contacting a non-pluripotent donor cell obtained from a mammalian (particularly a human) donor with a compound **characterized by** general formula (4),

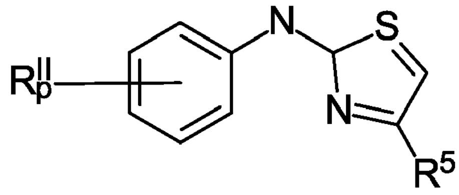

(4),

wherein

- each $R^{II}$, independently from any other $R^{II}$, is selected from

  - -H,
  - substituted or unsubstituted $C_1$-$C_3$ alkyl, particularly methyl,
  - substituted or unsubstituted $C_1$-$C_3$ alkoxy, particularly O-$CH_3$,
  - -F, -Cl, -Br, -I, -$NO_2$, -OH, -$NH_2$, -$SO_3H$, -CN, -NCO, -CNO, -NCS, -SCN, particularly -F, -Cl, -Br, -$NO_2$, -OH, and -CN,
  - substituted or unsubstituted $C_1$-$C_3$ carboxylic acid, -$CH_2F$, -$CHF_2$, or -$CF_3$, particularly COOH;

- p is 1, 2 or 3, particularly p is 1 or 2;
- $R^5$ is selected from

  - -H,
  - substituted or unsubstituted $C_1$-$C_4$ alkyl, particularly t-butyl,
  - substituted or unsubstituted aryl, particularly unsubstituted phenyl or naphtyl;
  - COOR, wherein R is an unsubstituted or substituted $C_1$-$C_4$ alkyl, particularly R is an unsubstituted $C_1$-$C_4$ alkyl, more particularly R is ethyl;

with the proviso that the following combinations are not encompassed in the definition of compound (4):

- $R^5$ is COOR with R being an unsubstituted or substituted $C_1$-$C_4$ alkyl, particularly R is an unsubstituted $C_1$-$C_4$ alkyl, more particularly R is ethyl, and
- $R^{II}$ is halogen or hydrogen and p is selected from 1 or 2.

**7.** The method according to claim 6, wherein the compound according to formula (4) is the compound according to formula (5), (6) or (7):

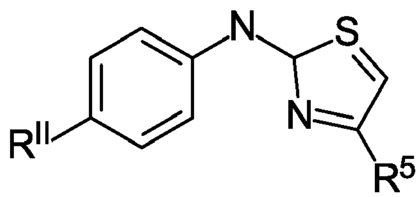

(5)

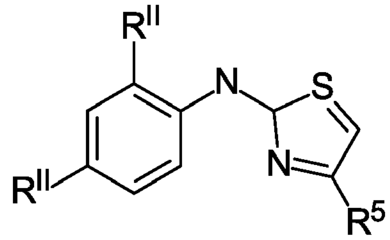

(6)

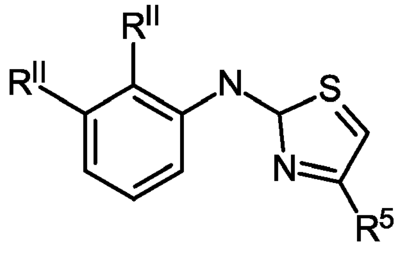

(7)

wherein $R^{II}$ and $R^5$ have the same meanings as indicated in claim 6.

**8.** The method according to claim 6 or 7, wherein each $R^{II}$, independently from any other $R^{II}$, is selected from methyl and ethyl, particularly all $R^{II}$ are methyl.

**9.** The method according to any one of claims 6 to 8, wherein $R^5$ is selected from

- substituted or unsubstituted $C_3$-$C_4$ alkyl, particularly unsubstituted t-butyl, and
- substituted or unsubstituted aryl, particularly unsubstituted phenyl or naphtyl.

**10.** The method according to claim 6, wherein the compound according to formula (4) is selected from any one of the formulas (419), (420), (421), (422) and (423), particularly wherein the compound according to formula (4) is (423)

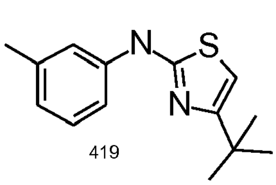
419

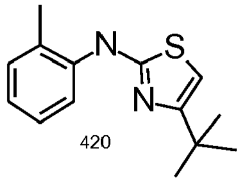
420

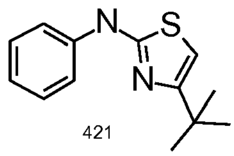
421

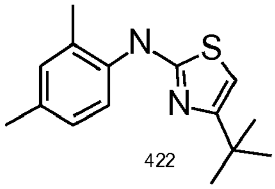
422

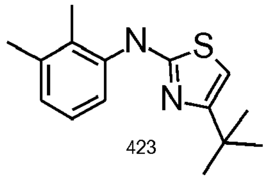
423

**11.** The method according to any one of the above claims, wherein the method further comprises introducing into said mammalian non-pluripotent donor cell at least one element selected from:

- a nucleic acid sequence encoding a polypeptide,
- a polypeptide,
- a non-coding nucleic acid sequence,

wherein said polypeptide is selected from:

- the family of OCT polypeptides, in particular OCT4,
- the family of KLF polypeptides, in particular KLF4,
- the family of SOX polypeptides, in particular SOX2,
- the family of MYC polypeptides, in particular L-MYC, and
- the family of LIN polypeptides, in particular Lin28.

and wherein said non-coding nucleic acid sequence is selected from miR-302b, miR-372, miR-302, miR-367, miR-200c and miR-369.

**12.** A method of inducing OCT4 and/or NANOG in a mammalian cell, or a method of increasing methylation of histone 3 lysine 4 (H3K4) in a mammalian non-pluripotent donor cell to augment a method of producing an induced pluripotent stem cell, said method comprising contacting a mammalian donor cell with a compound as specified in any one of claims 1 to 10.

**13.** A method of maintaining pluripotency in mammalian pluripotent stem cells comprising contacting said mammalian pluripotent stem cell with a compound as specified in any one of claims 1 to 10.

**14.** The method according to any one of the above claims, wherein said compound is used in a final concentration between 0.0005 μmol/l and 100 μmol/l, in particular between 0.001 μmol/l and 15 μmol/l, more particular between 0.001 μmol/l and 10 μmol/l.

**15.** A cell culture medium comprising a compound as specified in any one of claims 1 to 10.

Fig. 1

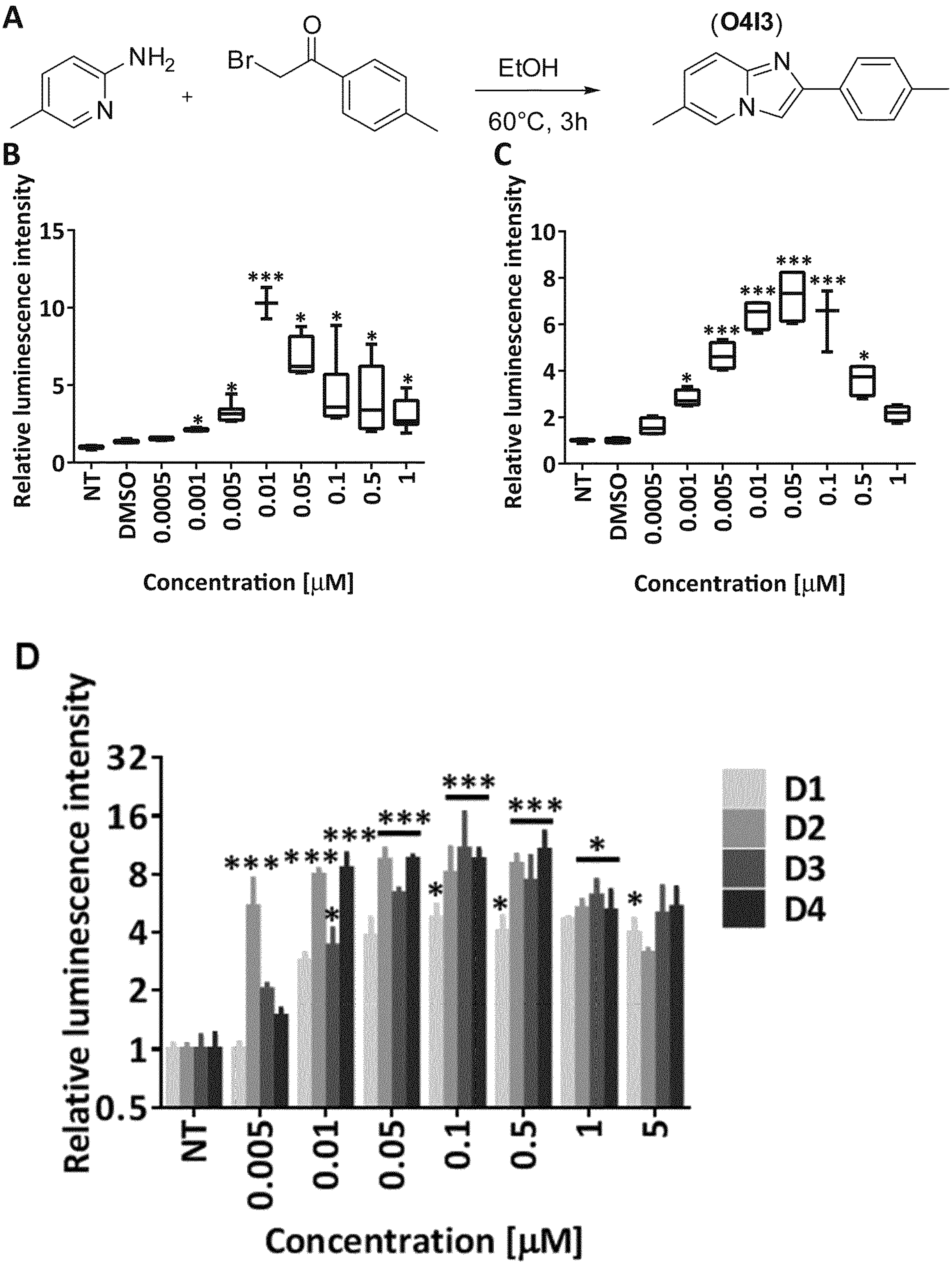
A
(O4I3)
EtOH
60°C, 3h
B
C
Relative luminescence intensity
Concentration [μM]
NT
DMSO
0.0005
0.001
0.005
0.01
0.05
0.1
0.5
1
D
Relative luminescence intensity
Concentration [μM]
NT
0.005
0.01
0.05
0.1
0.5
1
5
D1
D2
D3
D4

Fig. 2

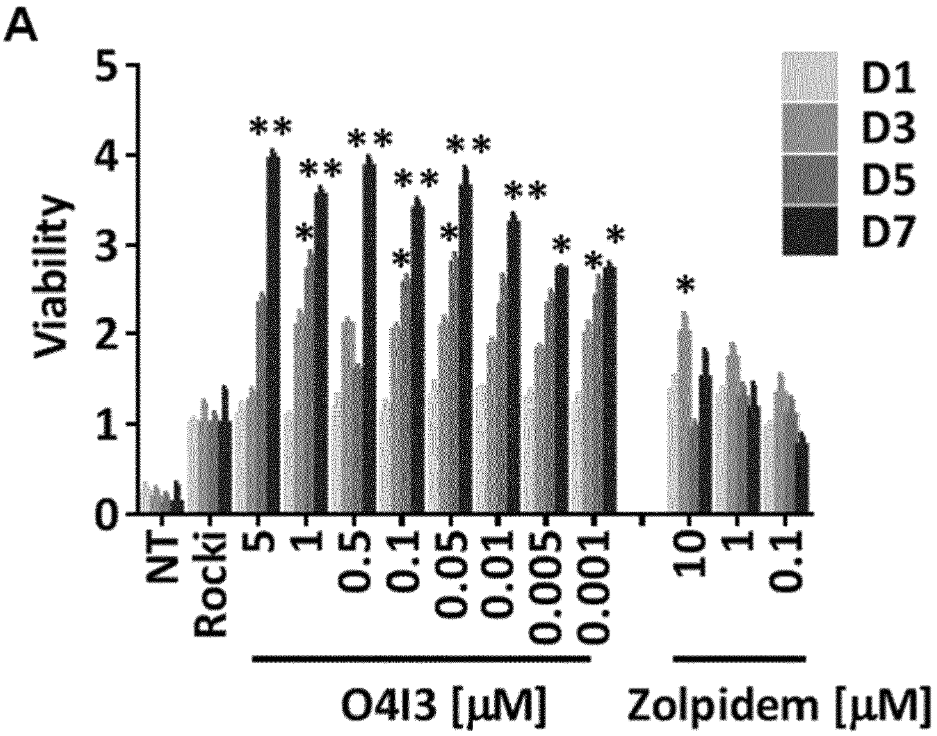
A
Viability
5
4
3
2
1
0
D1
D3
D5
D7
NT
Rocki
5
1
0.5
0.1
0.05
0.01
0.005
0.001
10
1
0.1
O4I3 [μM]
Zolpidem [μM]

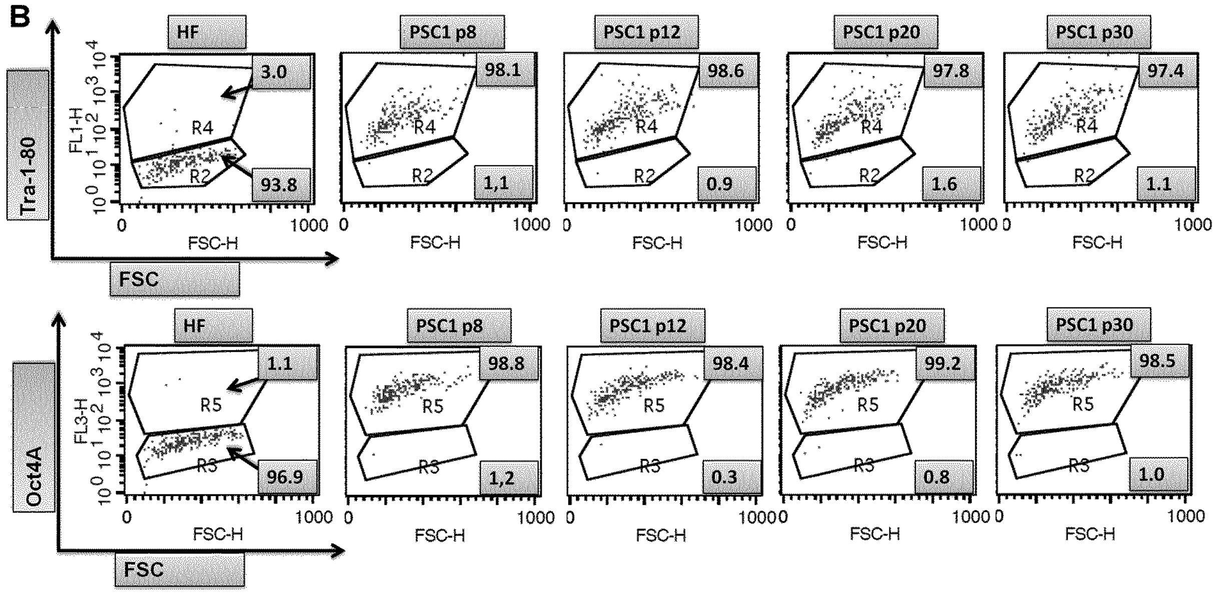
B
Tra-1-80
FSC
HF
PSC1 p8
PSC1 p12
PSC1 p20
PSC1 p30
FL1-H
FSC-H
R4
R2
3.0
93.8
98.1
1,1
98.6
0.9
97.8
1.6
97.4
1.1
Oct4A
FSC
FL3-H
R5
R3
1.1
96.9
98.8
1,2
98.4
0.3
99.2
0.8
98.5
1.0

C

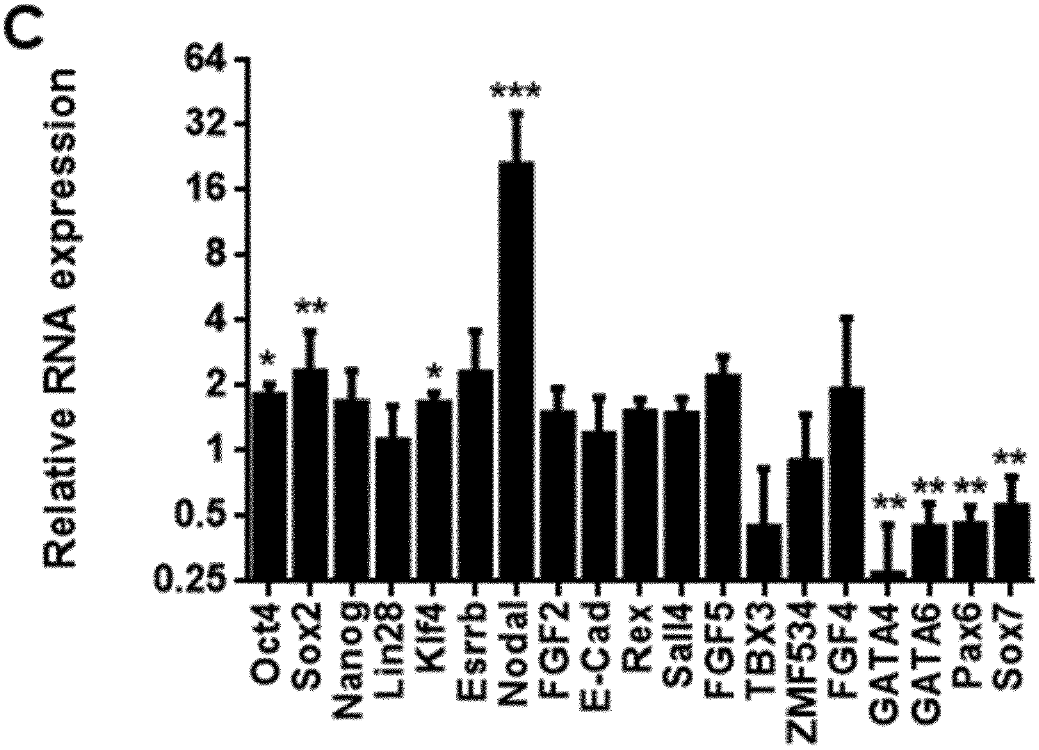
Relative RNA expression
64
32
16
8
4
2
1
0.5
0.25
*
**
*
***
**
** ** **
**
Oct4
Sox2
Nanog
Lin28
Klf4
Esrrb
Nodal
FGF2
E-Cad
Rex
Sall4
FGF5
TBX3
ZMF534
FGF4
GATA4
GATA6
Pax6
Sox7

D

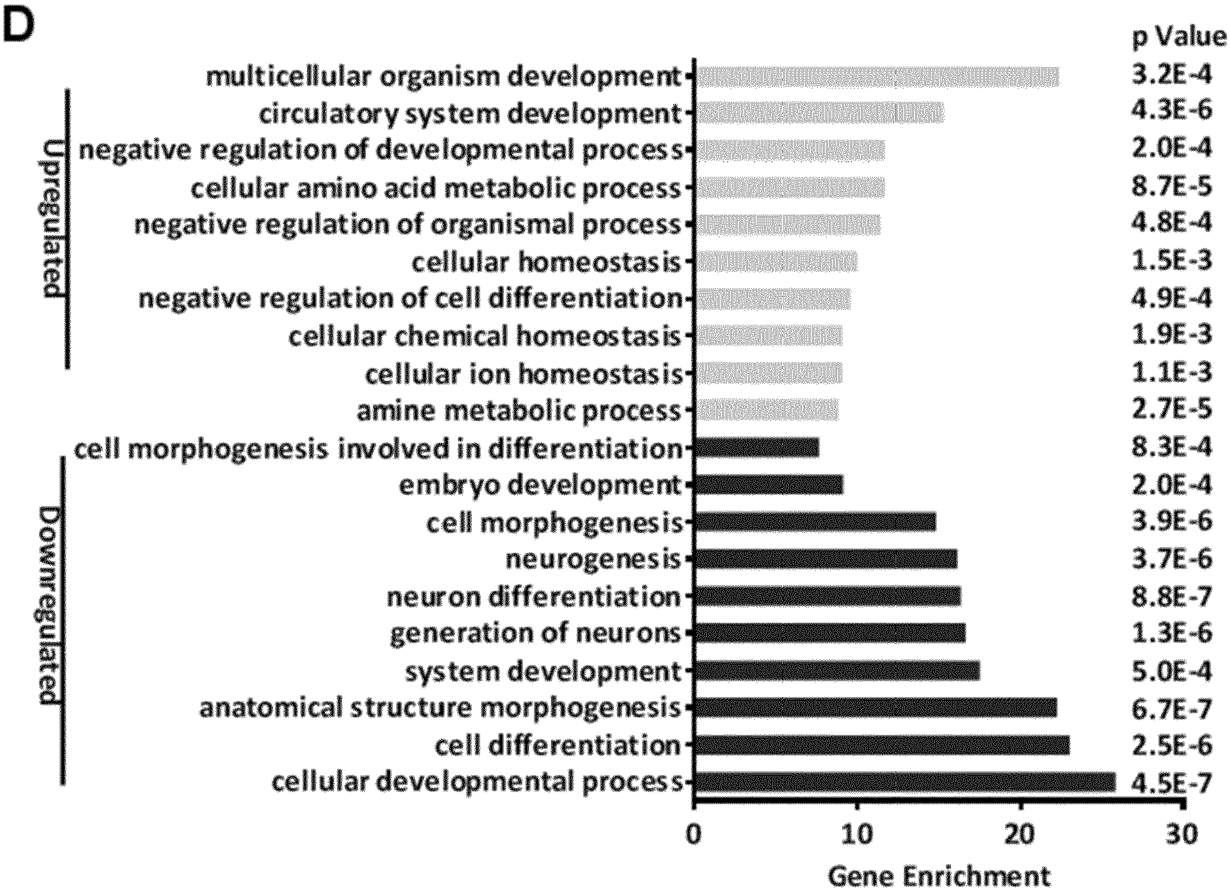
p Value
Upregulated
multicellular organism development 3.2E-4
circulatory system development 4.3E-6
negative regulation of developmental process 2.0E-4
cellular amino acid metabolic process 8.7E-5
negative regulation of organismal process 4.8E-4
cellular homeostasis 1.5E-3
negative regulation of cell differentiation 4.9E-4
cellular chemical homeostasis 1.9E-3
cellular ion homeostasis 1.1E-3
amine metabolic process 2.7E-5
Downregulated
cell morphogenesis involved in differentiation 8.3E-4
embryo development 2.0E-4
cell morphogenesis 3.9E-6
neurogenesis 3.7E-6
neuron differentiation 8.8E-7
generation of neurons 1.3E-6
system development 5.0E-4
anatomical structure morphogenesis 6.7E-7
cell differentiation 2.5E-6
cellular developmental process 4.5E-7
0
10
20
30
Gene Enrichment

Fig. 3

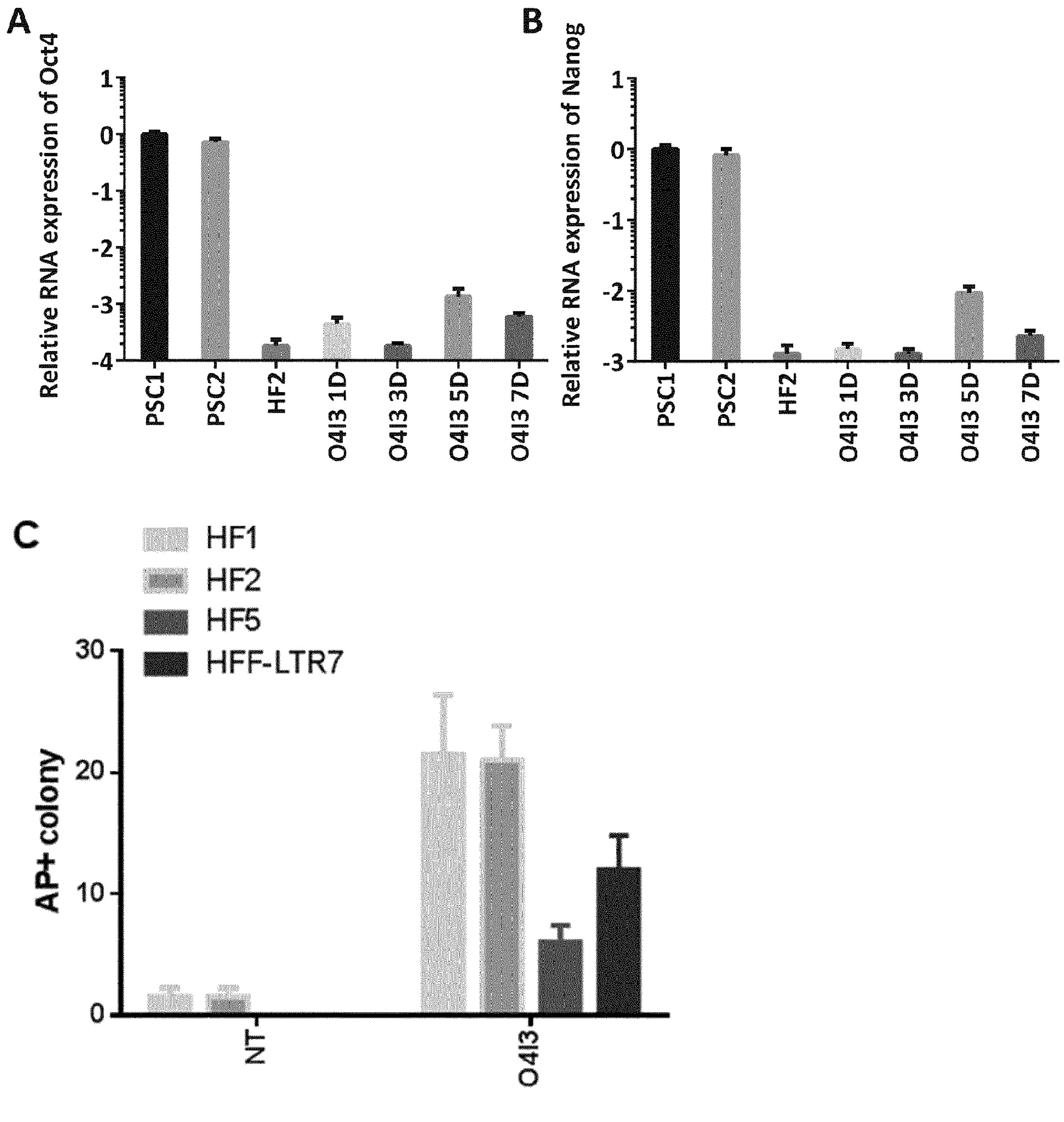
A
Relative RNA expression of Oct4
1
0
-1
-2
-3
-4
PSC1
PSC2
HF2
O4I3 1D
O4I3 3D
O4I3 5D
O4I3 7D
B
Relative RNA expression of Nanog
1
0
-1
-2
-3
PSC1
PSC2
HF2
O4I3 1D
O4I3 3D
O4I3 5D
O4I3 7D
C
HF1
HF2
HF5
HFF-LTR7
AP+ colony
30
20
10
0
NT
O4I3

Fig. 4

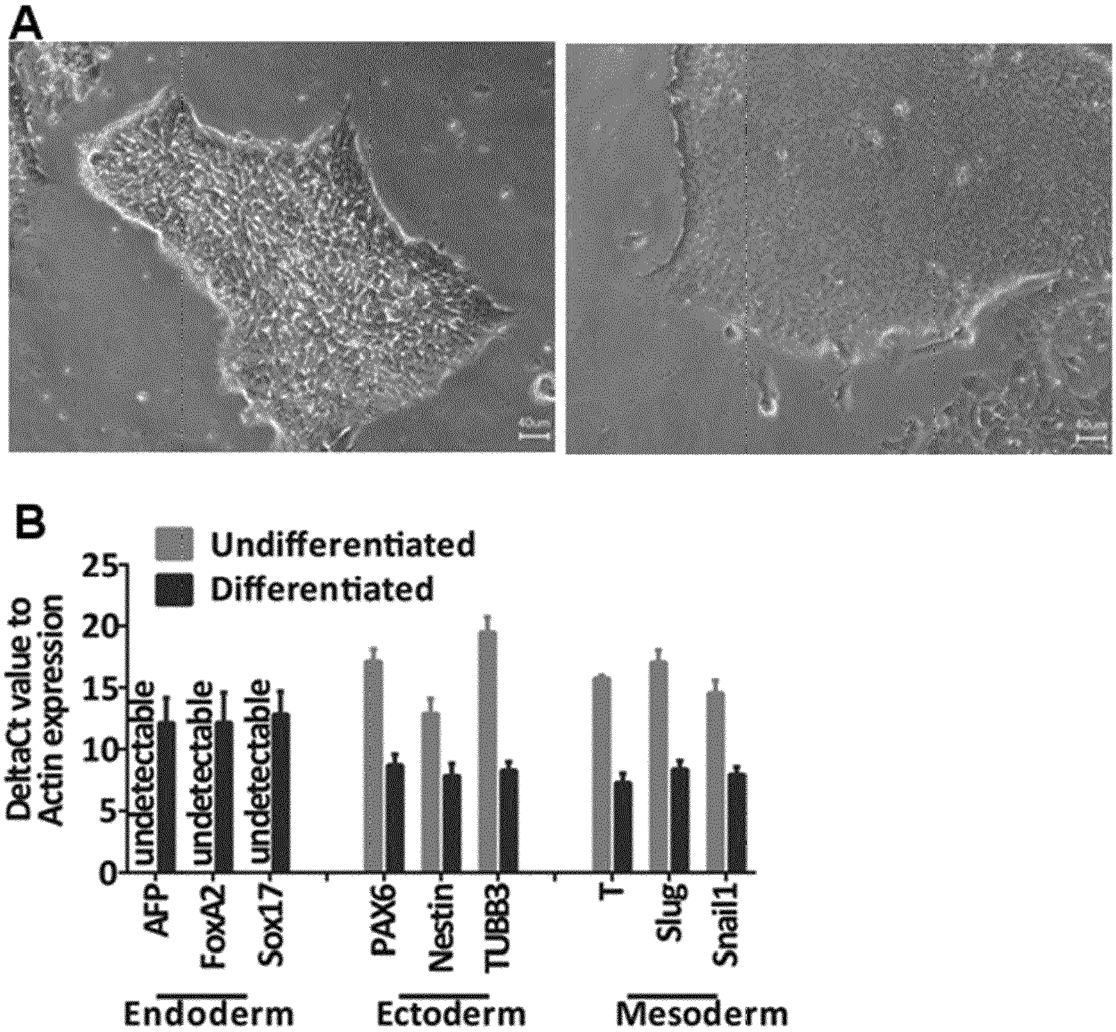
A
B
Undifferentiated
Differentiated
DeltaCt value to Actin expression
25
20
15
10
5
0
undetectable
undetectable
undetectable
AFP
FoxA2
Sox17
PAX6
Nestin
TUBB3
T
Slug
Snail1
Endoderm
Ectoderm
Mesoderm

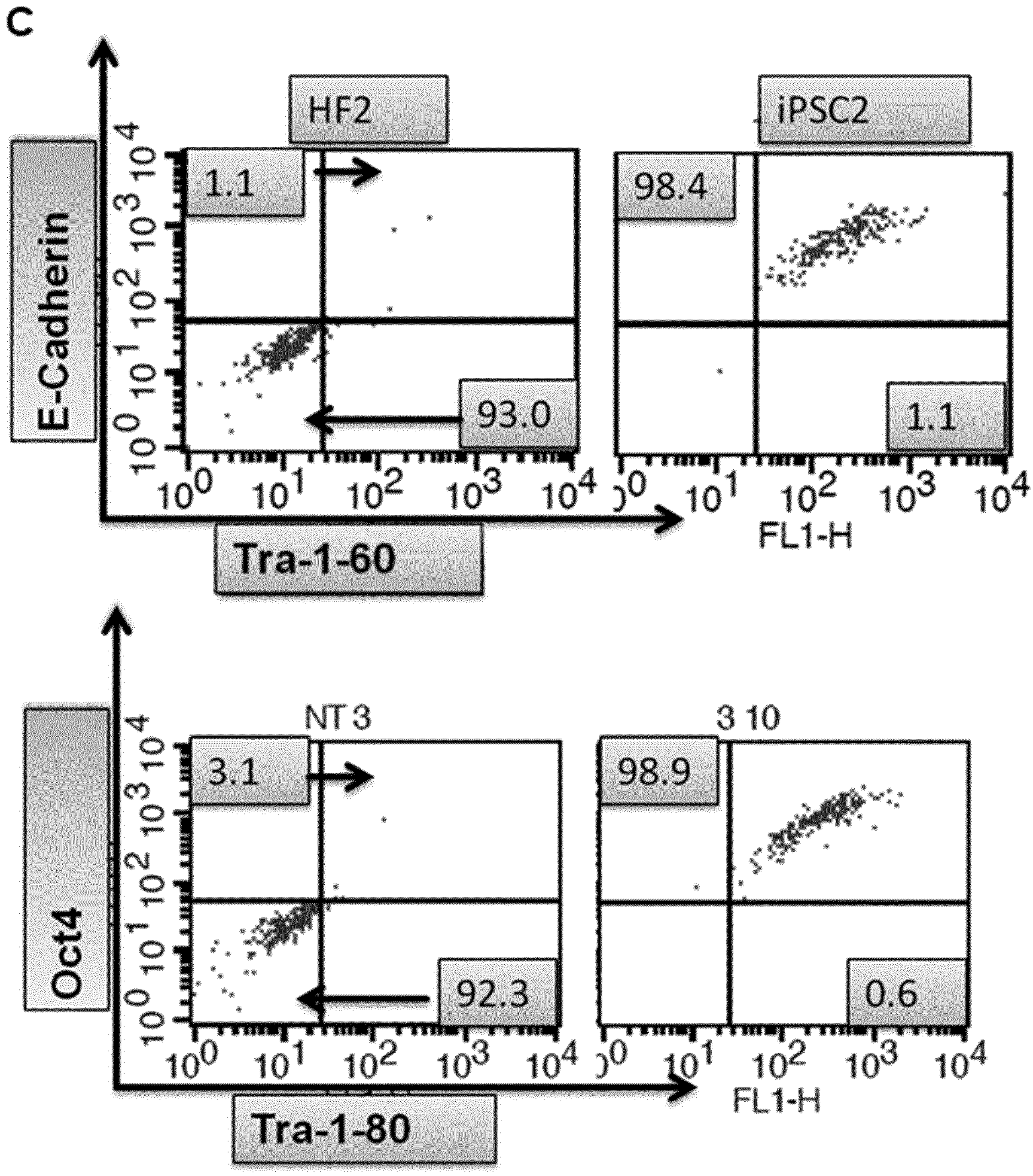
C
HF2
iPSC2
E-Cadherin
1.1
93.0
98.4
1.1
Tra-1-60
FL1-H
NT 3
3 10
Oct4
3.1
92.3
98.9
0.6
Tra-1-80
FL1-H

D

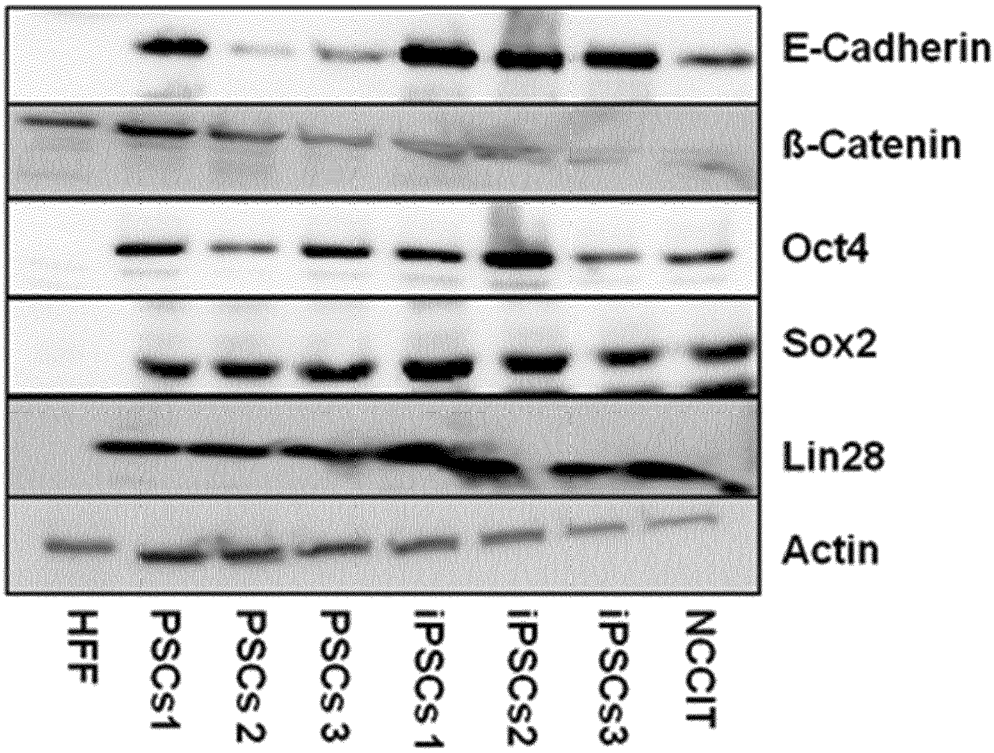
E-Cadherin
ß-Catenin
Oct4
Sox2
Lin28
Actin
HFF
PSCs1
PSCs 2
PSCs 3
iPSCs 1
iPSCs2
iPSCs3
NCCIT

E

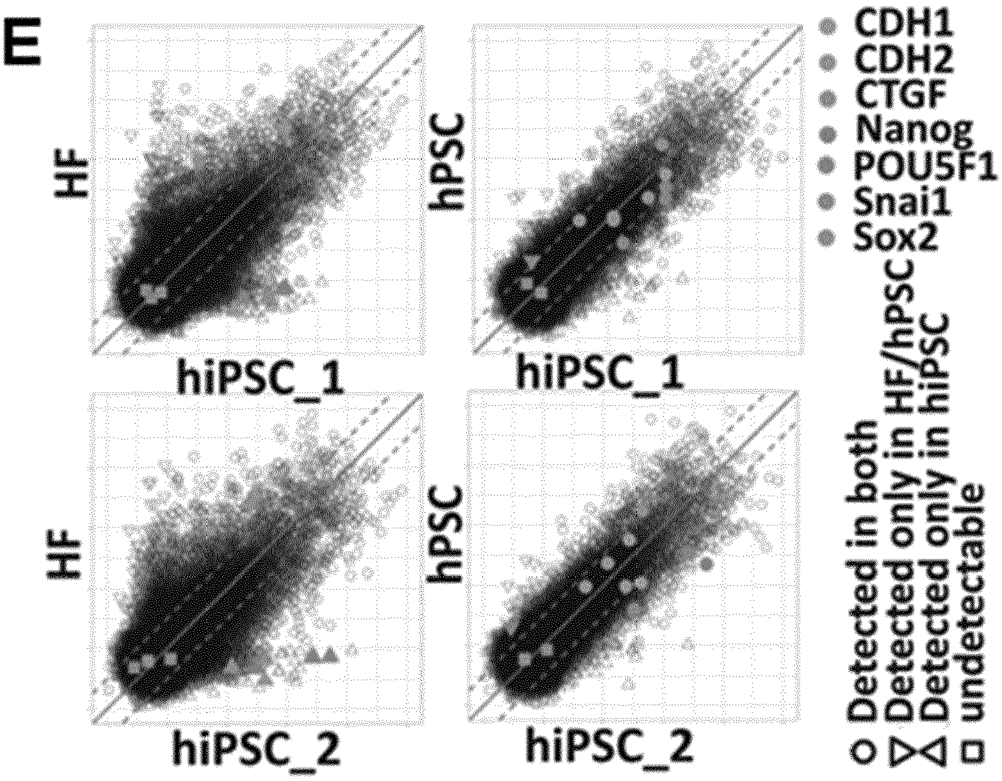
HF
hiPSC_1
hPSC
hiPSC_1
HF
hiPSC_2
hPSC
hiPSC_2
CDH1
CDH2
CTGF
Nanog
POU5F1
Snai1
Sox2
Detected in both
Detected only in HF/hPSC
Detected only in hiPSC
undetectable

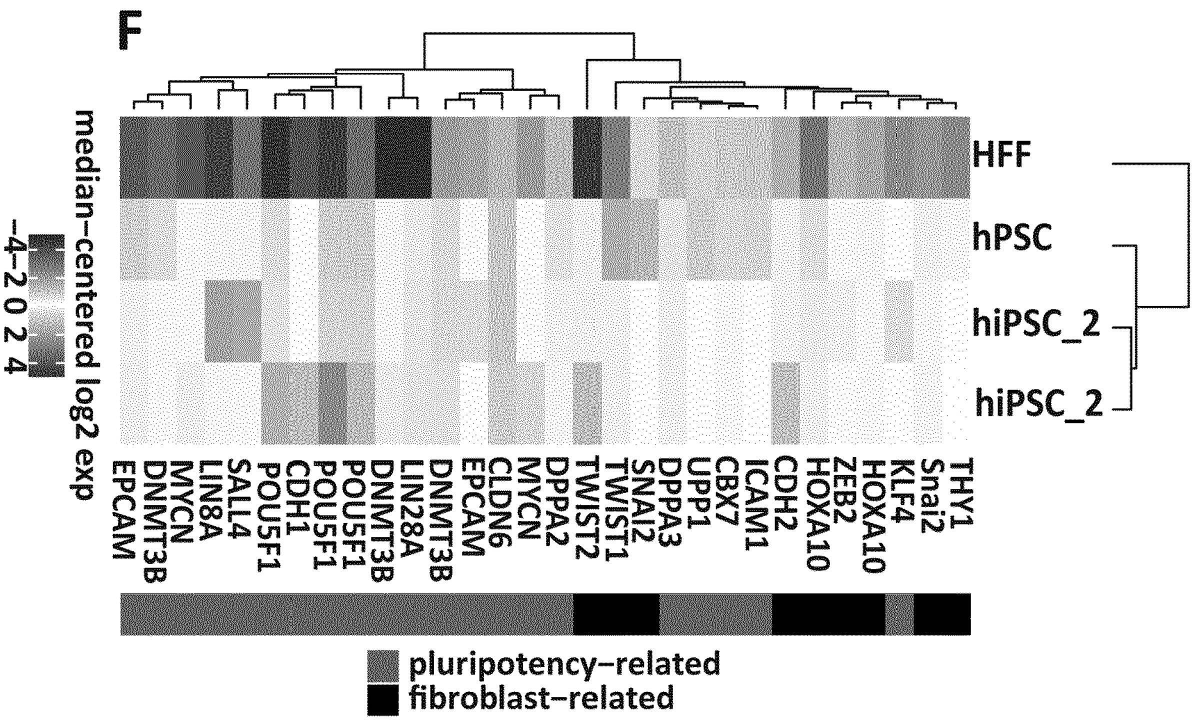
F
median-centered log2 exp
-4 -2 0 2 4
HFF
hPSC
hiPSC_2
hiPSC_2
EPCAM
DNMT3B
MYCN
LIN8A
SALL4
POU5F1
CDH1
POU5F1
POU5F1
DNMT3B
LIN28A
DNMT3B
EPCAM
CLDN6
MYCN
DPPA2
TWIST2
TWIST1
SNAI2
DPPA3
UPP1
CBX7
ICAM1
CDH2
HOXA10
ZEB2
HOXA10
KLF4
Snai2
THY1
pluripotency-related
fibroblast-related

Fig. 5

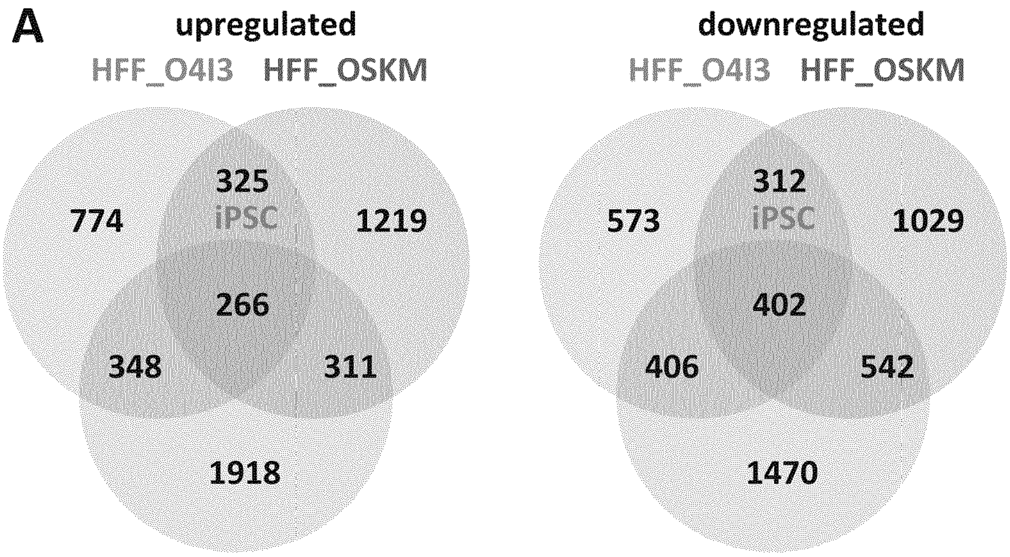
A
upregulated
HFF_O4I3
HFF_OSKM
774
325
iPSC
1219
266
348
311
1918
downregulated
HFF_O4I3
HFF_OSKM
573
312
iPSC
1029
402
406
542
1470

C

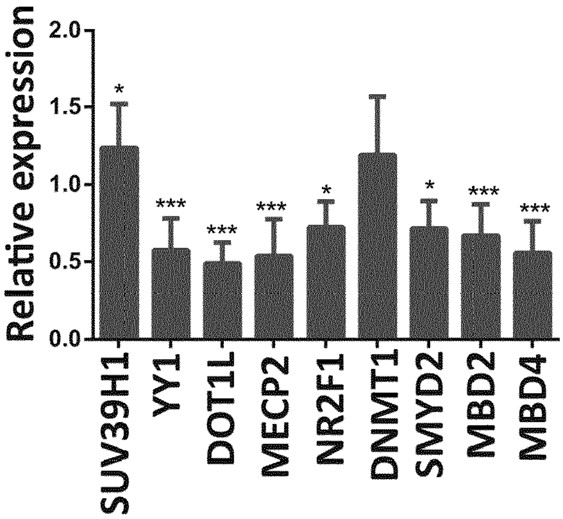
Relative expression
2.0
1.5
1.0
0.5
0.0
*
***
***
***
*
*
***
***
SUV39H1
YY1
DOT1L
MECP2
NR2F1
DNMT1
SMYD2
MBD2
MBD4

D

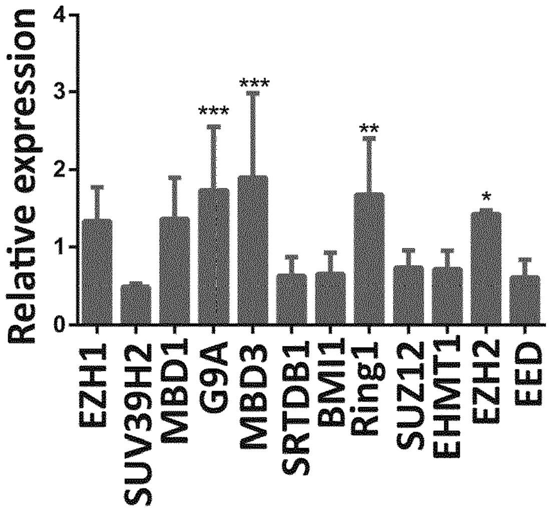
Relative expression
4
3
2
1
0
***
***
**
*
EZH1
SUV39H2
MBD1
G9A
MBD3
SRTDB1
BMI1
Ring1
SUZ12
EHMT1
EZH2
EED

E

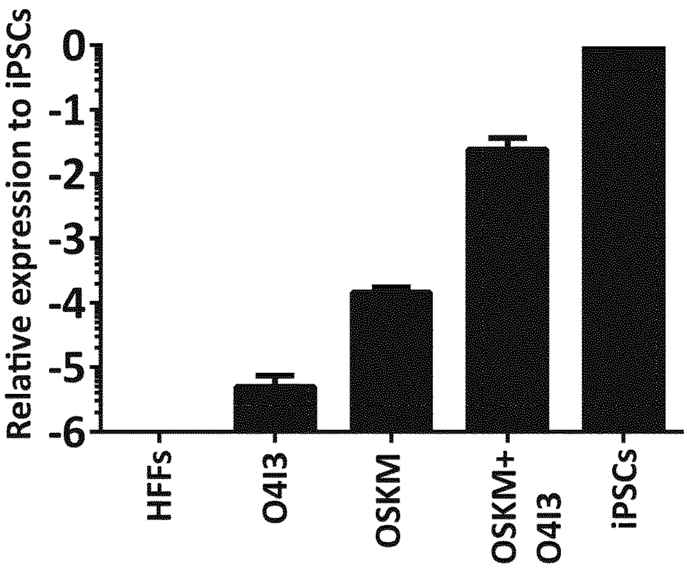
Relative expression to iPSCs
0
-1
-2
-3
-4
-5
-6
HFFs
O4I3
OSKM
OSKM+
O4I3
iPSCs

B

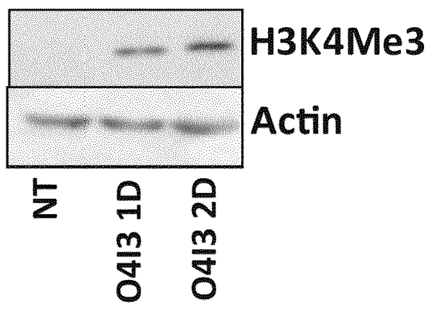
H3K4Me3
Actin
NT
O4I3 1D
O4I3 2D

Fig. 6

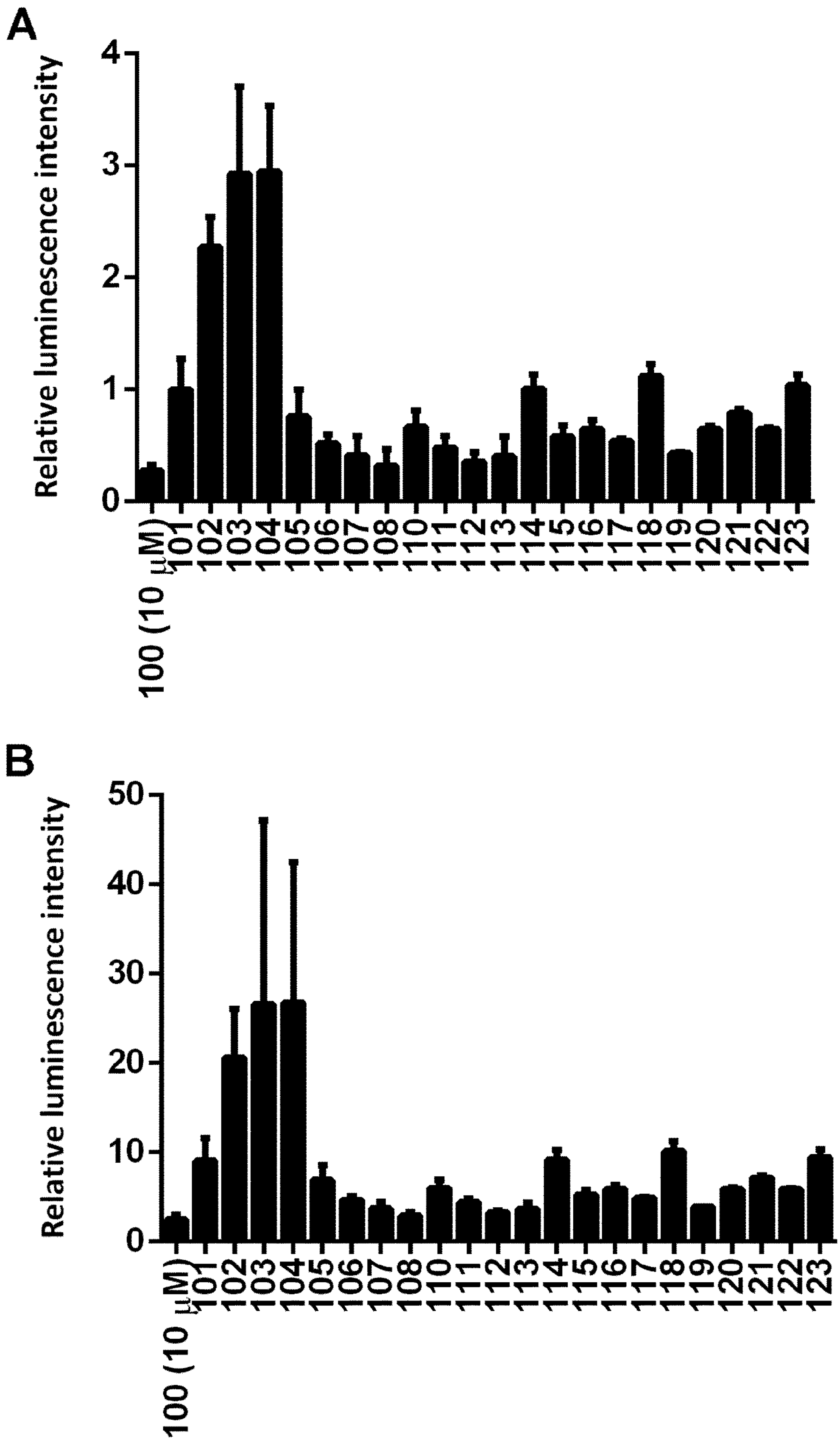
A
Relative luminescence intensity
4
3
2
1
0
100 (10 μM)
101
102
103
104
105
106
107
108
110
111
112
113
114
115
116
117
118
119
120
121
122
123
B
Relative luminescence intensity
50
40
30
20
10
0
100 (10 μM)
101
102
103
104
105
106
107
108
110
111
112
113
114
115
116
117
118
119
120
121
122
123

Fig. 7

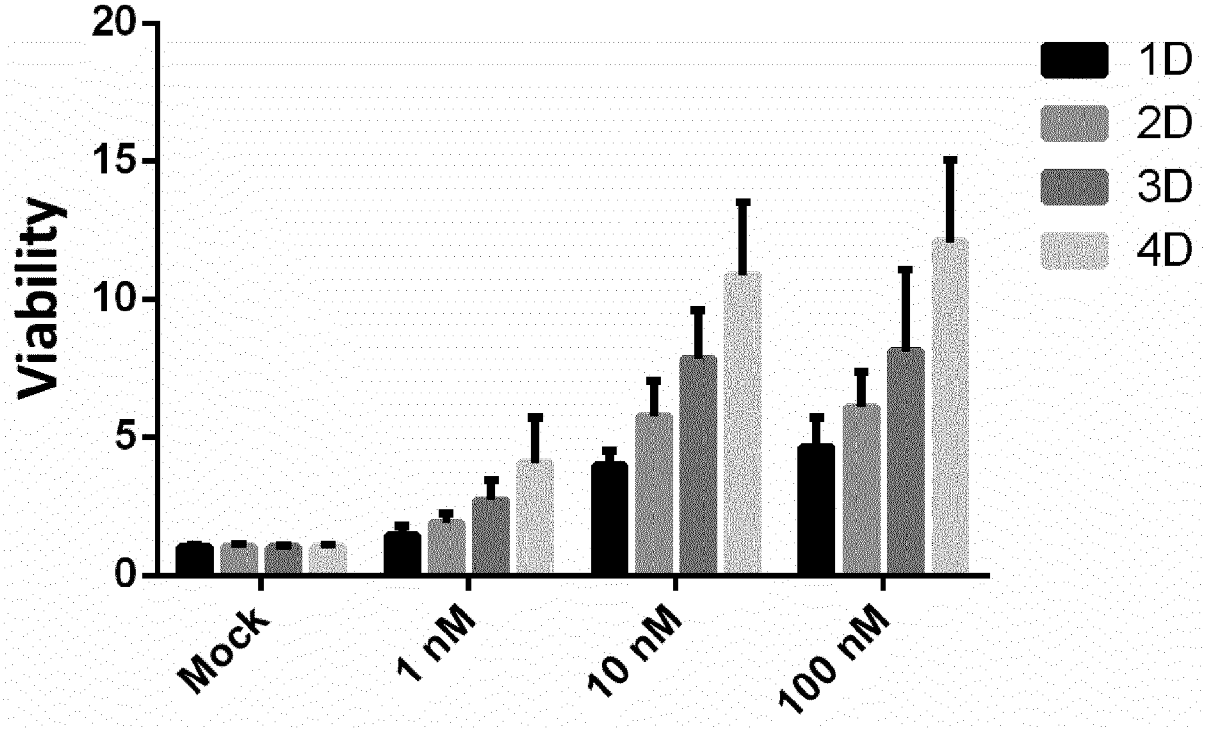
Viability
20
15
10
5
0
1D
2D
3D
4D
Mock
1 nM
10 nM
100 nM

Fig. 8

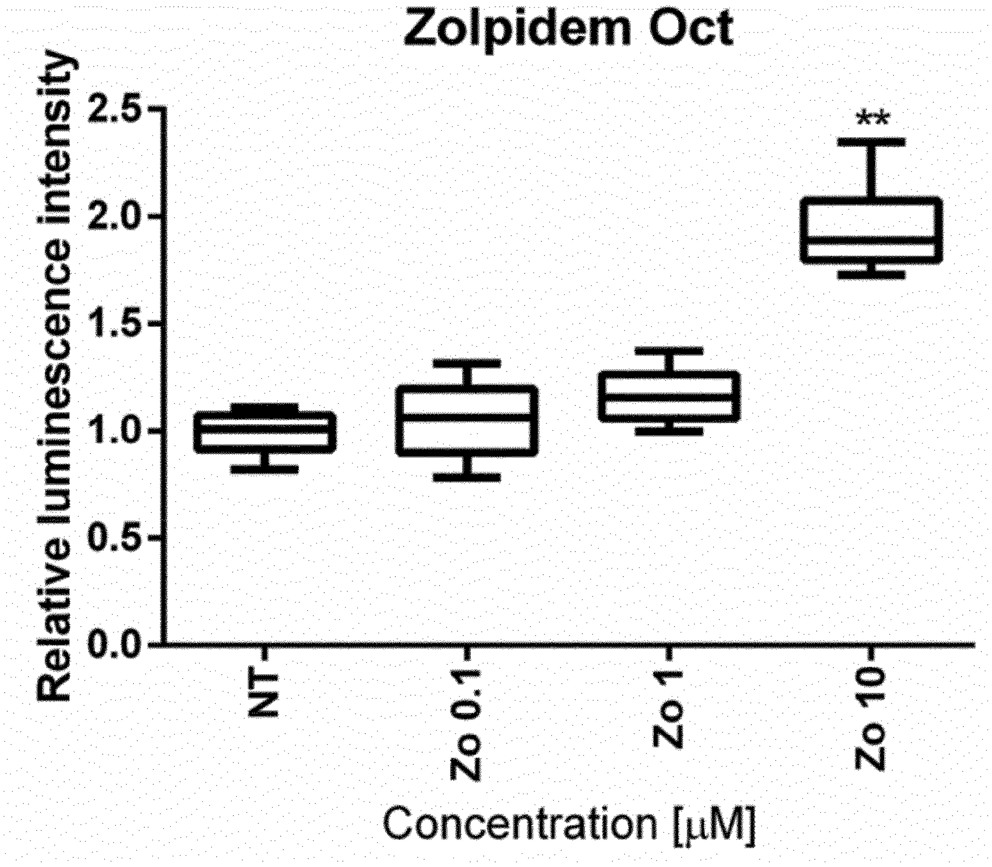
Zolpidem Oct
Relative luminescence intensity
2.5
2.0
1.5
1.0
0.5
0.0
**
NT
Zo 0.1
Zo 1
Zo 10
Concentration [μM]

Fig. 9

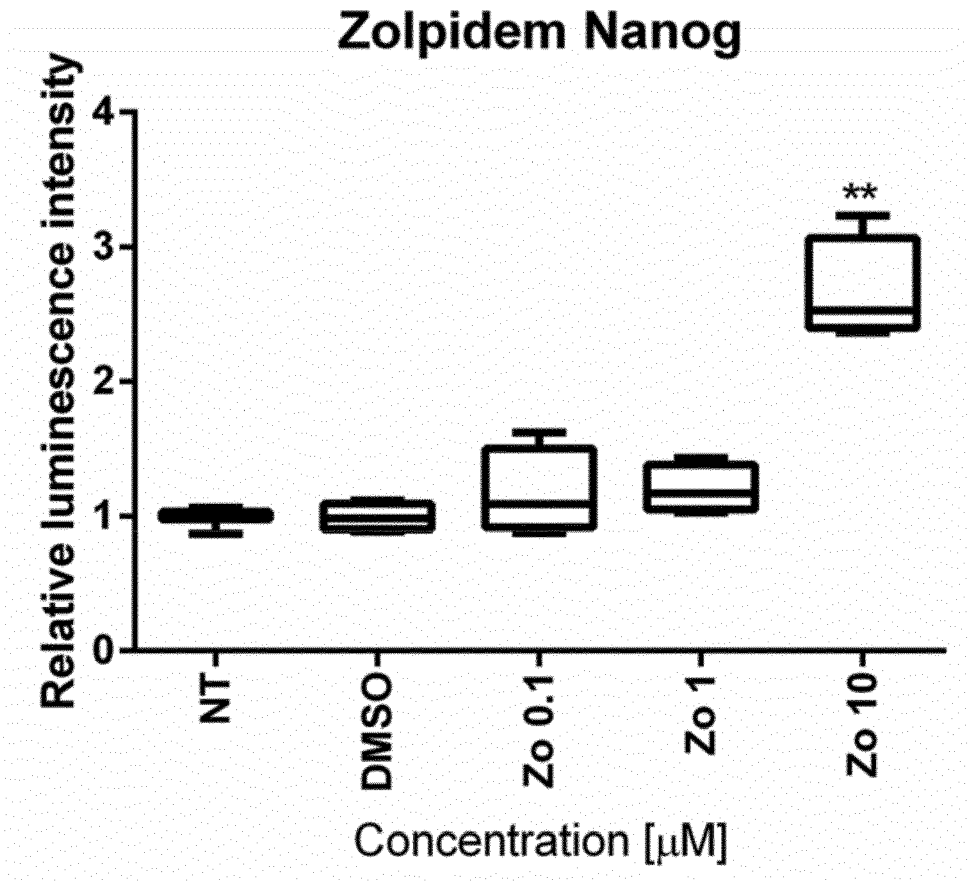
Zolpidem Nanog
Relative luminescence intensity
4
3
2
1
0
**
NT
DMSO
Zo 0.1
Zo 1
Zo 10
Concentration [μM]

Fig 10

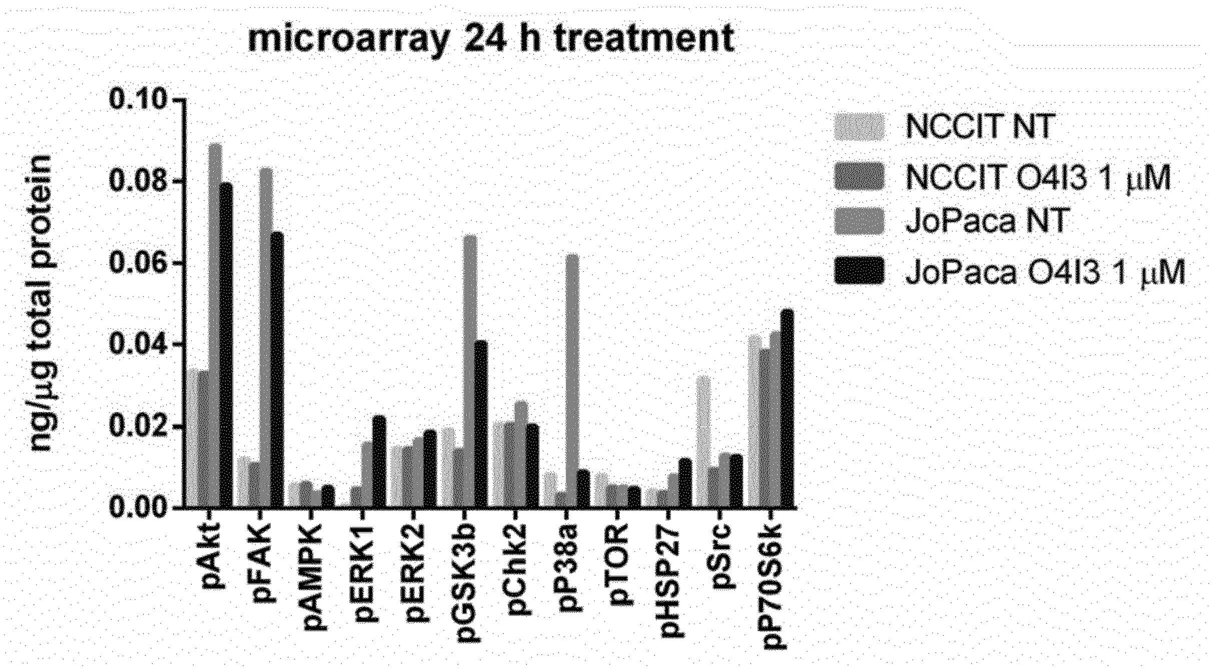
microarray 24 h treatment
ng/μg total protein
0.10
0.08
0.06
0.04
0.02
0.00
NCCIT NT
NCCIT O4I3 1 μM
JoPaca NT
JoPaca O4I3 1 μM
pAkt
pFAK
pAMPK
pERK1
pERK2
pGSK3b
pChk2
pP38a
pTOR
pHSP27
pSrc
pP70S6k

Fig. 11

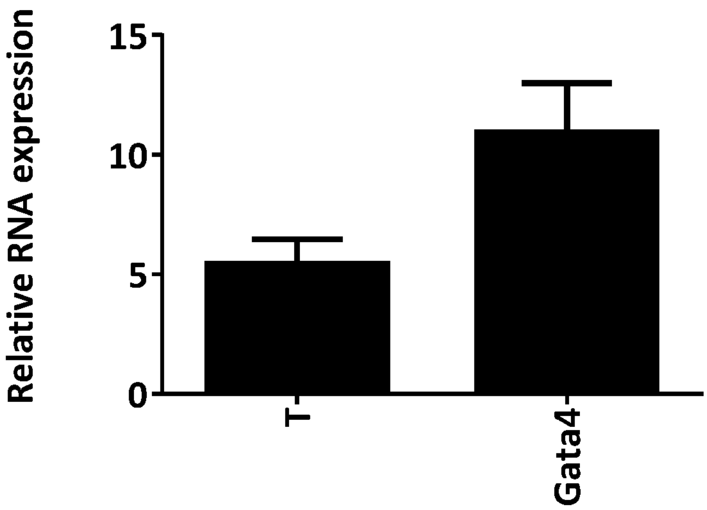
Relative RNA expression
15
10
5
0
T
Gata4

Fig. 12

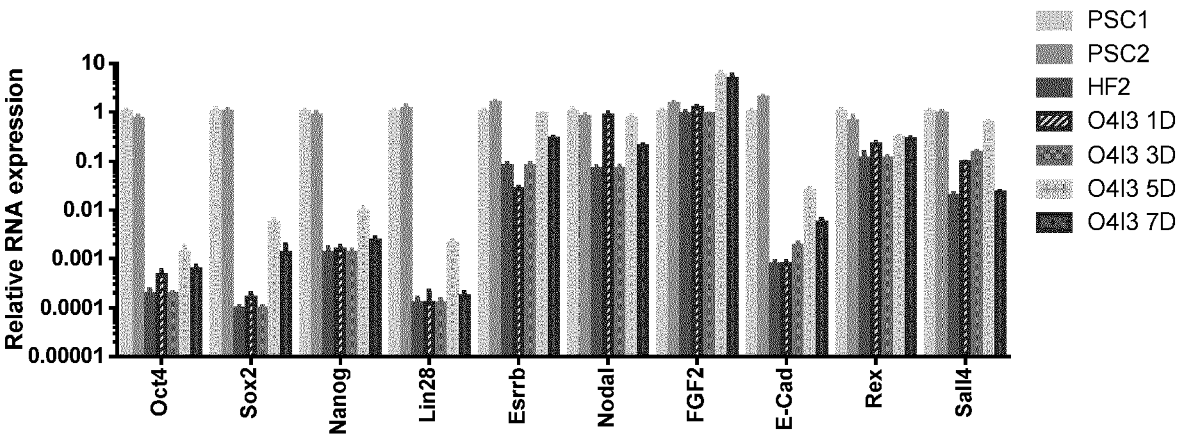
Relative RNA expression
10
1
0.1
0.01
0.001
0.0001
0.00001
Oct4
Sox2
Nanog
Lin28
Esrrb
Nodal
FGF2
E-Cad
Rex
Sall4
PSC1
PSC2
HF2
O4I3 1D
O4I3 3D
O4I3 5D
O4I3 7D

Fig. 13

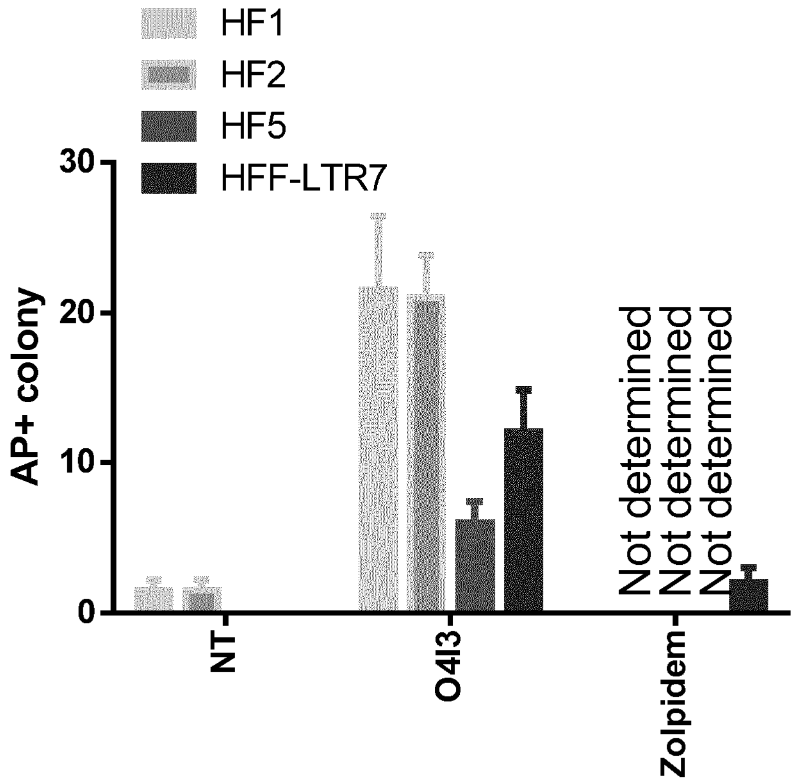
HF1
HF2
HF5
HFF-LTR7
AP+ colony
30
20
10
0
Not determined
Not determined
Not determined
NT
O4I3
Zolpidem

Fig. 14

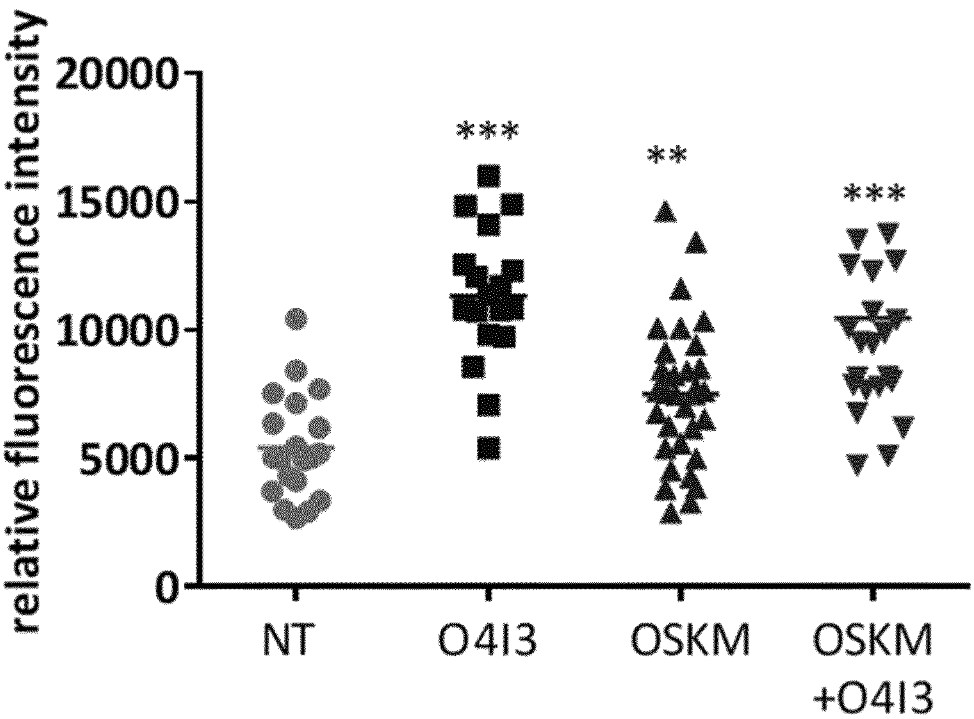
relative fluorescence intensity
20000
15000
10000
5000
0
***
**
***
NT
O4I3
OSKM
OSKM
+O4I3

Fig. 15.

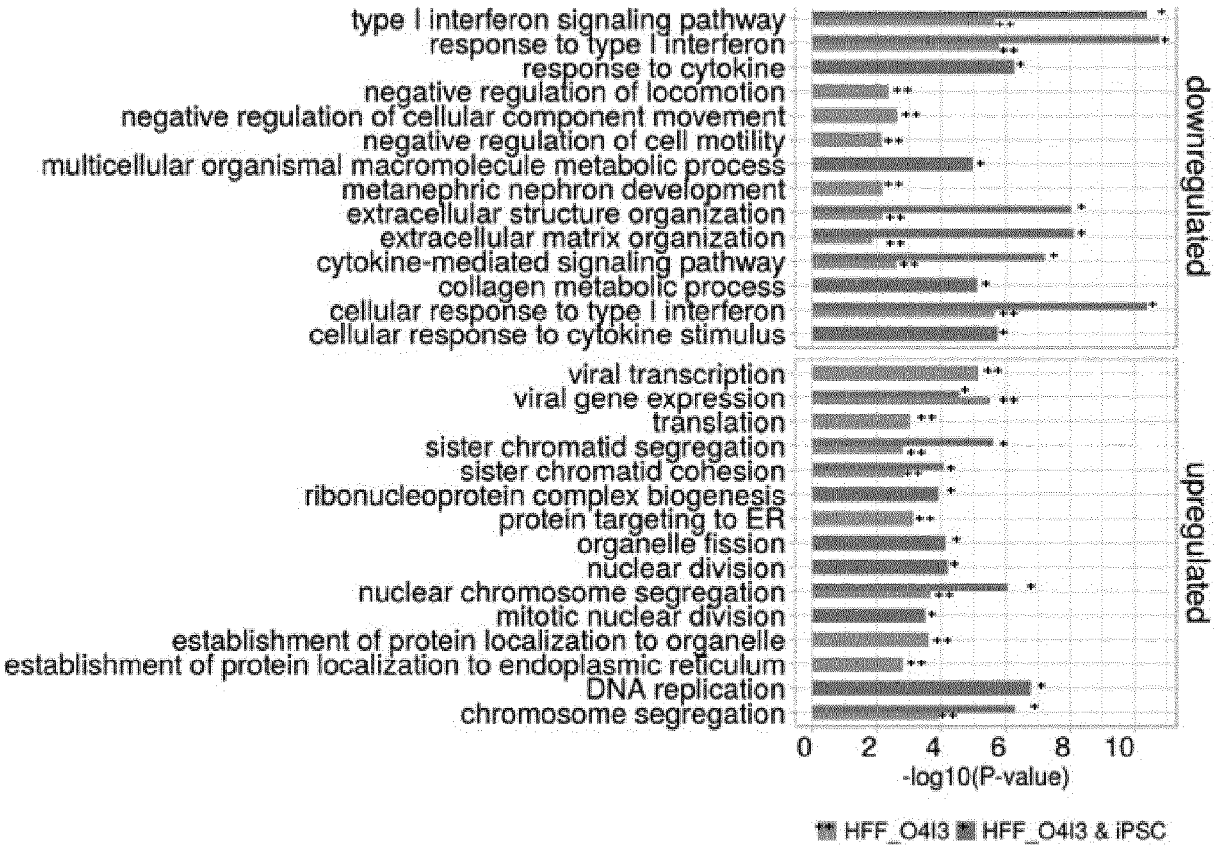
type I interferon signaling pathway
response to type I interferon
response to cytokine
negative regulation of locomotion
negative regulation of cellular component movement
negative regulation of cell motility
multicellular organismal macromolecule metabolic process
metanephric nephron development
extracellular structure organization
extracellular matrix organization
cytokine-mediated signaling pathway
collagen metabolic process
cellular response to type I interferon
cellular response to cytokine stimulus
viral transcription
viral gene expression
translation
sister chromatid segregation
sister chromatid cohesion
ribonucleoprotein complex biogenesis
protein targeting to ER
organelle fission
nuclear division
nuclear chromosome segregation
mitotic nuclear division
establishment of protein localization to organelle
establishment of protein localization to endoplasmic reticulum
DNA replication
chromosome segregation
downregulated
upregulated
0 2 4 6 8 10
-log10(P-value)
HFF_O4I3 HFF_O4I3 & iPSC

Fig. 16

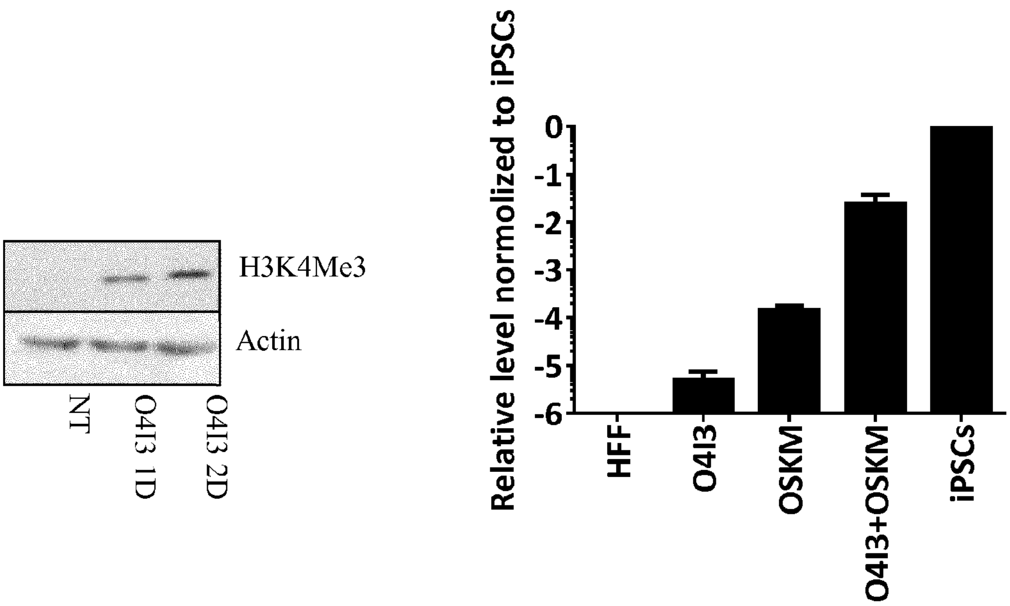
H3K4Me3
Actin
NT
O4I3 1D
O4I3 2D
Relative level normolized to iPSCs
0
-1
-2
-3
-4
-5
-6
HFF
O4I3
OSKM
O4I3+OSKM
iPSCs

Fig. 17

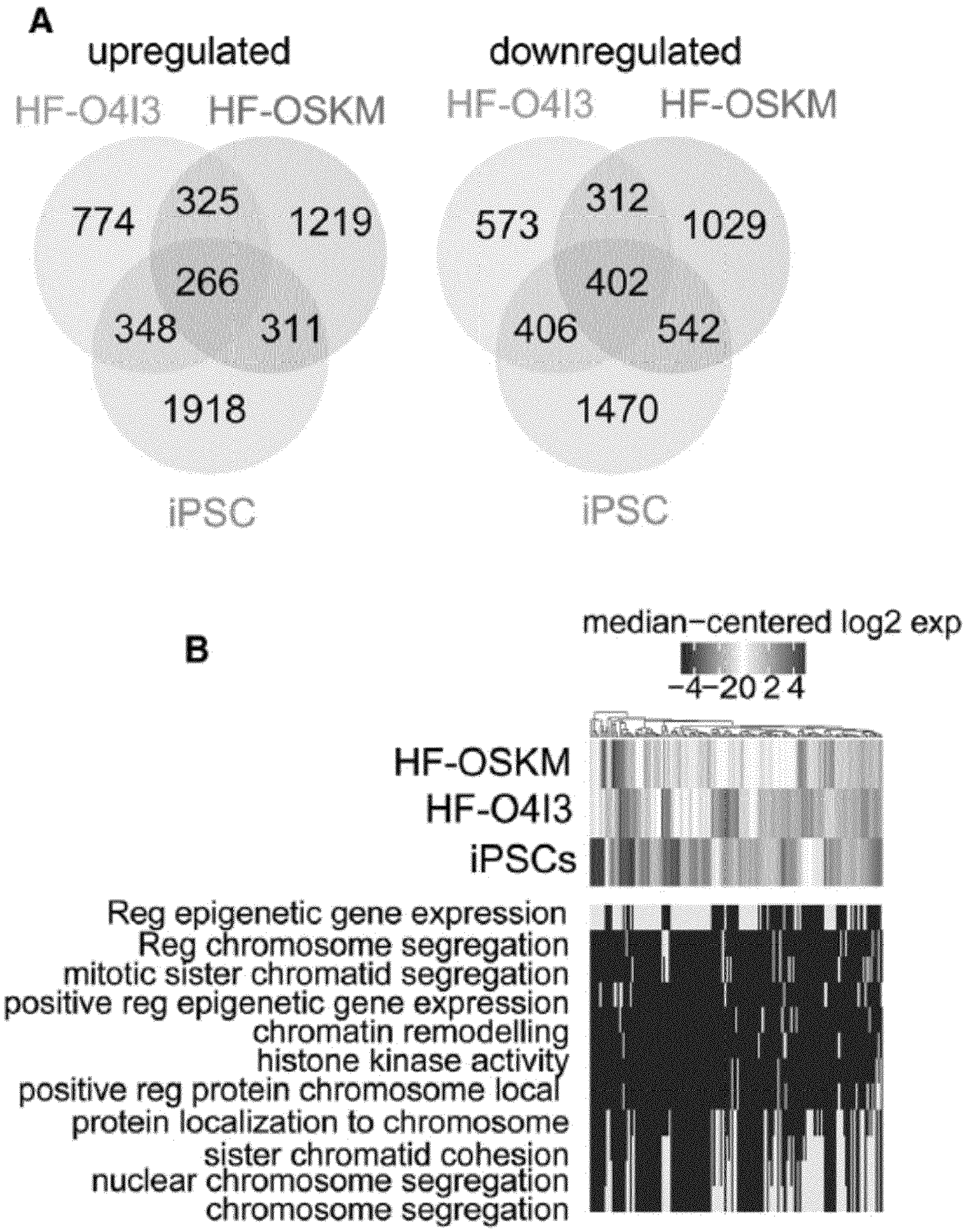
A
upregulated
HF-O4I3
HF-OSKM
774
325
1219
266
348
311
1918
iPSC
downregulated
HF-O4I3
HF-OSKM
573
312
1029
402
406
542
1470
iPSC
B
median−centered log2 exp
−4−2 0 2 4
HF-OSKM
HF-O4I3
iPSCs
Reg epigenetic gene expression
Reg chromosome segregation
mitotic sister chromatid segregation
positive reg epigenetic gene expression
chromatin remodelling
histone kinase activity
positive reg protein chromosome local
protein localization to chromosome
sister chromatid cohesion
nuclear chromosome segregation
chromosome segregation

Fig. 17 (continued)

C

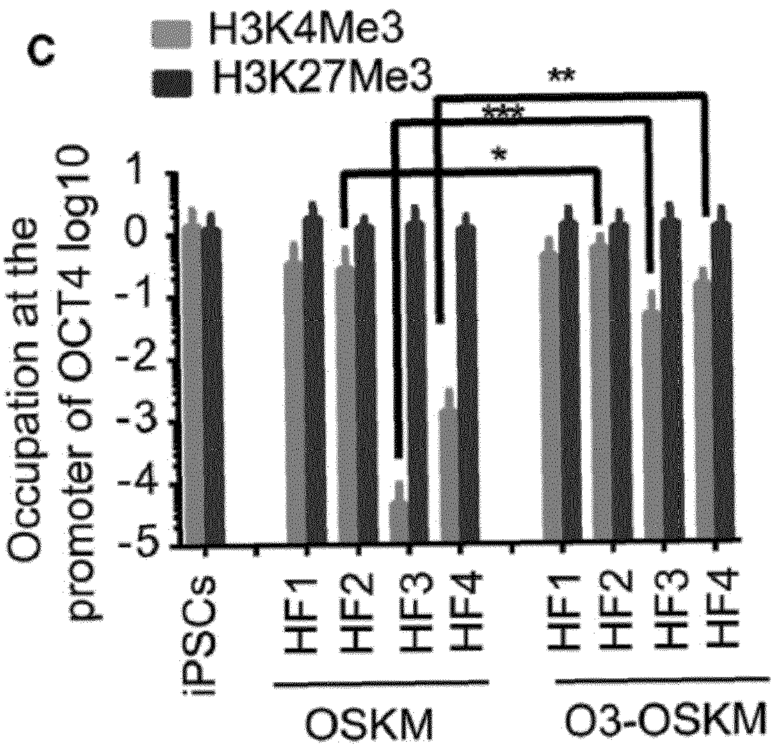


D

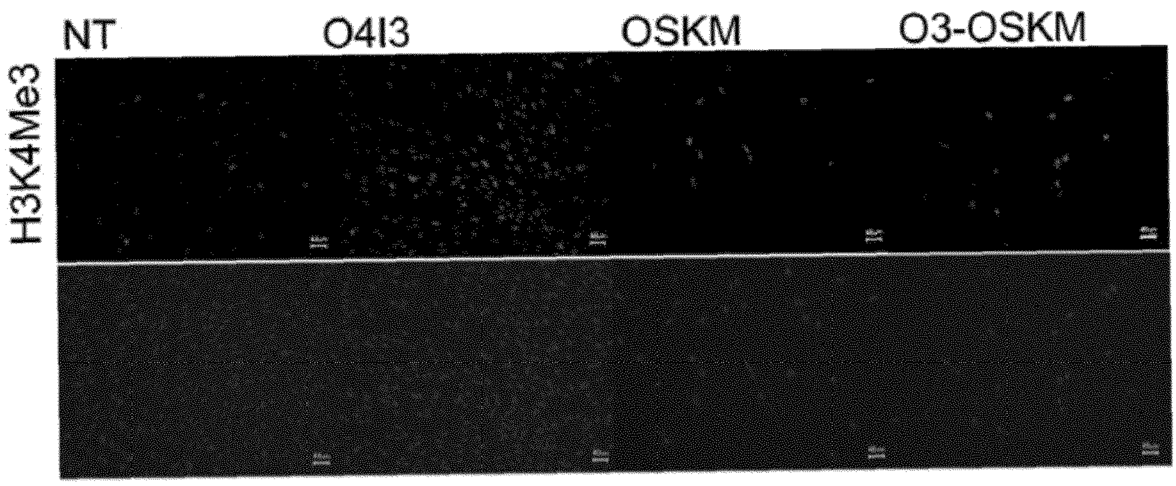

Fig. 17 (continued)

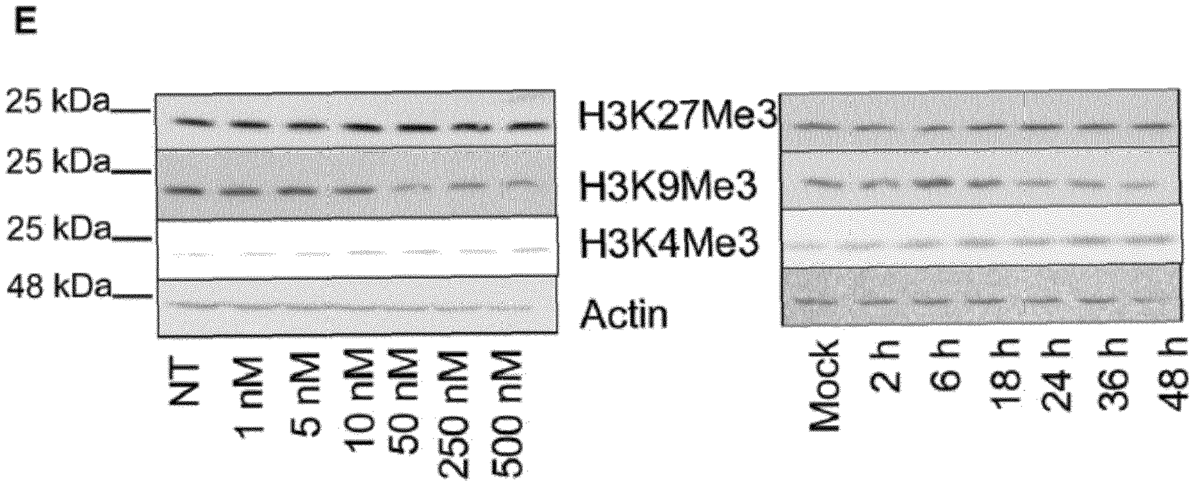


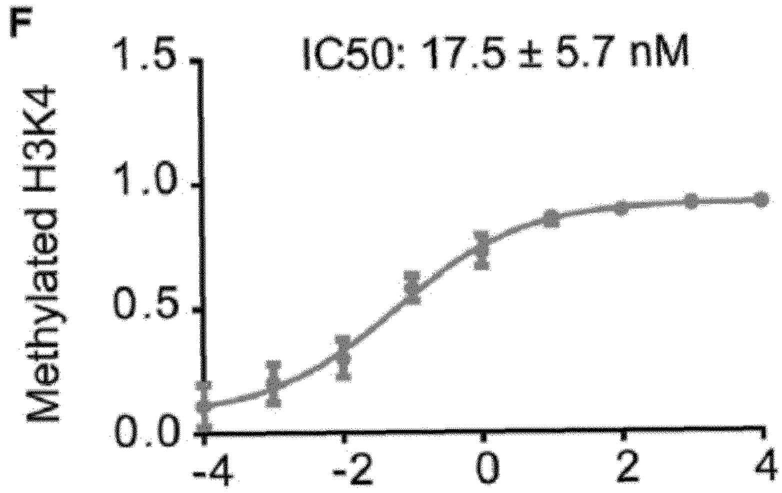

Fig. 18

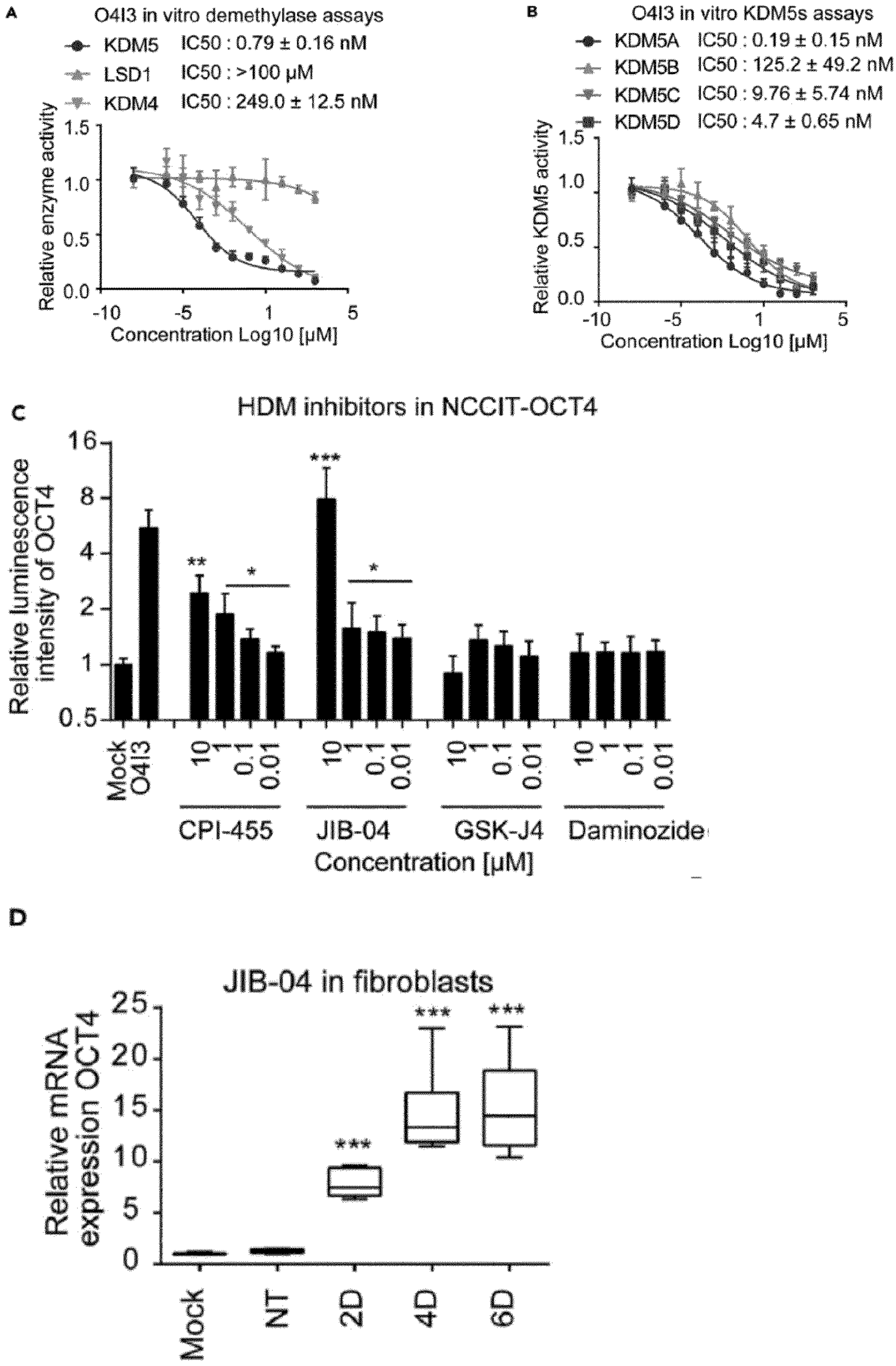
A
O4I3 in vitro demethylase assays
KDM5 IC50 : 0.79 ± 0.16 nM
LSD1 IC50 : >100 µM
KDM4 IC50 : 249.0 ± 12.5 nM
Relative enzyme activity
Concentration Log10 [µM]
B
O4I3 in vitro KDM5s assays
KDM5A IC50 : 0.19 ± 0.15 nM
KDM5B IC50 : 125.2 ± 49.2 nM
KDM5C IC50 : 9.76 ± 5.74 nM
KDM5D IC50 : 4.7 ± 0.65 nM
Relative KDM5 activity
Concentration Log10 [µM]
C
HDM inhibitors in NCCIT-OCT4
Relative luminescence intensity of OCT4
Mock
O4I3
CPI-455
JIB-04
GSK-J4
Daminozide
Concentration [µM]
D
JIB-04 in fibroblasts
Relative mRNA expression OCT4
Mock
NT
2D
4D
6D

Fig. 18 (continued)

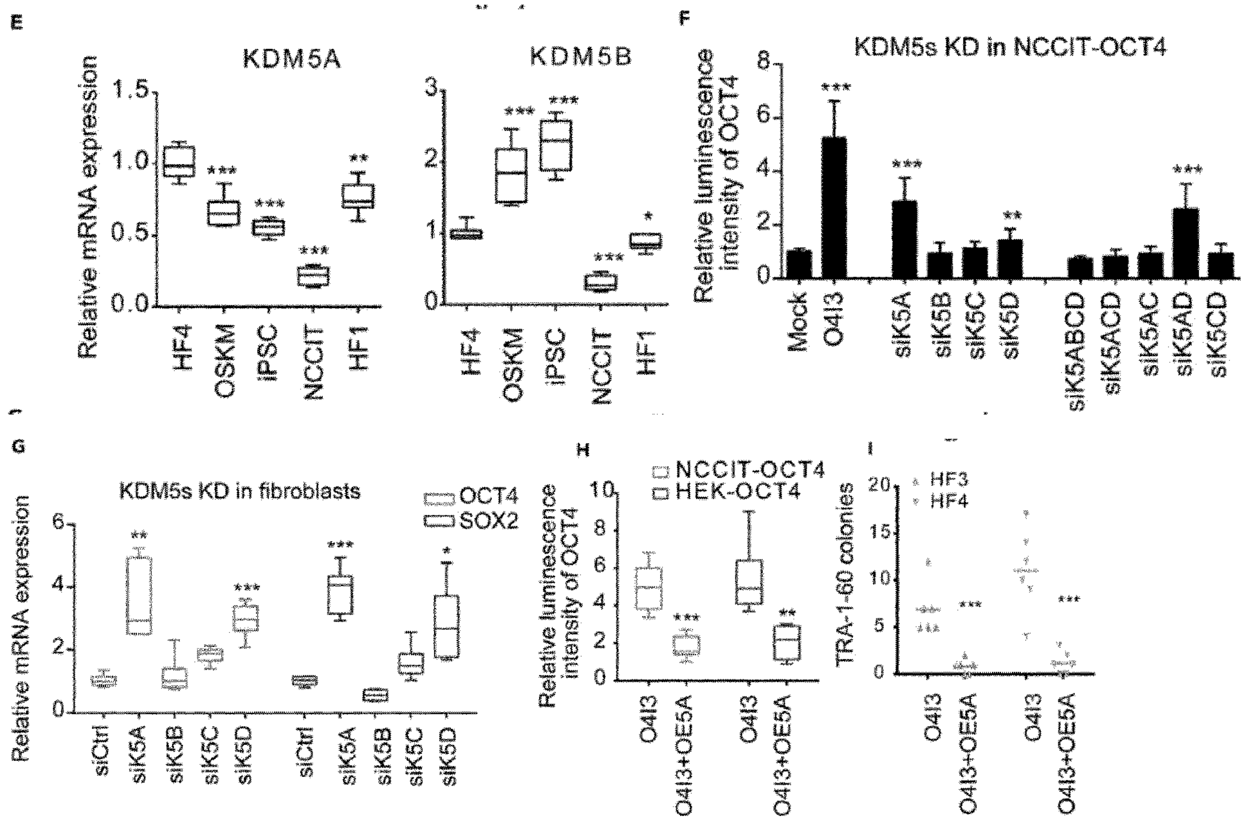

Fig. 19

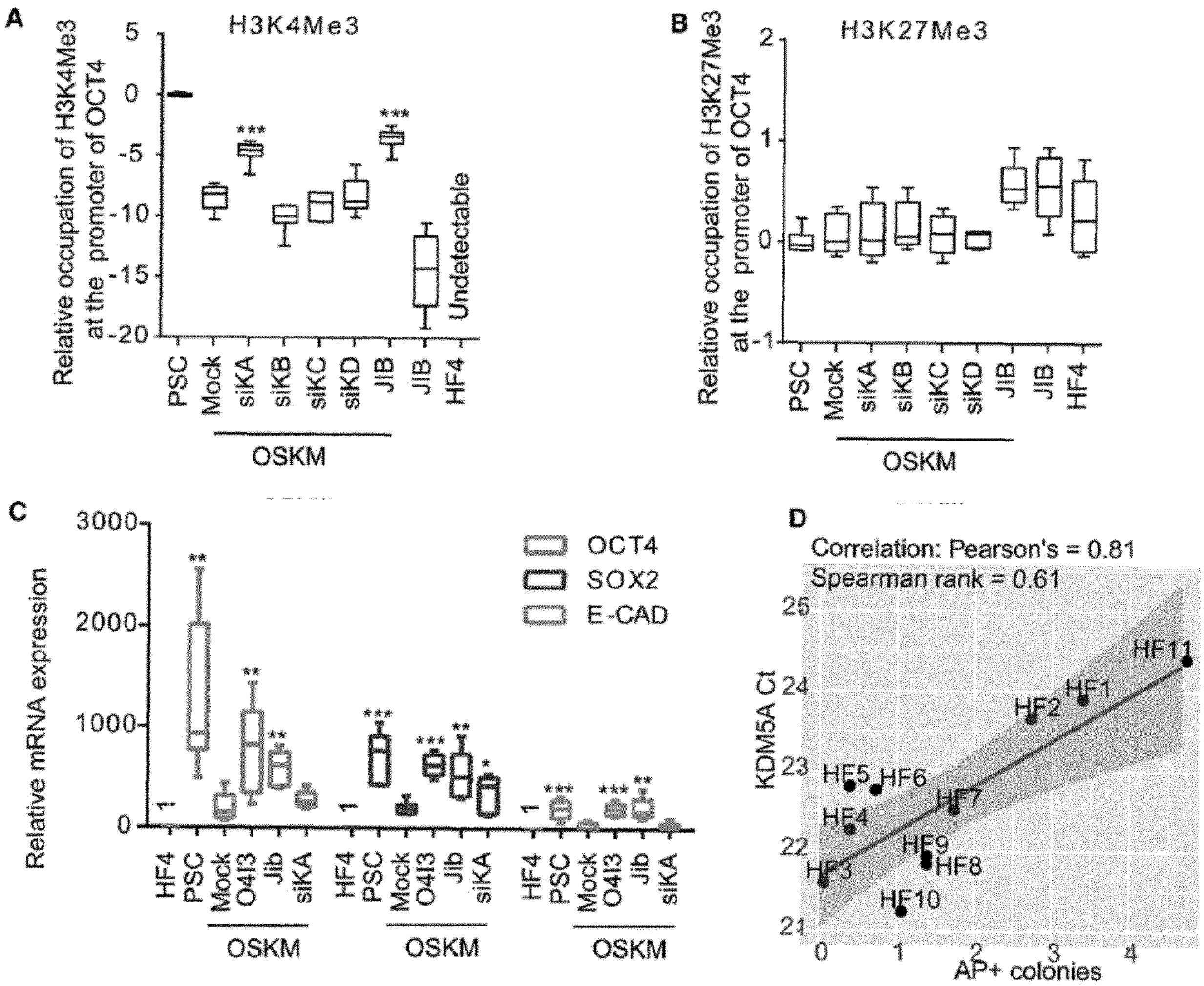
A
H3K4Me3
Relative occupation of H3K4Me3 at the promoter of OCT4
5
0
-5
-10
-15
-20
***
***
Undetectable
PSC
Mock
siKA
siKB
siKC
siKD
JIB
JIB
HF4
OSKM
B
H3K27Me3
Relatiove occupation of H3K27Me3 at the promoter of OCT4
2
1
0
-1
PSC
Mock
siKA
siKB
siKC
siKD
JIB
JIB
HF4
OSKM
C
Relative mRNA expression
3000
2000
1000
0
OCT4
SOX2
E-CAD
HF4
PSC
Mock
O4I3
Jib
siKA
OSKM
D
Correlation: Pearson's = 0.81
Spearman rank = 0.61
KDM5A Ct
AP+ colonies
HF1
HF2
HF3
HF4
HF5
HF6
HF7
HF8
HF9
HF10
HF11

Fig. 19 (continued)

E

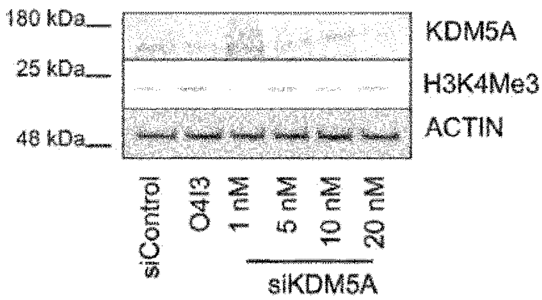


F

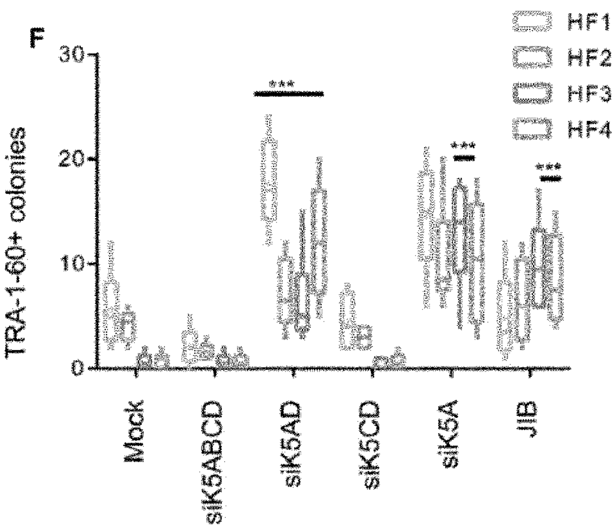

Fig. 20

A

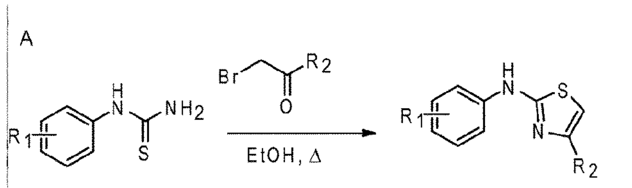


$R_2$=COOEt; 401-404
=$C(CH_3)_3$; 415, 418-423
=Ph; 416
=Naph; 417

B

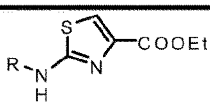

| Nr. | R | Nr. | R |
|---|---|---|---|
| O4I2 | 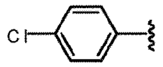 | 409 | 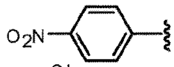 |
| 401 | 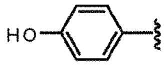 | 410 | 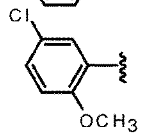 |
| 402 | 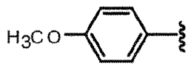 | | |
| 403 | 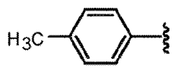 | 411 | 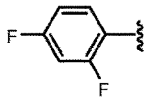 |
| 404 | 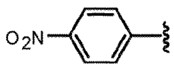 | 412 | 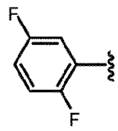 |
| 405 | 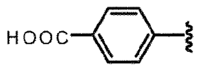 | | |
| 406 | 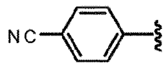 | 413 | 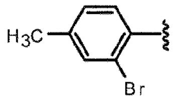 |
| 407 | 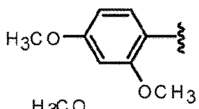 | 414 | 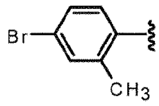 |
| 408 | 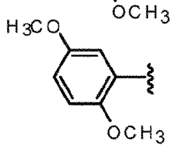 | | |

C

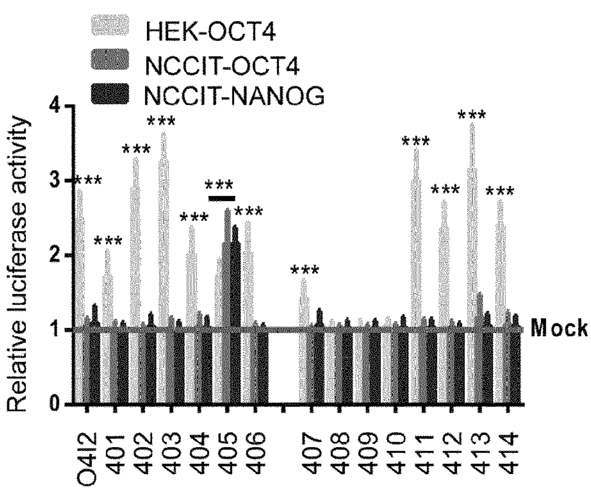

Fig. 20 (continued)

D

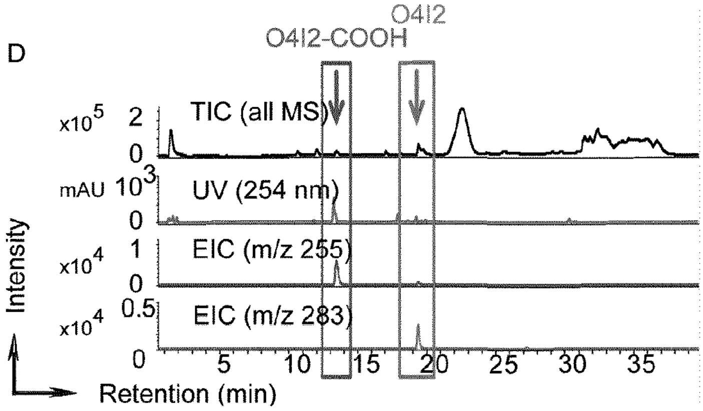


E

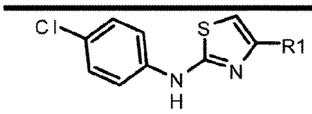 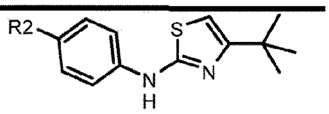

| Nr. | R | Nr. | R |
| --- | --- | --- | --- |
| 415 | 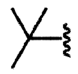 | 418 | 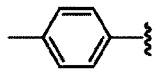 |
| 416 | 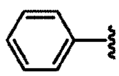 | 419 | 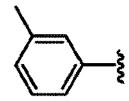 |
| 417 | 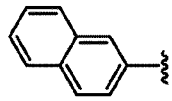 | 420 | 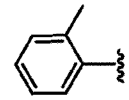 |
| | | 421 | 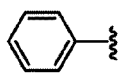 |
| | | 422 | 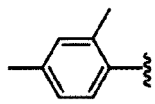 |
| | | 423 | 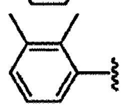 |

F

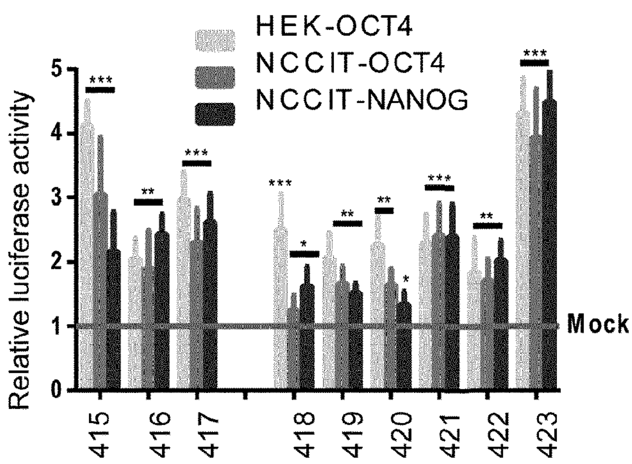

Fig. 21

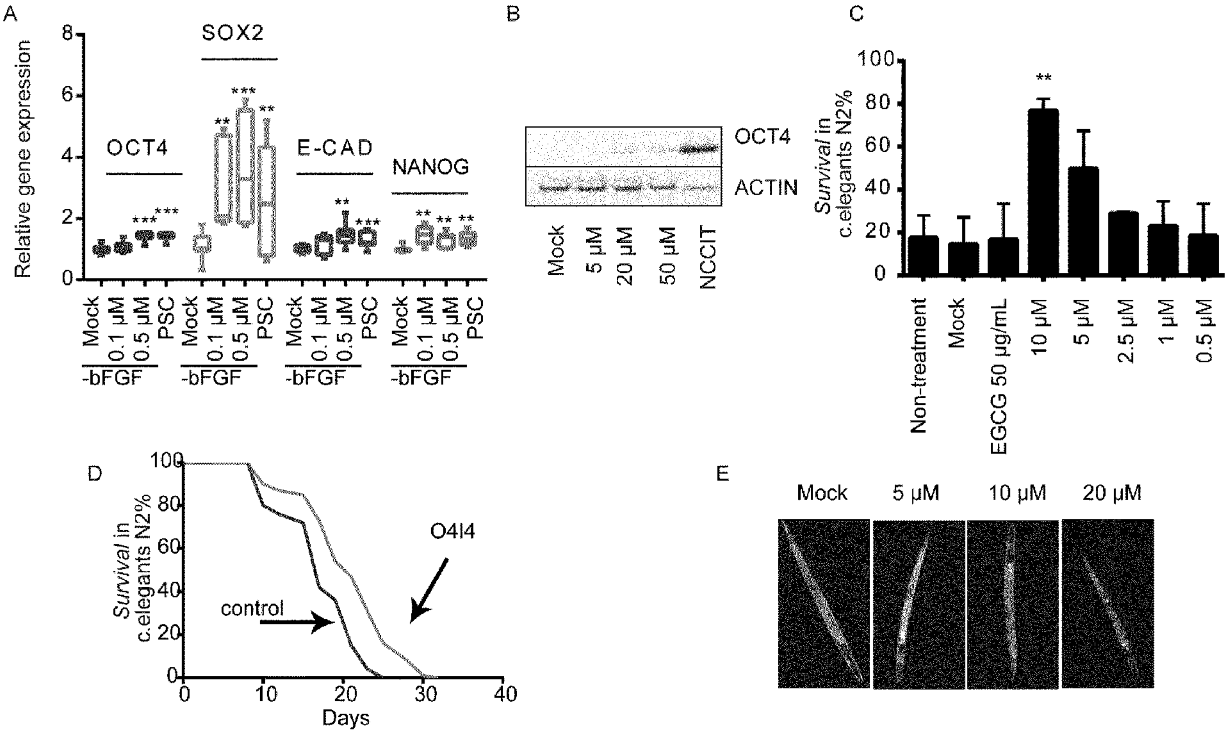
A
Relative gene expression
OCT4
SOX2
E-CAD
NANOG
Mock
0.1 μM
0.5 μM
PSC
-bFGF
B
OCT4
ACTIN
Mock
5 μM
20 μM
50 μM
NCCIT
C
Survival in c.elegants N2%
Non-treatment
Mock
EGCG 50 μg/mL
10 μM
5 μM
2.5 μM
1 μM
0.5 μM
D
Survival in c.elegants N2%
O4I4
control
Days
E
Mock
5 μM
10 μM
20 μM

Fig. 22

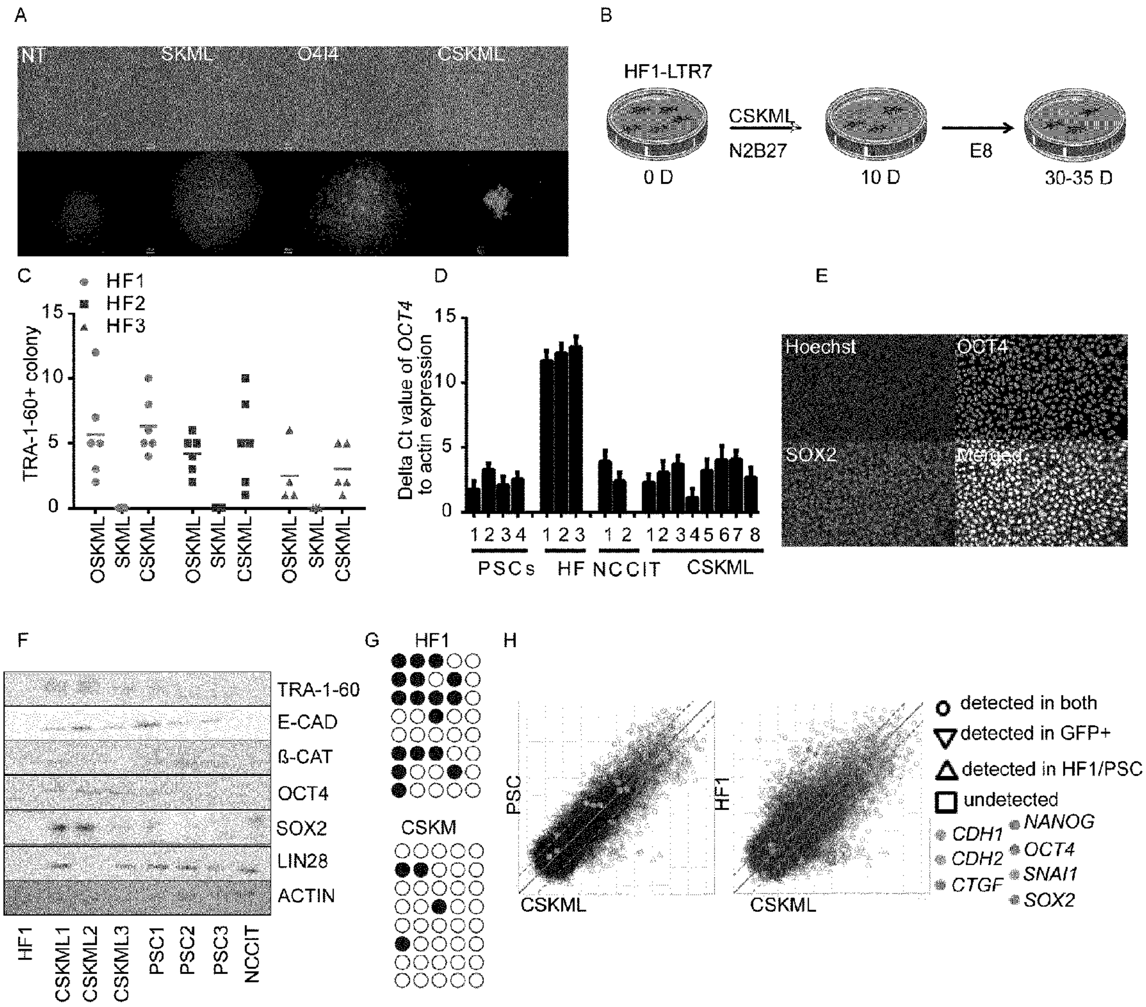
A
NT
SKML
O4I4
CSKML
B
HF1-LTR7
CSKML
N2B27
E8
0 D
10 D
30-35 D
C
HF1
HF2
HF3
TRA-1-60+ colony
OSKML
SKML
CSKML
D
Delta Ct value of OCT4 to actin expression
PSCs
HF
NCCIT
CSKML
E
Hoechst
OCT4
SOX2
Merged
F
TRA-1-60
E-CAD
ß-CAT
OCT4
SOX2
LIN28
ACTIN
HF1
CSKML1
CSKML2
CSKML3
PSC1
PSC2
PSC3
NCCIT
G
HF1
CSKM
H
PSC
HF1
CSKML
detected in both
detected in GFP+
detected in HF1/PSC
undetected
CDH1
CDH2
CTGF
NANOG
OCT4
SNAI1
SOX2

Fig. 23

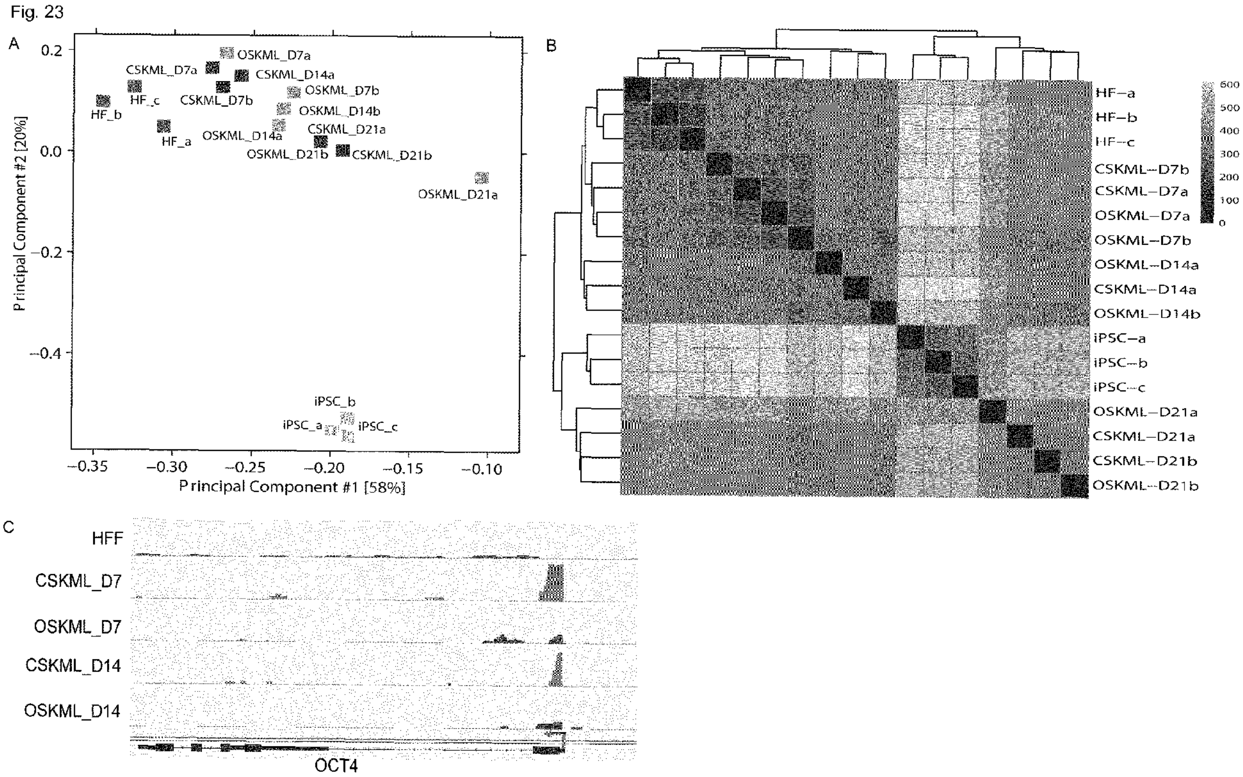
A
Principal Component #2 [20%]
Principal Component #1 [58%]
OSKML_D7a
CSKML_D7a
CSKML_D14a
OSKML_D7b
HF_c
CSKML_D7b
HF_b
OSKML_D14b
CSKML_D21a
OSKML_D14a
HF_a
OSKML_D21b
CSKML_D21b
OSKML_D21a
iPSC_b
iPSC_a
iPSC_c
B
HF-a
HF-b
HF-c
CSKML-D7b
CSKML-D7a
OSKML-D7a
OSKML-D7b
OSKML-D14a
CSKML-D14a
OSKML-D14b
iPSC-a
iPSC-b
iPSC-c
OSKML-D21a
CSKML-D21a
CSKML-D21b
OSKML-D21b
C
HFF
CSKML_D7
OSKML_D7
CSKML_D14
OSKML_D14
OCT4

Fig. 24

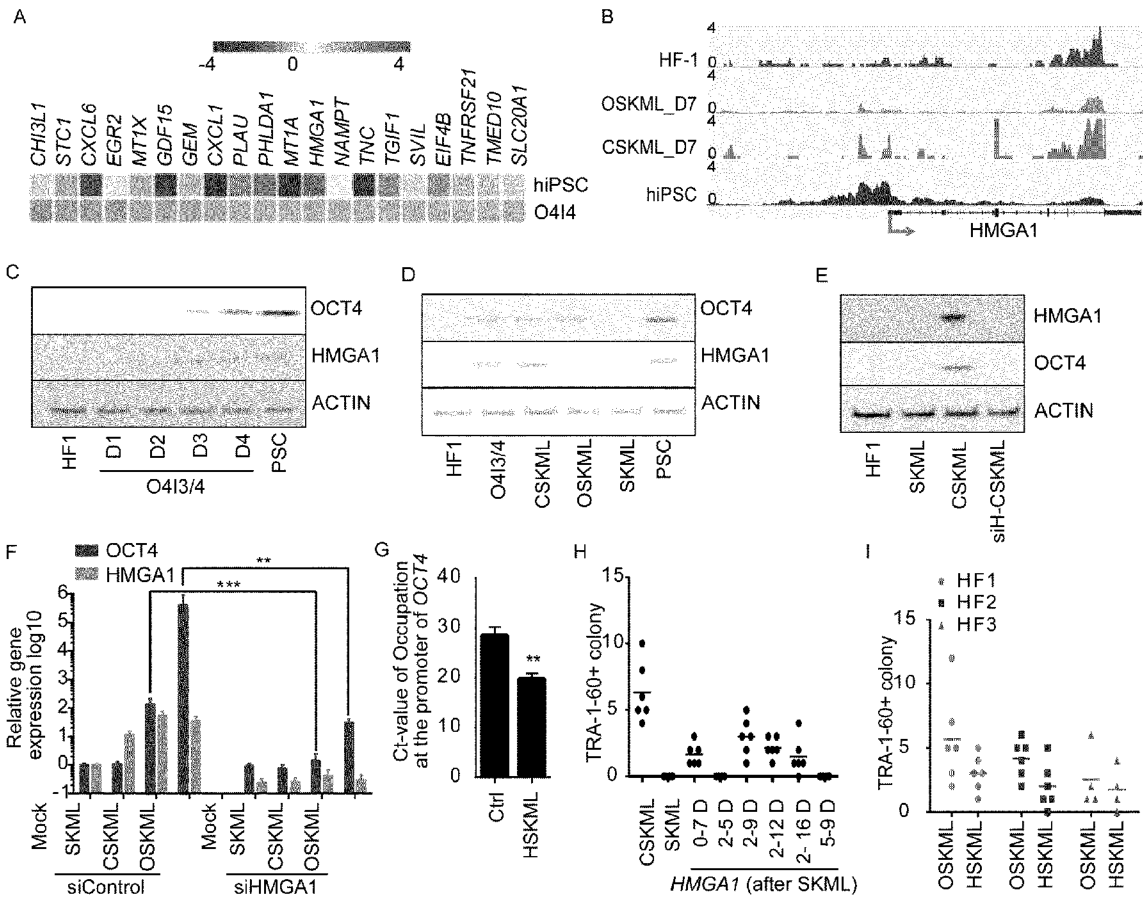
A
-4
0
4
CHI3L1
STC1
CXCL6
EGR2
MT1X
GDF15
GEM
CXCL1
PLAU
PHLDA1
MT1A
HMGA1
NAMPT
TNC
TGIF1
SVIL
EIF4B
TNFRSF21
TMED10
SLC20A1
hiPSC
O4I4
B
HF-1
OSKML_D7
CSKML_D7
hiPSC
HMGA1
C
OCT4
HMGA1
ACTIN
HF1
D1
D2
D3
D4
PSC
O4I3/4
D
OCT4
HMGA1
ACTIN
HF1
O4I3/4
CSKML
OSKML
SKML
PSC
E
HMGA1
OCT4
ACTIN
HF1
SKML
CSKML
siH-CSKML
F
OCT4
HMGA1
Relative gene expression log10
Mock
SKML
CSKML
OSKML
Mock
SKML
CSKML
OSKML
siControl
siHMGA1
G
Ct-value of Occupation at the promoter of OCT4
Ctrl
HSKML
H
TRA-1-60+ colony
CSKML
SKML
0-7 D
2-5 D
2-9 D
2-12 D
2- 16 D
5-9 D
HMGA1 (after SKML)
I
HF1
HF2
HF3
TRA-1-60+ colony
OSKML
HSKML
OSKML
HSKML
OSKML
HSKML

Fig. 25

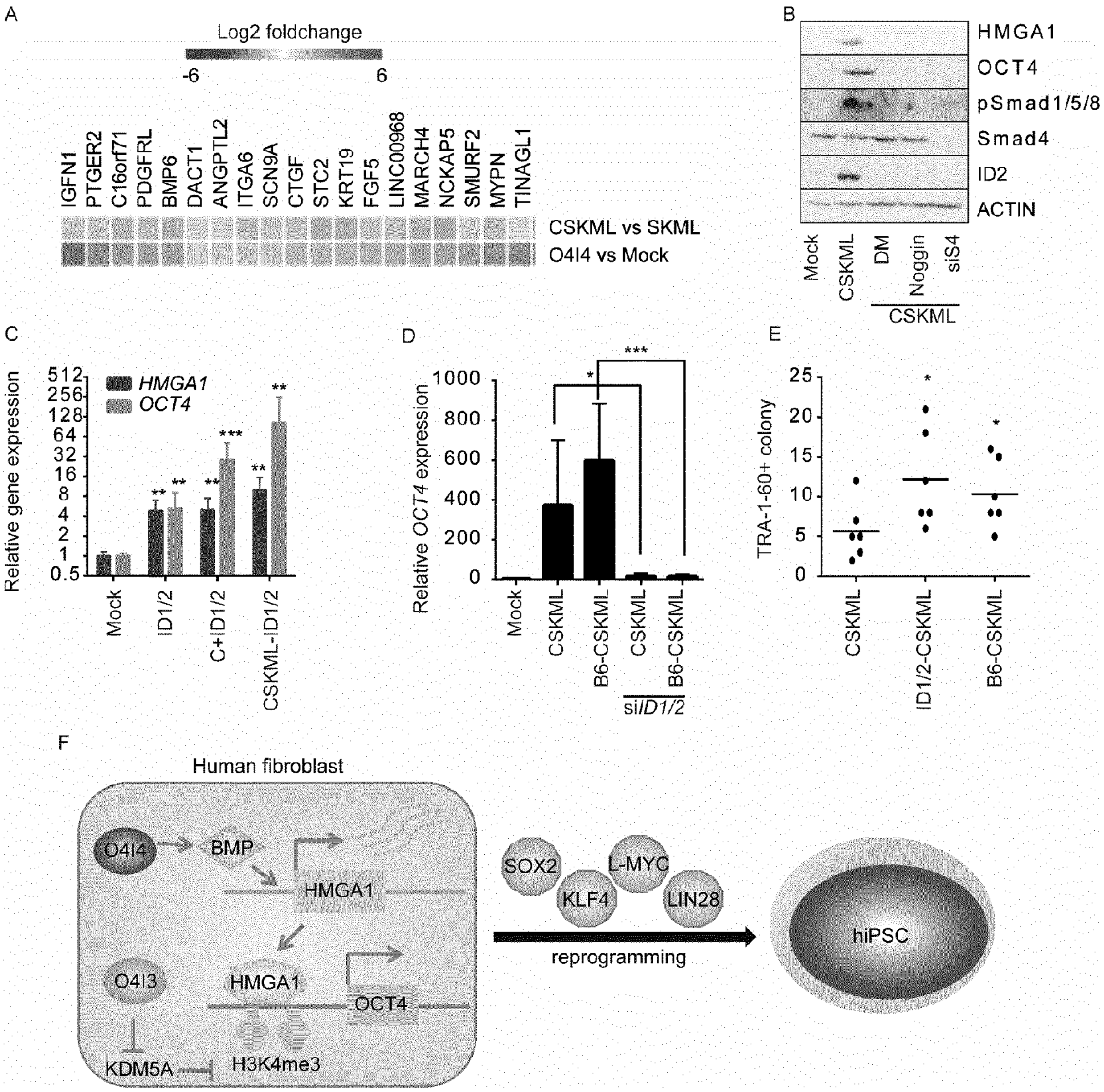
A
Log2 foldchange
-6
6
IGFN1
PTGER2
C16orf71
PDGFRL
BMP6
DACT1
ANGPTL2
ITGA6
SCN9A
CTGF
STC2
KRT19
FGF5
LINC00968
MARCH4
NCKAP5
SMURF2
MYPN
TINAGL1
CSKML vs SKML
O4I4 vs Mock
B
HMGA1
OCT4
pSmad1/5/8
Smad4
ID2
ACTIN
Mock
CSKML
DM
Noggin
siS4
CSKML
C
Relative gene expression
HMGA1
OCT4
Mock
ID1/2
C+ID1/2
CSKML-ID1/2
D
Relative OCT4 expression
Mock
CSKML
B6-CSKML
CSKML
B6-CSKML
siID1/2
E
TRA-1-60+ colony
CSKML
ID1/2-CSKML
B6-CSKML
F
Human fibroblast
O4I4
BMP
HMGA1
O4I3
HMGA1
OCT4
KDM5A
H3K4me3
SOX2
L-MYC
KLF4
LIN28
reprogramming
hiPSC

Europäisches Patentamt
European Patent Office
Office européen des brevets

# EUROPEAN SEARCH REPORT

Application Number
EP 19 19 1147

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LISA L. VON MOLTKE ET AL: "Effect of zolpidem on human Cytochrome P450 activity, and on transport mediated by P-glycoprotein", BIOPHARMACEUTICS AND DRUG DISPOSITION., vol. 23, no. 9, 1 December 2002 (2002-12-01), pages 361-367, XP55652468, US ISSN: 0142-2782, DOI: 10.1002/bdd.329 * page 363, left-hand column, last paragraph - right-hand column, last paragraph * ----- | 1-5, 12-15 | INV. C12N5/074 |
| X | DARREN M. BREY ET AL: "High-throughput screening of a small molecule library for promoters and inhibitors of mesenchymal stem cell osteogenic differentiation", BIOTECHNOLOGY AND BIOENGINEERING, vol. 108, no. 1, 26 January 2011 (2011-01-26), pages 163-174, XP55242559, ISSN: 0006-3592, DOI: 10.1002/bit.22925 * table II * * page 164, right-hand column, last paragraph - page 166, left-hand column, paragraph 2 * ----- | 13-15 | TECHNICAL FIELDS SEARCHED (IPC) C12N |
| A | EP 1 038 875 A2 (SYNTHON BV [NL]) 27 September 2000 (2000-09-27) * paragraph [0040] * ----- -/-- | 1-5, 12-15 | |

The present search report has been drawn up for all claims

1

| Place of search | Date of completion of the search | Examiner |
|---|---|---|
| Munich | 12 December 2019 | Petri, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 19 19 1147

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | QIN HUA ET AL: "Small molecules for reprogramming and transdifferentiation", CMLS CELLULAR AND MOLECULAR LIFE SCIENCES, BIRKHAUSER VERLAG, HEIDELBERG, DE, vol. 74, no. 19, 11 July 2017 (2017-07-11), pages 3553-3575, XP036314164, ISSN: 1420-682X, DOI: 10.1007/S00018-017-2586-X [retrieved on 2017-07-11] * page 3567, left-hand column, paragraph 1; table 2 * ----- | 1-15 | |
| A | MIN XIE ET AL: "Pharmacological Reprogramming of Somatic Cells for Regenerative Medicine", ACCOUNTS OF CHEMICAL RESEARCH., vol. 50, no. 5, 16 May 2017 (2017-05-16), pages 1202-1211, XP55652043, US ISSN: 0001-4842, DOI: 10.1021/acs.accounts.7b00020 * the whole document * ----- | 1-15 | TECHNICAL FIELDS SEARCHED (IPC) |
| A | SUSANNE MARIJE KOOISTRA ET AL: "Molecular mechanisms and potential functions of histone demethylases", NAT REV MOL CELL BIOL., vol. 13, no. 5, 1 May 2012 (2012-05-01), pages 297-311, XP55652045, London ISSN: 1471-0072, DOI: 10.1038/nrm3327 * table 1 * ----- -/-- | 1-15 | |

1

The present search report has been drawn up for all claims

| Place of search | Date of completion of the search | Examiner |
|---|---|---|
| Munich | 12 December 2019 | Petri, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 4

Europäisches Patentamt
European Patent Office
Office européen des brevets

# EUROPEAN SEARCH REPORT

Application Number
EP 19 19 1147

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JIEKAI CHEN ET AL: "H3K9 methylation is a barrier during somatic cell reprogramming into iPSCs", NATURE GENETICS., vol. 45, no. 1, 1 January 2013 (2013-01-01), pages 34-42, XP55652039, NEW YORK, US ISSN: 1061-4036, DOI: 10.1038/ng.2491 * abstract * ----- | 1-15 | |
| X,P | YASAMIN DABIRI ET AL: "Imidazopyridines as Potent KDM5 Demethylase Inhibitors Promoting Reprogramming Efficiency of Human iPSCs", ISCIENCE, vol. 12, 1 February 2019 (2019-02-01), pages 168-181, XP55650351, ISSN: 2589-0042, DOI: 10.1016/j.isci.2019.01.012 * page 172, paragraph 2-3 * * table S1 * ----- | 1-5, 10-15 | TECHNICAL FIELDS SEARCHED (IPC) |
| X | WO 02/30358 A2 (TULARIK INC [US]; CHEMOCENTRYX INC [US] ET AL.) 18 April 2002 (2002-04-18) * claim 64; example 21; table 1; compound 2 * ----- -/-- | 6-15 | |

1

The present search report has been drawn up for all claims

| Place of search | Date of completion of the search | Examiner |
|---|---|---|
| Munich | 12 December 2019 | Petri, Bernhard |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 3 of 4

Europäisches Patentamt
European Patent Office
Office européen des brevets

# EUROPEAN SEARCH REPORT

Application Number
EP 19 19 1147

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANG XUEMEI ET AL: "Optimization of 2-aminothiazole derivatives as CCR4 antagonists", BIOORGANIC & MEDICINAL CHEMISTRY LETTERS, vol. 16, no. 10, 23 February 2006 (2006-02-23), pages 2800-2803, XP028674513, ISSN: 0960-894X, DOI: 10.1016/J.BMCL.2006.01.126 * page 2803, right-hand column, paragraph 8; table 2; compound 19 * ----- | 6-15 | |
| X | XINLAI CHENG ET AL: "Ethyl 2-((4-Chlorophenyl)amino)thiazole-4-carboxylate and Derivatives Are Potent Inducers of Oct3/4", JOURNAL OF MEDICINAL CHEMISTRY, vol. 58, no. 15, 13 August 2015 (2015-08-13), pages 5742-5750, XP55650400, US ISSN: 0022-2623, DOI: 10.1021/acs.jmedchem.5b00226 * figures 1-3 * ----- | 6-15 | |

TECHNICAL FIELDS SEARCHED (IPC)

1

The present search report has been drawn up for all claims

| Place of search | Date of completion of the search | Examiner |
|---|---|---|
| Munich | 12 December 2019 | Petri, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 19 19 1147

This annex lists the patent family members relating to the patent documents cited in the above-mentioned European search report. The members are as contained in the European Patent Office EDP file on
The European Patent Office is in no way liable for these particulars which are merely given for the purpose of information.

12-12-2019

| Patent document cited in search report | Publication date | Patent family member(s) | Publication date |
|---|---|---|---|
| EP 1038875 A2 | 27-09-2000 | AT 242241 T | 15-06-2003 |
| | | DE 69908546 T2 | 29-04-2004 |
| | | DK 1038875 T3 | 29-09-2003 |
| | | EP 1038875 A2 | 27-09-2000 |
| | | ES 2195510 T3 | 01-12-2003 |
| | | PT 1038875 E | 31-10-2003 |
| | | SI 1038875 T1 | 31-12-2003 |
| | | US 6242460 B1 | 05-06-2001 |
| | | US 6281360 B1 | 28-08-2001 |
| WO 0230358 A2 | 18-04-2002 | AU 1346702 A | 22-04-2002 |
| | | AU 2002213467 A8 | 30-07-2009 |
| | | CA 2425259 A1 | 18-04-2002 |
| | | EP 1578341 A2 | 28-09-2005 |
| | | US 2002173524 A1 | 21-11-2002 |
| | | US 2004039035 A1 | 26-02-2004 |
| | | WO 0230358 A2 | 18-04-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MORIZANE et al.** *Stem Cell Reports,* 26 September 2013, vol. 1 (4), 283-9
- **WATANABE et al.** *Nat Biotechnol.,* June 2007, vol. 25 (6), 681-6
- **YING et al.** *Nature,* 22 May 2008, vol. 453 (7194), 519-23
- **HOU et al.** *Science,* 09 August 2013, vol. 341 (6146), 651-4
- **ZHAO et al.** *Cell,* 17 December 2015, vol. 163 (7), 1678-91
- *CHEMICAL ABSTRACTS,* 82626-48-0
- **A. SOUFI ; G. DONAHUE ; K. S. ZARET.** *Cell,* 2012, vol. 151, 994-1004
- **KOCHE et al.** *Cell Stem Cell,* 2011, vol. 8, 96-105
- **POLO ; HOCHEDLINGER.** *Cell Stem Cell,* 2010, vol. 7, 5-6
- **HOLENYA et al.** *Proteomics,* May 2011, vol. 11 (10), 2129-33
- **RECENTLY ; ONDER et al.** *Nature,* 2012, vol. 483, 598-602
- **CHENG et al.** *J Med Chem,* 2015, vol. 58 (12), 4976-83
- **CHENG et al.** *J Med Chem,* 2015, vol. 58 (15), 5742-50
- **WANG et al.** *Nature,* 2014, vol. 516 (7531), 405-9
- **CHENG et al.** *Mol Oncol.,* June 2016, vol. 10 (6), 806-24
- **OKITA et al.** *Nat Protoc,* 2010, vol. 5, 418-428
- **OKITA et al.** *Nat Methods,* 2011, vol. 8
- **OKITA et al.** *Science,* 2008, vol. 322, 949-953
- **CHENG et al.** *J Med Chem,* 2015, vol. 58, 4976-4983
- **CHENG et al.** *J Med Chem,* 2015, vol. 58, 5742-5750
- **WANG et al.** *Nature,* 2014, vol. 516, 405-409
- **GRABUNDZIJA et al.** *Nucleic acids research,* 2013, vol. 41, 1829-1847
- **WATANABE et al.** *Nat Biotechnol,* 2007, vol. 25 (6), 681-6

SEQUENCE LISTING

<110>  University of Heidelberg

<120>  Application of Imidazopyridine Derivatives in Regenerative Medicine

<130>  rku101ep-b

<160>  66

<170>  PatentIn version 3.5

<210>  1
<211>  20
<212>  DNA
<213>  Artificial Sequence

<220>
<223>  Primer

<400>  1
gaaggatgtg gtccgagtgt                                                    20

<210>  2
<211>  20
<212>  DNA
<213>  Artificial Sequence

<220>
<223>  Primer

<400>  2
gtgaagtgag ggctcccata                                                    20

<210>  3
<211>  20
<212>  DNA
<213>  Artificial Sequence

<220>
<223>  Primer

<400>  3
aaccccaaga tgcacaactc                                                    20

<210>  4
<211>  20
<212>  DNA
<213>  Artificial Sequence

<220>
<223>  Primer

<400>  4
gcttagcctc gtcgatgaac                                                    20

<210>  5
<211>  23

<212> DNA
<213> Artificial Sequence

<220>
<223> Primer

<400> 5
aatacctcag cctccagcag atg 23

<210> 6
<211> 24
<212> DNA
<213> Artificial Sequence

<220>
<223> Primer

<400> 6
tgcgtcacac cattgctatt cttc 24

<210> 7
<211> 21
<212> DNA
<213> Artificial Sequence

<220>
<223> Primer

<400> 7
gtggatgtct ttgtgcacca g 21

<210> 8
<211> 20
<212> DNA
<213> Artificial Sequence

<220>
<223> Primer

<400> 8
gacacggatg gattccagac 20

<210> 9
<211> 22
<212> DNA
<213> Artificial Sequence

<220>
<223> Primer

<400> 9
ggacctggac tttattctct cc 22

<210> 10
<211> 20
<212> DNA
<213> Artificial Sequence

<220>
<223> Primer

<400> 10
gataggtgaa gctgcaggtg 20

<210> 11
<211> 20
<212> DNA
<213> Artificial Sequence

<220>
<223> Primer

<400> 11
ccttccaatc agctgccttc 20

<210> 12
<211> 20
<212> DNA
<213> Artificial Sequence

<220>
<223> Primer

<400> 12
gatctgcctg cctctctcat 20

<210> 13
<211> 20
<212> DNA
<213> Artificial Sequence

<220>
<223> Primer

<400> 13
gtcgacatca cttgccagac 20

<210> 14
<211> 20
<212> DNA
<213> Artificial Sequence

<220>
<223> Primer

<400> 14
tggtcggatg aaactcctcc 20

<210> 15
<211> 20
<212> DNA
<213> Artificial Sequence

<220>
<223> Primer

<400> 15
aggagtgtgt gctaaccgtt 20

<210> 16
<211> 20
<212> DNA
<213> Artificial Sequence

<220>
<223> Primer

<400> 16
cagttcgttt cagtgccaca 20

<210> 17
<211> 20
<212> DNA
<213> Artificial Sequence

<220>
<223> Primer

<400> 17
gagagactgg gttattcctc 20

<210> 18
<211> 21
<212> DNA
<213> Artificial Sequence

<220>
<223> Primer

<400> 18
gatgctgtag aaaaccttgc c 21

<210> 19
<211> 20
<212> DNA
<213> Artificial Sequence

<220>
<223> Primer

<400> 19
tggacacgtc tgtgctcttc 20

<210> 20
<211> 20
<212> DNA
<213> Artificial Sequence

<220>
<223> Primer

<400> 20
gtcttggcgt cttctcgaac 20

<210> 21
<211> 20
<212> DNA
<213> Artificial Sequence

<220>
<223> Primer

<400> 21
atttgtggga ccctcgacat 20

<210> 22
<211> 20
<212> DNA
<213> Artificial Sequence

<220>
<223> Primer

<400> 22
ggtaaaagct cgcccacaaa 20

<210> 23
<211> 23
<212> DNA
<213> Artificial Sequence

<220>
<223> Primer

<400> 23
ctgactacct catgaagatc ctc 23

<210> 24
<211> 22
<212> DNA
<213> Artificial Sequence

<220>
<223> Primer

<400> 24
cattgccaat ggtgatgacc tg 22

<210> 25
<211> 20
<212> DNA
<213> Artificial Sequence

<220>
<223> Primer

<400> 25
agagggacat ctcacggttc 20

<210> 26
<211> 19

<212> DNA
<213> Artificial Sequence

<220>
<223> Primer

<400> 26
ggttgcccca gaagtatcg 19

<210> 27
<211> 21
<212> DNA
<213> Artificial Sequence

<220>
<223> Primer

<400> 27
ggtgccagtt aaagatgacg c 21

<210> 28
<211> 20
<212> DNA
<213> Artificial Sequence

<220>
<223> Primer

<400> 28
gaggcaaaat ggtcggcaag 20

<210> 29
<211> 21
<212> DNA
<213> Artificial Sequence

<220>
<223> Primer

<400> 29
gacctccaca gagaagtcga g 21

<210> 30
<211> 21
<212> DNA
<213> Artificial Sequence

<220>
<223> Primer

<400> 30
tgcctttttc ttagggcaga g 21

<210> 31
<211> 19
<212> DNA
<213> Artificial Sequence

<220>
<223>  Primer

<400>  31
gcaagggcaa gctctatgg                                                    19

<210>  32
<211>  22
<212>  DNA
<213>  Artificial Sequence

<220>
<223>  Primer

<400>  32
aggactcgta ggcgttgtag tt                                                22

<210>  33
<211>  18
<212>  DNA
<213>  Artificial Sequence

<220>
<223>  Primer

<400>  33
cgctttctcc cagaccaa                                                     18

<210>  34
<211>  24
<212>  DNA
<213>  Artificial Sequence

<220>
<223>  Primer

<400>  34
ggcagacagg ttaaagtaga ggag                                              24

<210>  35
<211>  18
<212>  DNA
<213>  Artificial Sequence

<220>
<223>  Primer

<400>  35
caccagcagc gactctga                                                     18

<210>  36
<211>  22
<212>  DNA
<213>  Artificial Sequence

<220>
<223>  Primer

<400> 36
gatccagact ctgacctttt gc 22

<210> 37
<211> 20
<212> DNA
<213> Artificial Sequence

<220>
<223> Primer

<400> 37
agatgcctca cacggagact 20

<210> 38
<211> 20
<212> DNA
<213> Artificial Sequence

<220>
<223> Primer

<400> 38
ggactggtgg aagaatcagg 20

<210> 39
<211> 18
<212> DNA
<213> Artificial Sequence

<220>
<223> Primer

<400> 39
cgaccatctg ccgctttg 18

<210> 40
<211> 21
<212> DNA
<213> Artificial Sequence

<220>
<223> Primer

<400> 40
gccgcagctt acacatgttc t 21

<210> 41
<211> 20
<212> DNA
<213> Artificial Sequence

<220>
<223> Primer

<400> 41
agcacatcaa ctcggaggag 20

<210> 42
<211> 20
<212> DNA
<213> Artificial Sequence

<220>
<223> Primer

<400> 42
agcacatcaa ctcggaggag 20

<210> 43
<211> 21
<212> DNA
<213> Artificial Sequence

<220>
<223> Primer

<400> 43
acagcaaatg acagctgcaa a 21

<210> 44
<211> 17
<212> DNA
<213> Artificial Sequence

<220>
<223> Primer

<400> 44
tcggcatcgc ggttttt 17

<210> 45
<211> 20
<212> DNA
<213> Artificial Sequence

<220>
<223> Primer

<400> 45
ggttggtatc cggggacttc 20

<210> 46
<211> 20
<212> DNA
<213> Artificial Sequence

<220>
<223> Primer

<400> 46
tccgttggaa ctgatggagt 20

<210> 47
<211> 18

<212> DNA
<213> Artificial Sequence

<220>
<223> Primer

<400> 47
accaggccat ggatgaag 18

<210> 48
<211> 20
<212> DNA
<213> Artificial Sequence

<220>
<223> Primer

<400> 48
cttaattgct ggggaattgg 20

<210> 49
<211> 20
<212> DNA
<213> Artificial Sequence

<220>
<223> Primer

<400> 49
ctggctgtgg caaggtcttc 20

<210> 50
<211> 20
<212> DNA
<213> Artificial Sequence

<220>
<223> Primer

<400> 50
cagccctcaa actcgcactt 20

<210> 51
<211> 20
<212> DNA
<213> Artificial Sequence

<220>
<223> Primer

<400> 51
ggaagcccaa gaacctgaat 20

<210> 52
<211> 19
<212> DNA
<213> Artificial Sequence

<220>
<223> Primer

<400> 52
gttgctggag ttgctggaa 19

<210> 53
<211> 21
<212> DNA
<213> Artificial Sequence

<220>
<223> Primer

<400> 53
aatacttccc ccacaacaca a 21

<210> 54
<211> 18
<212> DNA
<213> Artificial Sequence

<220>
<223> Primer

<400> 54
ctctcccgca ccagtcat 18

<210> 55
<211> 18
<212> DNA
<213> Artificial Sequence

<220>
<223> Primer

<400> 55
acgccgagtt gagcaaga 18

<210> 56
<211> 18
<212> DNA
<213> Artificial Sequence

<220>
<223> Primer

<400> 56
tctgcctcct ccacgaag 18

<210> 57
<211> 19
<212> DNA
<213> Artificial Sequence

<220>
<223> Primer

<400> 57
accgccacca aactgagat 19

<210> 58
<211> 20
<212> DNA
<213> Artificial Sequence

<220>
<223> Primer

<400> 58
ttgtagtggg cagtgggatt 20

<210> 59
<211> 19
<212> DNA
<213> Artificial Sequence

<220>
<223> Primer

<400> 59
aactcaagaa ggcggatgg 19

<210> 60
<211> 19
<212> DNA
<213> Artificial Sequence

<220>
<223> Primer

<400> 60
ggaggaaaac cttcgtgct 19

<210> 61
<211> 20
<212> DNA
<213> Artificial Sequence

<220>
<223> Primer

<400> 61
acccagttca tagcggtgac 20

<210> 62
<211> 20
<212> DNA
<213> Artificial Sequence

<220>
<223> Primer

<400> 62
ccattgggag tacccaggtt 20

<210> 63
<211> 19
<212> DNA
<213> Artificial Sequence

<220>
<223> Primer

<400> 63
agccacatcg ctcagacac 19

<210> 64
<211> 19
<212> DNA
<213> Artificial Sequence

<220>
<223> Primer

<400> 64
gcccaatacg accaaatcc 19

<210> 65
<211> 27
<212> DNA
<213> Artificial Sequence

<220>
<223> Primer

<400> 65
ggatgttatt aagatgaaga tagttgg 27

<210> 66
<211> 25
<212> DNA
<213> Artificial Sequence

<220>
<223> Primer

<400> 66
cctaaactcc ccttcaaaat ctatt 25